(19) Europäisches Patentamt
European Patent Office
Office européen des brevets

(11) EP 1 681 347 A1

(12) EUROPEAN PATENT APPLICATION

(43) Date of publication:
**19.07.2006 Bulletin 2006/29**

(51) Int Cl.:
***C12N 15/11*** *(2006.01)* ***A61K 48/00*** *(2006.01)*

(21) Application number: **05000877.0**

(22) Date of filing: **18.01.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Metanomics GmbH & Co. KGaA**
**10589 Berlin-Charlottenburg (DE)**

(72) Inventor: **Ebneth, Marcus, Dr.**
**10965 Berlin (DE)**

(74) Representative: **Bieberbach, Andreas et al**
**BASF Aktiengesellschaft**
**67056 Ludwigshafen (DE)**

# (54) Improved methods for double-stranded RNA mediated gene silencing

(57) The present invention is in the field of genetics, especially plant genetics, and provides agents capable of gene-specific silencing. The present invention specifically provides polycistronic RNA molecules capable to generate double-stranded RNA (dsRNA) agents, methods for utilizing such molecules and cells and organism, especially plants, containing such molecules. The polycistronic RNA molecules, compositions and methods of the invention can be used to enhance performance of agricultural relevant crops and for therapy, prophylaxis, research and diagnostics in diseases and disorders which afflict mammalian species, in which the goal is to reduce or inhibit the function of a selected target polynucleotide sequence. These compositions and methods have utility both in vitro and in vivo.

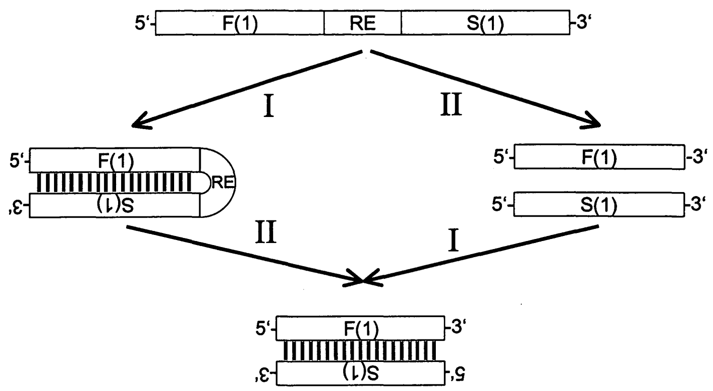


Fig. 1

EP 1 681 347 A1

SEQUENCE LISTING

<110> metanomics GmbH&Co.KGaA

<120> Improved methods for double-stranded RNA mediated gene silencing

<130> AE20040971

<160> 19

<170> PatentIn version 3.3

<210> 1
<211> 652
<212> DNA
<213> Arabidopsis thaliana

<220>
<221> misc_feature
<222> (1)..(652)
<223> coding for Arabidopsis thaliana snoRNA cluster 9

<220>
<221> snoRNA
<222> (47)..(155)
<223> mature z43 snoRNA

<220>
<221> snoRNA
<222> (233)..(388)
<223> mature snR74(z4) snoRNA

<220>
<221> snoRNA
<222> (496)..(563)
<223> mature snR60(z15) snoRNA

<400> 1
```
ttcatcttct tcttctggtg ttaaaacttt tgtttttggt aaaagtaatg gacagtgacg      60
attgatatta aggtctcgtt cgtcgtaaaa acggccgtaa aaagcctatt tgccgaccat     120
tctgttatat caccacccat attctgagtc ctattgattt ttgtttttttc aatctttttc     180
taatcgattt tttggccaat gatttaatca aaattgacaa gcatatgtct gaattaatcc     240
ttgtttgata aactctaatc ttctctatga ggcctttttt caattacctt tcaaatccga     300
tttcgtattt atttctttgg ttctgttttt ggttgattta gtggcctatg atttaaatga     360
ttatagacaa gcatatgtct gaattaatcc ttgttgataa atctaatctt ctctatgagg     420
tctaaatccg attttgtttt ttggttattg gttttaaaat tgtcttggat ttatttgatg     480
agaaaagagg aaattgaaag gcatcaagtg atgattgata acgcatagtt cagatgataa     540
attctattat gattaacatc ttcagcatag tctttcgctc ctatctgatg acccttttcct     600
ctctattttc atctattatc caaatttttc aatctcaaat tgttccttaa gt             652
```

<210> 2
<211> 478
<212> DNA
<213> Arabidopsis thaliana

<220>
<221> misc_feature
<222> (1)..(478)
<223> coding for Arabidopsis thaliana snoRNA cluster 2

<220>
<221> snoRNA
<222> (41)..(134)
<223> coding for mature snoRNA z44a

<220>
<221> snoRNA
<222> (218)..(313)
<223> coding for mature snoRNA z44b

<220>
<221> snoRNA
<222> (362)..(460)
<223> coding for mature snoRNA u24

<400> 2
ttggttgaat attggaactt ttgtggagca gtattgatat tgatggtgag gatgacgaaa 60
aaatcattcg gattcccttt gaattcctcc ggaaacatgt gattatataa tcagctactt 120
ctgactcatc atcatcatca taacaccttt ttgtttttcg ccgatttaga aaacgttttt 180
tgaggtcttt tatgacattt tctttgataa atttttttga tggtaaagat gacgaaaaaa 240
atcgttcgga ttccctttga attccctcgg gaaagatgtg atcacaaacc agctacttct 300
gattttacca tcatcgatat agatttccca aaataagttc cacttttgaa ttgtttttga 360
atgaggccag tgatgtaata aaaatatttg ctactcttga tgaagaactt tgttctttca 420
gttgatgatt ggtttaccac caagatctct gagggcctta gttttctact gtttatct 478

<210> 3
<211> 207
<212> DNA
<213> Arabidopsis thaliana

<220>
<221> misc_feature
<222> (1)..(207)
<223> encoding Arabidopsis thaliana pre-RNA for tRNAGln

<220>
<221> tRNA
<222> (129)..(200)
<223> encoding mature tRNAGln

<400> 3
agacaaagaa cactgcaatc tgtggtgctg ctggactgct gatgttgaat ctattcattg 60
gccgtgtcta aaatacaata ttttggatta gattctaaac tggtacatgc aacagaacaa 120
gcacctgagg ttctatggtg tagtggttag cactctggac tttgaatcca gcgacctggg 180
ttcgactccc ggtaggacct ctcatgc 207

<210> 4
<211> 147
<212> DNA
<213> Arabidopsis thaliana

<220>
<221> misc_feature
<222> (1)..(147)
<223> encoding pre-RNA for Arabidopsis thaliana tRNAHis

<220>
<221> tRNA
<222> (51)..(122)
<223> encoding mature Arabidopsis thaliana tRNAHis

<400> 4
ttattaaatc tttggttaat taaatactaa gggaaagaaa taacacaaaa gtggttgtag 60
tatagcggtt agtatcccac gttgtggccg tggggacccg ggctcgaatc ccggcagcca 120
cactttttgt tacctcttct tttttat 147

<210> 5
<211> 160
<212> DNA
<213> Arabidopsis thaliana

<220>
<221> misc_feature
<222> (1)..(160)
<223> encoding pre-RNA for Arabidopsis thaliana tRNAMet

<220>
<221> tRNA
<222> (51)..(135)

<223> encoding mature Arabidopsis thaliana tRNAMet

<400> 5

```
atggaaaata ggggtaaaaa atacataata ttacggtact caacaacatt ggggtggtgg    60
cgcagttggc tagcgcgtag gtctcatagc tagtgagtga tcctgaggtc gagagttcga   120
gcctctctca ccccaatatt tctttttgca ttgtttttat                         160
```

<210> 6
<211> 188
<212> DNA
<213> Arabidopsis thaliana

<220>
<221> misc_feature
<222> (1)..(188)
<223> encoding pre-RNA for Arabidopsis dicistronic tRNA snoRNA gene

<220>
<221> tRNA
<222> (14)..(84)
<223> encoding mature Arabidopsis tRNAGly

<220>
<221> snoRNA
<222> (85)..(168)
<223> encoding mature Arabidopsis snoRNA R43.1

<400> 6

```
aaggttcaac aaagcaccag tggtctagtg gtagaatagt accctgccac ggtacagacc    60
cgggttcgat tcccggctgg tgcatgagct gtgatgaaat tggctttagt attggttggg   120
tccaatgcat tctcctgaat atcagatgat ttcgcctact ctgagctcta tttttaattt   180
ttattttt                                                            188
```

<210> 7
<211> 491
<212> DNA
<213> Arabidopsis thaliana

<220>
<221> misc_feature
<222> (1)..(491)
<223> fragment of Arabidopsis thaliana gene at4g39400; coding for Arabidopsis thaliana brassinosteroid insensitive 1 (BRI1) mRNA; GenBank Acc.-No.: NM_120100

<400> 7
gaggtaacat ggcatgtgag tgcacagctg tgaccactct tcccttcacc atttgataaa 60
taccttcttc catcttcctt cttctgtctt ttgactctct tctctctctc ctctctttct 120
gctttcctca atctctctct ttctatctct agagcttcca cttcctctct aatggtggaa 180
ccaaaaccct agattccccc tttcatcttc tctacttccc acacttttct ctctcacaaa 240
ctcttgagaa atgaagactt tttcaagctt ctttctctct gtaacaactc tcttcttctt 300
ctccttcttt tctctttcat ttcaagcttc accatctcag tctttataca gagaaatcca 360
tcagcttata agcttcaaag acgttcttcc tgacaagaat cttctcccag actggtcttc 420
caacaaaaac ccgtgtactt tcgatggcgt tacttgcaga gacgacaaag ttacttcgat 480
tgatctcagc t 491

<210> 8
<211> 540
<212> DNA
<213> Arabidopsis thaliana

<220>
<221> misc_feature
<222> (1)..(540)
<223> fragment of Arabidopsis thaliana gene at4g18710; coding for Arabidopsis thaliana shaggy-related protein kinase eta / ASK-eta (ASK7) mRNA; GenBank Acc.-No.: NM_117987

<400> 8
aagggtgttt acgtaattgt agcttaacaa ataaaaaaca cgaaagaaga tggcgaagaa 60
aacggaaagt agtagataga taaagaaaga gaaagagaag agctggattc acatggtctt 120
gttctttctc tctccttctt ttctcatctt gcggcttccc tttttctctc tatcgccaca 180
atgatcatta ccaaccaaac tgattgaaac tcatttgttc tctctctctc aaatccactc 240
tctctctttc ttttctcttc tcctctctgt gtctctatcg ccatggctga tgataaggag 300
atgcctgctg ctgtagttga tggacatgat caagtcactg gtcatattat ttccaccaca 360
atcggtggca aaaatggtga accaaaacag acaattagtt acatggcgga gcgagttgtt 420
ggtacaggct cgttcgggat cgttttccaa gcaaaatgtt tggagactgg agaaaccgtg 480
gcgataaaga aggtttgca agatagaaga tacaagaacc gagaacttca gttgatgcgt 540

<210> 9
<211> 39
<212> DNA
<213> Artificial

<220>
<223> Oligonucleotide primer

<400> 9
atacccgggt accgtcgaca gacaaagaac actgcaatc 39

<210> 10
<211> 38
<212> DNA
<213> Artificial

<220>
<223> Oligonucleotide primer

<400> 10
atagagctct gtacactcga ggcatgagag gtcctacc 38

<210> 11
<211> 37
<212> DNA
<213> Artificial

<220>
<223> Oligonucleotide primer

<400> 11
atacccgggt accgtcgact tcatcttctt cttctgg 37

<210> 12
<211> 39
<212> DNA
<213> Artificial

<220>
<223> Oligonucleotide primer

<400> 12
atagagctct gtacactcga gacttaagga acaatttga 39

<210> 13
<211> 27
<212> DNA
<213> Artificial

<220>
<223> Oligonucleotide primer

<400> 13
ataggtaccg aggtaacatg gcatgtg 27

<210> 14

<211> 27
<212> DNA
<213> Artificial

<220>
<223> Oligonucleotide primer

<400> 14
atagtcgaca gctgagatca atcgaag 27

<210> 15
<211> 28
<212> DNA
<213> Artificial

<220>
<223> Oligonucleotide primer

<400> 15
ataggtacca agggtgttta cgtaattg 28

<210> 16
<211> 27
<212> DNA
<213> Artificial

<220>
<223> Oligonucleotide primer

<400> 16
atagtcgaca cgcatcaact gaagttc 27

<210> 17
<211> 8885
<212> DNA
<213> Artificial

<220>
<223> Binary vector 1bxPcUbiLic

<400> 17
gggttgctct tccatggcaa tgattaatta acgaagagca agagctcgaa tttccccgat 60
cgttcaaaca tttggcaata aagtttctta agattgaatc ctgttgccgg tcttgcgatg 120
attatcatat aatttctgtt gaattacgtt aagcatgtaa taattaacat gtaatgcatg 180
acgttattta tgagatgggt ttttatgatt agagtcccgc aattatacat ttaatacgcg 240

```
atagaaaaca aaatatagcg cgcaaactag gataaattat cgcgcgcggt gtcatctatg    300
ttactagatc gggaattggc atgcaagctt ggcactggcc gtcgttttac aacgtcgtga    360
ctgggaaaac cctggcgtta cccaacttaa tcgccttgca gcacatcccc ctttcgccag    420
ctggcgtaat agcgaagagg cccgcaccga tcgcccttcc caacagttgc gcagcctgaa    480
tggcgaatgc tagagcagct tgagcttgga tcagattgtc gtttcccgcc ttcagtttaa    540
actatcagtg tttgacagga tatattggcg ggtaaaccta agagaaaaga gcgtttatta    600
gaataacgga tatttaaaag ggcgtgaaaa ggtttatccg ttcgtccatt tgtatgtgca    660
tgccaaccac agggttcccc tcgggatcaa agtactttga tccaacccct ccgctgctat    720
agtgcagtcg gcttctgacg ttcagtgcag ccgtcttctg aaaacgacat gtcgcacaag    780
tcctaagtta cgcgacaggc tgccgccctg cccttttcct ggcgttttct tgtcgcgtgt    840
tttagtcgca taaagtagaa tacttgcgac tagaaccgga gacattacgc catgaacaag    900
agcgccgccg ctggcctgct gggctatgcc cgcgtcagca ccgacgacca ggacttgacc    960
aaccaacggg ccgaactgca cgcggccggc tgcaccaagc tgttttccga gaagatcacc   1020
ggcaccaggc gcgaccgccc ggagctggcc aggatgcttg accacctacg ccctggcgac   1080
gttgtgacag tgaccaggct agaccgcctg gcccgcagca cccgcgacct actggacatt   1140
gccgagcgca tccaggaggc cggcgcgggc ctgcgtagcc tggcagagcc gtgggccgac   1200
accaccacgc cggccggccg catggtgttg accgtgttcg ccggcattgc cgagttcgag   1260
cgttccctaa tcatcgaccg cacccggagc gggcgcgagg ccgccaaggc ccgaggcgtg   1320
aagtttggcc cccgccctac cctcaccccg gcacagatcg cgcacgcccg cgagctgatc   1380
gaccaggaag gccgcaccgt gaaagaggcg gctgcactgc ttggcgtgca tcgctcgacc   1440
ctgtaccgcg cacttgagcg cagcgaggaa gtgacgccca ccgaggccag gcggcgcggt   1500
gccttccgtg aggacgcatt gaccgaggcc gacgccctgg cggccgccga gaatgaacgc   1560
caagaggaac aagcatgaaa ccgcaccagg acggccagga cgaaccgttt ttcattaccg   1620
aagagatcga ggcggagatg atcgcggccg ggtacgtgtt cgagccgccc gcgcacgtct   1680
caaccgtgcg gctgcatgaa atcctggccg gtttgtctga tgccaagctg gcggcctggc   1740
cggccagctt ggccgctgaa gaaaccgagc gccgccgtct aaaaaggtga tgtgtatttg   1800
agtaaaacag cttgcgtcat gcggtcgctg cgtatatgat gcgatgagta aataaacaaa   1860
tacgcaaggg gaacgcatga aggttatcgc tgtacttaac cagaaaggcg ggtcaggcaa   1920
gacgaccatc gcaacccatc tagcccgcgc cctgcaactc gccggggccg atgttctgtt   1980
agtcgattcc gatccccagg gcagtgcccg cgattgggcg gccgtgcggg aagatcaacc   2040
gctaaccgtt gtcggcatcg accgcccgac gattgaccgc gacgtgaagg ccatcggccg   2100
gcgcgacttc gtagtgatcg acggagcgcc ccaggcggcg gacttggctg tgtccgcgat   2160
caaggcagcc gacttcgtgc tgattccggt gcagccaagc ccttacgaca tatgggccac   2220
cgccgacctg gtggagctgg ttaagcagcg cattgaggtc acggatggaa ggctacaagc   2280
ggcctttgtc gtgtcgcggg cgatcaaagg cacgcgcatc ggcggtgagg ttgccgaggc   2340
gctggccggg tacgagctgc ccattcttga gtcccgtatc acgcagcgcg tgagctaccc   2400
aggcactgcc gccgccggca caaccgttct tgaatcagaa cccgagggcg acgctgcccg   2460
cgaggtccag gcgctggccg ctgaaattaa atcaaaactc atttgagtta atgaggtaaa   2520
gagaaaatga gcaaaagcac aaacacgcta agtgccggcc gtccgagcgc acgcagcagc   2580
aaggctgcaa cgttggccag cctggcagac acgccagcca tgaagcgggt caactttcag   2640
ttgccggcgg aggatcacac caagctgaag atgtacgcgg tacgccaagg caagaccatt   2700
accgagctgc tatctgaata catcgcgcag ctaccagagt aaatgagcaa atgaataaat   2760
gagtagatga attttagcgg ctaaaggagg cggcatggaa aatcaagaac aaccaggcac   2820
cgacgccgtg gaatgcccca tgtgtggagg aacgggcggt tggccaggcg taagcggctg   2880
ggttgtctgc cggccctgca atggcactgg aacccccaag cccgaggaat cggcgtgacg   2940
```

gtcgcaaacc atccggcccg gtacaaatcg gcgcggcgct gggtgatgac ctggtggaga 3000
agttgaaggc cgcgcaggcc gcccagcggc aacgcatcga ggcagaagca cgccccggtg 3060
aatcgtggca agcggccgct gatcgaatcc gcaaagaatc ccggcaaccg ccggcagccg 3120
gtgcgccgtc gattaggaag ccgcccaagg gcgacgagca accagatttt ttcgttccga 3180
tgctctatga cgtgggcacc cgcgatagtc gcagcatcat ggacgtggcc gttttccgtc 3240
tgtcgaagcg tgaccgacga gctggcgagg tgatccgcta cgagcttcca gacgggcacg 3300
tagaggtttc cgcagggccg gccggcatgg ccagtgtgtg ggattacgac ctggtactga 3360
tggcggtttc ccatctaacc gaatccatga accgataccg ggaagggaag ggagacaagc 3420
ccggccgcgt gttccgtcca cacgttgcgg acgtactcaa gttctgccgg cgagccgatg 3480
gcggaaagca gaaagacgac ctggtagaaa cctgcattcg gttaaacacc acgcacgttg 3540
ccatgcagcg tacgaagaag gccaagaacg gccgcctggt gacggtatcc gagggtgaag 3600
ccttgattag ccgctacaag atcgtaaaga gcgaaaccgg gcggccggag tacatcgaga 3660
tcgagctagc tgattggatg taccgcgaga tcacagaagg caagaacccg gacgtgctga 3720
cggttcaccc cgattacttt ttgatcgatc ccggcatcgg ccgttttctc taccgcctgg 3780
cacgccgcgc cgcaggcaag gcagaagcca gatggttgtt caagacgatc tacgaacgca 3840
gtggcagcgc cggagagttc aagaagttct gtttcaccgt gcgcaagctg atcgggtcaa 3900
atgacctgcc ggagtacgat ttgaaggagg aggcggggca ggctggcccg atcctagtca 3960
tgcgctaccg caacctgatc gagggcgaag catccgccgg ttcctaatgt acggagcaga 4020
tgctagggca aattgcccta gcaggggaaa aaggtcgaaa aggtctcttt cctgtggata 4080
gcacgtacat tgggaaccca aagccgtaca ttgggaaccg gaacccgtac attgggaacc 4140
caaagccgta cattgggaac cggtcacaca tgtaagtgac tgatataaaa gagaaaaaag 4200
gcgatttttc cgcctaaaac tctttaaaac ttattaaaac tcttaaaacc cgcctggcct 4260
gtgcataact gtctggccag cgcacagccg aagagctgca aaaagcgcct acccttcggt 4320
cgctgcgctc cctacgcccc gccgcttcgc gtcggcctat cgcggccgct ggccgctcaa 4380
aaatggctgg cctacggcca ggcaatctac cagggcgcgg acaagccgcg ccgtcgccac 4440
tcgaccgccg gcgcccacat caaggcaccc tgcctcgcgc gtttcggtga tgacggtgaa 4500
aacctctgac acatgcagct cccggagacg gtcacagctt gtctgtaagc ggatgccggg 4560
agcagacaag cccgtcaggg cgcgtcagcg ggtgttggcg ggtgtcgggg cgcagccatg 4620
acccagtcac gtagcgatag cggagtgtat actggcttaa ctatgcggca tcagagcaga 4680
ttgtactgag agtgcaccat atgcggtgtg aaataccgca cagatgcgta aggagaaaat 4740
accgcatcag gcgctcttcc gcttcctcgc tcactgactc gctgcgctcg gtcgttcggc 4800
tgcggcgagc ggtatcagct cactcaaagg cggtaatacg gttatccaca gaatcagggg 4860
ataacgcagg aaagaacatg tgagcaaaag gccagcaaaa ggccaggaac cgtaaaaagg 4920
ccgcgttgct ggcgtttttc cataggctcc gcccccctga cgagcatcac aaaaatcgac 4980
gctcaagtca gaggtggcga aacccgacag gactataaag ataccaggcg tttccccctg 5040
gaagctccct cgtgcgctct cctgttccga ccctgccgct taccggatac ctgtccgcct 5100
ttctcccttc gggaagcgtg gcgctttctc atagctcacg ctgtaggtat ctcagttcgg 5160
tgtaggtcgt tcgctccaag ctgggctgtg tgcacgaacc ccccgttcag cccgaccgct 5220
gcgccttatc cggtaactat cgtcttgagt ccaacccggt aagacacgac ttatcgccac 5280
tggcagcagc cactggtaac aggattagca gagcgaggta tgtaggcggt gctacagagt 5340
tcttgaagtg gtggcctaac tacggctaca ctagaaggac agtatttggt atctgcgctc 5400
tgctgaagcc agttaccttc ggaaaaagag ttggtagctc ttgatccggc aaacaaacca 5460
ccgctggtag cggtggtttt tttgtttgca agcagcagat tacgcgcaga aaaaaaggat 5520
ctcaagaaga tcctttgatc ttttctacgg ggtctgacgc tcagtggaac gaaaactcac 5580
gttaagggat tttggtcatg cattctaggt actaaaacaa ttcatccagt aaaatataat 5640

attttatttt ctcccaatca ggcttgatcc ccagtaagtc aaaaaatagc tcgacatact 5700
gttcttcccc gatatcctcc ctgatcgacc ggacgcagaa ggcaatgtca taccacttgt 5760
ccgccctgcc gcttctccca agatcaataa agccacttac tttgccatct ttcacaaaga 5820
tgttgctgtc tcccaggtcg ccgtgggaaa agacaagttc ctcttcgggc ttttccgtct 5880
ttaaaaaatc atacagctcg cgcggatctt taaatggagt gtcttcttcc cagttttcgc 5940
aatccacatc ggccagatcg ttattcagta agtaatccaa ttcggctaag cggctgtcta 6000
agctattcgt atagggacaa tccgatatgt cgatggagtg aaagagcctg atgcactccg 6060
catacagctc gataatcttt tcagggcttt gttcatcttc atactcttcc gagcaaagga 6120
cgccatcggc ctcactcatg agcagattgc tccagccatc atgccgttca aagtgcagga 6180
cctttggaac aggcagcttt ccttccagcc atagcatcat gtccttttcc cgttccacat 6240
cataggtggt ccctttatac cggctgtccg tcatttttaa atataggttt tcattttctc 6300
ccaccagctt atatacctta gcaggagaca ttccttccgt atcttttacg cagcggtatt 6360
tttcgatcag tttttttcaat tccggtgata ttctcatttt agccatttat tatttccttc 6420
ctcttttcta cagtatttaa agatacccca agaagctaat tataacaaga cgaactccaa 6480
ttcactgttc cttgcattct aaaaccttaa ataccagaaa acagcttttt caaagttgtt 6540
ttcaaagttg gcgtataaca tagtatcgac ggagccgatt ttgaaaccgc ggtgatcaca 6600
ggcagcaacg ctctgtcatc gttacaatca acatgctacc ctccgcgaga tcatccgtgt 6660
ttcaaacccg gcagcttagt tgccgttctt ccgaatagca tcggtaacat gagcaaagtc 6720
tgccgcctta caacggctct cccgctgacg ccgtcccgga ctgatgggct gcctgtatcg 6780
agtggtgatt ttgtgccgag ctgccggtcg gggagctgtt ggctggctgg tggcaggata 6840
tattgtggtg taaacaaatt gacgcttaga caacttaata acacattgcg gacgttttta 6900
atgtactgaa ttaacgccga attaagcttg gacaatcagt aaattgaacg gagaatatta 6960
ttcataaaaa tacgatagta acgggtgata tattcattag aatgaaccga aaccggcggt 7020
aaggatctga gctacacatg ctcaggtttt ttacaacgtg cacaacagaa ttgaaagcaa 7080
atatcatgcg atcataggcg tctcgcatat ctcattaaag cagggcatgc cggtcgagtc 7140
aaatctcggt gacgggcagg accggacggg gccgtaccgg caggctgaag tccagctgcc 7200
agaaacccac gtcatgccag ttcccgtgct tgaagccggc cgcccgcagc atgccgcggg 7260
gggcatatcc gagcgcctcg tgcatgcgca cgctcgggtc gttgggcagc ccgatgacag 7320
cgaccacgct cttgaagccc tgtgcctcca gggacttcag caggtgggtg tagagcgtgg 7380
agcccagtcc cgtccgctgg tggcgggggg agacgtacac gctcgactcg gccgtccagt 7440
cgtaggcgtt gcgtgccttc caggggcccg cgtaggcgat gccggcgacc tcgccgtcca 7500
cctcggcgac gagccaggga tagcgctccc gcagacggac gaggtcgtcc gtccactcct 7560
gcggttcctg cggctcggta cggaagttga ccgtgcttgt ctcgatgtag tggttgacga 7620
tggtgcagac cgccggcatg tccgcctcgg tggcacggcg gatgtcggcc gggcgtcgtt 7680
ctgggctcat ggtagactcg acggatccac gtgtggaaga tatgaatttt tttgagaaac 7740
tagataagat taatgaatat cggtgttttg gtttttttctt gtggccgtct ttgtttatat 7800
tgagattttt caaatcagtg cgcaagacgt gacgtaagta tccgagtcag tttttatttt 7860
tctactaatt tggtcgaagc tttgggcgga tcctctagag aattcgaatc caaaaattac 7920
ggatatgaat ataggcatat ccgtatccga attatccgtt tgacagctag caacgattat 7980
acaattgctt ctttaaaaaa ggaagaaaga aagaaagaaa agaatcaaca tcagcgttaa 8040
caaacggccc cgttacggcc caaacggtca tatagagtaa cggcgttaag cgttgaaaga 8100
ctcctatcga aatacgtaac cgcaaacgtg tcatagtcag atcccctctt ccttcaccgc 8160
ctcaaacaca aaaataatct tctacagcct atatatacaa cccccccttc tatctctcct 8220
ttctcacaat tcatcatctt tctttctcta cccccaattt taagaaatcc tctcttctcc 8280
tcttcatttt caaggtaaat ctctctctct ctctctctct ctgttattcc ttgttttaat 8340

taggtatgta ttattgctag tttgttaatc tgcttatctt atgtatgcct tatgtgaata 8400
tctttatctt gttcatctca tccgtttaga agctataaat ttgttgattt gactgtgtat 8460
ctacacgtgg ttatgtttat atctaatcag atatgaattt cttcatattg ttgcgtttgt 8520
gtgtaccaat ccgaaatcgt tgattttttt catttaatcg tgtagctaat tgtacgtata 8580
catatggatc tacgtatcaa ttgttcatct gtttgtgttt gtatgtatac agatctgaaa 8640
acatcacttc tctcatctga ttgtgttgtt acatacatag atatagatct gttatatcat 8700
tttttttatt aattgtgtat atatatatgt gcatagatct ggattacatg attgtgatta 8760
tttacatgat tttgttattt acgtatgtat atatgtagat ctggactttt tggagttgtt 8820
gacttgattg tatttgtgtg tgtatatgtg tgttctgatc ttgatatgtt atgtatgtgc 8880
agccc 8885

<210> 18
<211> 9525
<212> DNA
<213> Artificial

<220>
<223> Binary vector 1bxPcUbisnoRNA

<400> 18
cccgggtacc gtcgacttca tcttcttctt ctggtgttaa aacttttgtt tttggtaaaa 60
gtaatggaca gtgacgattg atattaaggt ctcgttcgtc gtaaaaacgg ccgtaaaaag 120
cctatttgcc gaccattctg ttatatcacc acccatattc tgagtcctat tgatttttgt 180
tttttcaatc tttttctaat cgattttttg gccaatgatt taatcaaaat tgacaagcat 240
atgtctgaat taatccttgt ttgataaact ctaatcttct ctatgaggcc tttttcaat 300
tacctttcaa atccgatttc gtatttattt ctttggttct gtttttggtt gatttagtgg 360
cctatgattt aaatgattat agacaagcat atgtctgaat taatccttgt tgataaatct 420
aatcttctct atgaggtcta aatccgattt tgtttttttgg ttattggttt taaaattgtc 480
ttggatttat ttgatgagaa aagaggaaat tgaaaggcat caagtgatga ttgataacgc 540
atagttcaga tgataaattc tattatgatt aacatcttca gcatagtctt tcgctcctat 600
ctgatgaccc tttcctctct attttcatct attatccaaa tttttcaatc tcaaattgtt 660
ccttaagtct cgagtgtaca gagctccgaa tttccccgat cgttcaaaca tttggcaata 720
aagtttctta agattgaatc ctgttgccgg tcttgcgatg attatcatat aatttctgtt 780
gaattacgtt aagcatgtaa taattaacat gtaatgcatg acgttattta tgagatgggt 840
ttttatgatt agagtcccgc aattatacat ttaatacgcg atagaaaaca aaatatagcg 900
cgcaaactag gataaattat cgcgcgcggt gtcatctatg ttactagatc gggaattggc 960
atgcaagctt ggcactggcc gtcgttttac aacgtcgtga ctgggaaaac cctggcgtta 1020
cccaacttaa tcgccttgca gcacatcccc ctttcgccag ctggcgtaat agcgaagagg 1080
cccgcaccga tcgcccttcc caacagttgc gcagcctgaa tggcgaatgc tagagcagct 1140
tgagcttgga tcagattgtc gtttcccgcc ttcagtttaa actatcagtg tttgacagga 1200
tatattggcg ggtaaaccta agagaaaaga gcgtttatta gaataacgga tatttaaaag 1260
ggcgtgaaaa ggtttatccg ttcgtccatt tgtatgtgca tgccaaccac agggttcccc 1320
tcgggatcaa agtactttga tccaacccct ccgctgctat agtgcagtcg gcttctgacg 1380
ttcagtgcag ccgtcttctg aaaacgacat gtcgcacaag tcctaagtta cgcgacaggc 1440
tgccgccctg cccttttcct ggcgttttct tgtcgcgtgt tttagtcgca taaagtagaa 1500

tacttgcgac tagaaccgga gacattacgc catgaacaag agcgccgccg ctggcctgct 1560
gggctatgcc cgcgtcagca ccgacgacca ggacttgacc aaccaacggg ccgaactgca 1620
cgcggccggc tgcaccaagc tgttttccga gaagatcacc ggcaccaggc gcgaccgccc 1680
ggagctggcc aggatgcttg accacctacg ccctggcgac gttgtgacag tgaccaggct 1740
agaccgcctg gcccgcagca cccgcgacct actggacatt gccgagcgca tccaggaggc 1800
cggcgcgggc ctgcgtagcc tggcagagcc gtgggccgac accaccacgc cggccggccg 1860
catggtgttg accgtgttcg ccggcattgc cgagttcgag cgttccctaa tcatcgaccg 1920
cacccggagc gggcgcgagg ccgccaaggc ccgaggcgtg aagtttggcc cccgccctac 1980
cctcaccccg gcacagatcg cgcacgcccg cgagctgatc gaccaggaag gccgcaccgt 2040
gaaagaggcg gctgcactgc ttggcgtgca tcgctcgacc ctgtaccgcg cacttgagcg 2100
cagcgaggaa gtgacgccca ccgaggccag gcggcgcggt gccttccgtg aggacgcatt 2160
gaccgaggcc gacgccctgg cggccgccga gaatgaacgc caagaggaac aagcatgaaa 2220
ccgcaccagg acggccagga cgaaccgttt ttcattaccg aagagatcga ggcggagatg 2280
atcgcggccg ggtacgtgtt cgagccgccc gcgcacgtct caaccgtgcg gctgcatgaa 2340
atcctggccg gtttgtctga tgccaagctg gcggcctggc cggccagctt ggccgctgaa 2400
gaaaccgagc gccgccgtct aaaaaggtga tgtgtatttg agtaaaacag cttgcgtcat 2460
gcggtcgctg cgtatatgat gcgatgagta aataaacaaa tacgcaaggg gaacgcatga 2520
aggttatcgc tgtacttaac cagaaaggcg ggtcaggcaa gacgaccatc gcaacccatc 2580
tagcccgcgc cctgcaactc gccggggccg atgttctgtt agtcgattcc gatccccagg 2640
gcagtgcccg cgattgggcg gccgtgcggg aagatcaacc gctaaccgtt gtcggcatcg 2700
accgcccgac gattgaccgc gacgtgaagg ccatcggccg gcgcgacttc gtagtgatcg 2760
acggagcgcc ccaggcggcg gacttggctg tgtccgcgat caaggcagcc gacttcgtgc 2820
tgattccggt gcagccaagc ccttacgaca tatgggccac cgccgacctg gtggagctgg 2880
ttaagcagcg cattgaggtc acggatggaa ggctacaagc ggcctttgtc gtgtcgcggg 2940
cgatcaaagg cacgcgcatc ggcggtgagg ttgccgaggc gctggccggg tacgagctgc 3000
ccattcttga gtcccgtatc acgcagcgcg tgagctaccc aggcactgcc gccgccggca 3060
caaccgttct tgaatcagaa cccgagggcg acgctgcccg cgaggtccag gcgctggccg 3120
ctgaaattaa atcaaaactc atttgagtta atgaggtaaa gagaaaatga gcaaaagcac 3180
aaacacgcta agtgccggcc gtccgagcgc acgcagcagc aaggctgcaa cgttggccag 3240
cctggcagac acgccagcca tgaagcgggt caactttcag ttgccggcgg aggatcacac 3300
caagctgaag atgtacgcgg tacgccaagg caagaccatt accgagctgc tatctgaata 3360
catcgcgcag ctaccagagt aaatgagcaa atgaataaat gagtagatga attttagcgg 3420
ctaaaggagg cggcatggaa aatcaagaac aaccaggcac cgacgccgtg gaatgcccca 3480
tgtgtggagg aacgggcggt tggccaggcg taagcggctg ggttgtctgc cggccctgca 3540
atggcactgg aacccccaag cccgaggaat cggcgtgacg gtcgcaaacc atccggcccg 3600
gtacaaatcg gcgcggcgct gggtgatgac ctggtggaga agttgaaggc cgcgcaggcc 3660
gcccagcggc aacgcatcga ggcagaagca cgccccggtg aatcgtggca agcggccgct 3720
gatcgaatcc gcaaagaatc ccggcaaccg ccggcagccg gtgcgccgtc gattaggaag 3780
ccgcccaagg gcgacgagca accagatttt ttcgttccga tgctctatga cgtgggcacc 3840
cgcgatagtc gcagcatcat ggacgtggcc gttttccgtc tgtcgaagcg tgaccgacga 3900
gctggcgagg tgatccgcta cgagcttcca gacgggcacg tagaggtttc cgcagggccg 3960
gccggcatgg ccagtgtgtg ggattacgac ctggtactga tggcggtttc ccatctaacc 4020
gaatccatga accgataccg ggaagggaag ggagacaagc ccggccgcgt gttccgtcca 4080
cacgttgcgg acgtactcaa gttctgccgg cgagccgatg gcggaaagca gaaagacgac 4140
ctggtagaaa cctgcattcg gttaaacacc acgcacgttg ccatgcagcg tacgaagaag 4200

gccaagaacg gccgcctggt gacggtatcc gagggtgaag ccttgattag ccgctacaag 4260
atcgtaaaga gcgaaaccgg gcggccggag tacatcgaga tcgagctagc tgattggatg 4320
taccgcgaga tcacagaagg caagaacccg gacgtgctga cggttcaccc cgattacttt 4380
ttgatcgatc ccggcatcgg ccgttttctc taccgcctgg cacgccgcgc cgcaggcaag 4440
gcagaagcca gatggttgtt caagacgatc tacgaacgca gtggcagcgc cggagagttc 4500
aagaagttct gtttcaccgt gcgcaagctg atcgggtcaa atgacctgcc ggagtacgat 4560
ttgaaggagg aggcggggca ggctggcccg atcctagtca tgcgctaccg caacctgatc 4620
gagggcgaag catccgccgg ttcctaatgt acggagcaga tgctagggca aattgcccta 4680
gcaggggaaa aaggtcgaaa aggtctcttt cctgtggata gcacgtacat tgggaaccca 4740
aagccgtaca ttgggaaccg gaacccgtac attgggaacc caaagccgta cattgggaac 4800
cggtcacaca tgtaagtgac tgatataaaa gagaaaaaag gcgatttttc cgcctaaaac 4860
tctttaaaac ttattaaaac tcttaaaacc cgcctggcct gtgcataact gtctggccag 4920
cgcacagccg aagagctgca aaaagcgcct acccttcggt cgctgcgctc cctacgcccc 4980
gccgcttcgc gtcggcctat cgcggccgct ggccgctcaa aaatggctgg cctacggcca 5040
ggcaatctac cagggcgcgg acaagccgcg ccgtcgccac tcgaccgccg gcgcccacat 5100
caaggcaccc tgcctcgcgc gtttcggtga tgacggtgaa aacctctgac acatgcagct 5160
cccggagacg gtcacagctt gtctgtaagc ggatgccggg agcagacaag cccgtcaggg 5220
cgcgtcagcg ggtgttggcg ggtgtcgggg cgcagccatg acccagtcac gtagcgatag 5280
cggagtgtat actggcttaa ctatgcggca tcagagcaga ttgtactgag agtgcaccat 5340
atgcggtgtg aaataccgca cagatgcgta aggagaaaat accgcatcag gcgctcttcc 5400
gcttcctcgc tcactgactc gctgcgctcg gtcgttcggc tgcggcgagc ggtatcagct 5460
cactcaaagg cggtaatacg gttatccaca gaatcagggg ataacgcagg aaagaacatg 5520
tgagcaaaag gccagcaaaa ggccaggaac cgtaaaaagg ccgcgttgct ggcgtttttc 5580
cataggctcc gcccccctga cgagcatcac aaaaatcgac gctcaagtca gaggtggcga 5640
aacccgacag gactataaag ataccaggcg tttccccctg gaagctccct cgtgcgctct 5700
cctgttccga ccctgccgct taccggatac ctgtccgcct ttctcccttc gggaagcgtg 5760
gcgctttctc atagctcacg ctgtaggtat ctcagttcgg tgtaggtcgt tcgctccaag 5820
ctgggctgtg tgcacgaacc ccccgttcag cccgaccgct gcgccttatc cggtaactat 5880
cgtcttgagt ccaacccggt aagacacgac ttatcgccac tggcagcagc cactggtaac 5940
aggattagca gagcgaggta tgtaggcggt gctacagagt tcttgaagtg gtggcctaac 6000
tacggctaca ctagaaggac agtatttggt atctgcgctc tgctgaagcc agttaccttc 6060
ggaaaaagag ttggtagctc ttgatccggc aaacaaacca ccgctggtag cggtggtttt 6120
tttgtttgca agcagcagat tacgcgcaga aaaaaaggat ctcaagaaga tcctttgatc 6180
ttttctacgg ggtctgacgc tcagtggaac gaaaactcac gttaagggat tttggtcatg 6240
cattctaggt actaaaacaa ttcatccagt aaaatataat attttatttt ctcccaatca 6300
ggcttgatcc ccagtaagtc aaaaaatagc tcgacatact gttcttcccc gatatcctcc 6360
ctgatcgacc ggacgcagaa ggcaatgtca taccacttgt ccgccctgcc gcttctccca 6420
agatcaataa agccacttac tttgccatct ttcacaaaga tgttgctgtc tcccaggtcg 6480
ccgtgggaaa agacaagttc ctcttcgggc ttttccgtct ttaaaaaatc atacagctcg 6540
cgcggatctt taaatggagt gtcttcttcc cagttttcgc aatccacatc ggccagatcg 6600
ttattcagta agtaatccaa ttcggctaag cggctgtcta agctattcgt atagggacaa 6660
tccgatatgt cgatggagtg aaagagcctg atgcactccg catacagctc gataatcttt 6720
tcagggcttt gttcatcttc atactcttcc gagcaaagga cgccatcggc ctcactcatg 6780
agcagattgc tccagccatc atgccgttca aagtgcagga cctttggaac aggcagcttt 6840
ccttccagcc atagcatcat gtccttttcc cgttccacat cataggtggt ccctttatac 6900

cggctgtccg tcatttttaa atataggttt tcattttctc ccaccagctt atatacctta 6960
gcaggagaca ttccttccgt atcttttacg cagcggtatt tttcgatcag ttttttcaat 7020
tccggtgata ttctcatttt agccatttat tatttccttc ctcttttcta cagtatttaa 7080
agatacccca agaagctaat tataacaaga cgaactccaa ttcactgttc cttgcattct 7140
aaaaccttaa ataccagaaa acagcttttt caaagttgtt ttcaaagttg gcgtataaca 7200
tagtatcgac ggagccgatt ttgaaaccgc ggtgatcaca ggcagcaacg ctctgtcatc 7260
gttacaatca acatgctacc ctccgcgaga tcatccgtgt ttcaaacccg gcagcttagt 7320
tgccgttctt ccgaatagca tcggtaacat gagcaaagtc tgccgcctta caacggctct 7380
cccgctgacg ccgtcccgga ctgatgggct gcctgtatcg agtggtgatt ttgtgccgag 7440
ctgccggtcg gggagctgtt ggctggctgg tggcaggata tattgtggtg taaacaaatt 7500
gacgcttaga caacttaata acacattgcg gacgttttta atgtactgaa ttaacgccga 7560
attaagcttg gacaatcagt aaattgaacg gagaatatta ttcataaaaa tacgatagta 7620
acgggtgata tattcattag aatgaaccga aaccggcggt aaggatctga gctacacatg 7680
ctcaggtttt ttacaacgtg cacaacagaa ttgaaagcaa atatcatgcg atcataggcg 7740
tctcgcatat ctcattaaag cagggcatgc cggtcgagtc aaatctcggt gacgggcagg 7800
accggacggg gccgtaccgg caggctgaag tccagctgcc agaaacccac gtcatgccag 7860
ttcccgtgct tgaagccggc cgcccgcagc atgccgcggg gggcatatcc gagcgcctcg 7920
tgcatgcgca cgctcgggtc gttgggcagc ccgatgacag cgaccacgct cttgaagccc 7980
tgtgcctcca gggacttcag caggtgggtg tagagcgtgg agcccagtcc cgtccgctgg 8040
tggcgggggg agacgtacac gctcgactcg gccgtccagt cgtaggcgtt gcgtgccttc 8100
caggggcccg cgtaggcgat gccggcgacc tcgccgtcca cctcggcgac gagccaggga 8160
tagcgctccc gcagacggac gaggtcgtcc gtccactcct gcggttcctg cggctcggta 8220
cggaagttga ccgtgcttgt ctcgatgtag tggttgacga tggtgcagac cgccggcatg 8280
tccgcctcgg tggcacggcg gatgtcggcc gggcgtcgtt ctgggctcat ggtagactcg 8340
acggatccac gtgtggaaga tatgaatttt tttgagaaac tagataagat taatgaatat 8400
cggtgttttg gttttttctt gtggccgtct ttgtttatat tgagattttt caaatcagtg 8460
cgcaagacgt gacgtaagta tccgagtcag tttttatttt tctactaatt tggtcgaagc 8520
tttgggcgga tcctctagag aattcgaatc caaaaattac ggatatgaat ataggcatat 8580
ccgtatccga attatccgtt tgacagctag caacgattat acaattgctt ctttaaaaaa 8640
ggaagaaaga aagaaagaaa agaatcaaca tcagcgttaa caaacggccc cgttacggcc 8700
caaacggtca tatagagtaa cggcgttaag cgttgaaaga ctcctatcga aatacgtaac 8760
cgcaaacgtg tcatagtcag atcccctctt ccttcaccgc ctcaaacaca aaaataatct 8820
tctacagcct atatatacaa cccccccttc tatctctcct ttctcacaat tcatcatctt 8880
tctttctcta cccccaattt taagaaatcc tctcttctcc tcttcatttt caaggtaaat 8940
ctctctctct ctctctctct ctgttattcc ttgttttaat taggtatgta ttattgctag 9000
tttgttaatc tgcttatctt atgtatgcct tatgtgaata tctttatctt gttcatctca 9060
tccgtttaga agctataaat ttgttgattt gactgtgtat ctacacgtgg ttatgtttat 9120
atctaatcag atatgaattt cttcatattg ttgcgtttgt gtgtaccaat ccgaaatcgt 9180
tgattttttt catttaatcg tgtagctaat tgtacgtata catatggatc tacgtatcaa 9240
ttgttcatct gtttgtgttt gtatgtatac agatctgaaa acatcacttc tctcatctga 9300
ttgtgttgtt acatacatag atatagatct gttatatcat ttttttatt aattgtgtat 9360
atatatatgt gcatagatct ggattacatg attgtgatta tttacatgat tttgttattt 9420
acgtatgtat atatgtagat ctggactttt tggagttgtt gacttgattg tatttgtgtg 9480
tgtatatgtg tgttctgatc ttgatatgtt atgtatgtgc agccc 9525

<210> 19
<211> 9080
<212> DNA
<213> Artificial

<220>
<223> Binary vector lbxPcUbitRNA

<400> 19
cccgggtacc gtcgacagac aaagaacact gcaatctgtg gtgctgctgg actgctgatg 60
ttgaatctat tcattggccg tgtctaaaat acaatatttt ggattagatt ctaaactggt 120
acatgcaaca gaacaagcac ctgaggttct atggtgtagt ggttagcact ctggactttg 180
aatccagcga cctgggttcg actcccggta ggacctctca tgcctcgagt gtacagagct 240
ccgaatttcc ccgatcgttc aaacatttgg caataaagtt tcttaagatt gaatcctgtt 300
gccggtcttg cgatgattat catataattt ctgttgaatt acgttaagca tgtaataatt 360
aacatgtaat gcatgacgtt atttatgaga tgggttttta tgattagagt cccgcaatta 420
tacatttaat acgcgataga aaacaaaata tagcgcgcaa actaggataa attatcgcgc 480
gcggtgtcat ctatgttact agatcgggaa ttggcatgca agcttggcac tggccgtcgt 540
tttacaacgt cgtgactggg aaaaccctgg cgttacccaa cttaatcgcc ttgcagcaca 600
tccccctttc gccagctggc gtaatagcga agaggcccgc accgatcgcc cttcccaaca 660
gttgcgcagc ctgaatggcg aatgctagag cagcttgagc ttggatcaga ttgtcgtttc 720
ccgccttcag tttaaactat cagtgtttga caggatatat tggcgggtaa acctaagaga 780
aaagagcgtt tattagaata acggatattt aaaagggcgt gaaaaggttt atccgttcgt 840
ccatttgtat gtgcatgcca accacagggt tcccctcggg atcaaagtac tttgatccaa 900
cccctccgct gctatagtgc agtcggcttc tgacgttcag tgcagccgtc ttctgaaaac 960
gacatgtcgc acaagtccta agttacgcga caggctgccg ccctgccctt ttcctggcgt 1020
tttcttgtcg cgtgttttag tcgcataaag tagaatactt gcgactagaa ccggagacat 1080
tacgccatga acaagagcgc cgccgctggc ctgctgggct atgcccgcgt cagcaccgac 1140
gaccaggact tgaccaacca acgggccgaa ctgcacgcgg ccggctgcac caagctgttt 1200
tccgagaaga tcaccggcac caggcgcgac cgcccggagc tggccaggat gcttgaccac 1260
ctacgccctg gcgacgttgt gacagtgacc aggctagacc gcctggcccg cagcacccgc 1320
gacctactgg acattgccga gcgcatccag gaggccggcg cgggcctgcg tagcctggca 1380
gagccgtggg ccgacaccac cacgccggcc ggccgcatgg tgttgaccgt gttcgccggc 1440
attgccgagt tcgagcgttc cctaatcatc gaccgcaccc ggagcgggcg cgaggccgcc 1500
aaggcccgag gcgtgaagtt tggcccccgc cctaccctca ccccggcaca gatcgcgcac 1560
gcccgcgagc tgatcgacca ggaaggccgc accgtgaaag aggcggctgc actgcttggc 1620
gtgcatcgct cgaccctgta ccgcgcactt gagcgcagcg aggaagtgac gcccaccgag 1680
gccaggcggc gcggtgcctt ccgtgaggac gcattgaccg aggccgacgc cctggcggcc 1740
gccgagaatg aacgccaaga ggaacaagca tgaaaccgca ccaggacggc caggacgaac 1800
cgtttttcat taccgaagag atcgaggcgg agatgatcgc ggccgggtac gtgttcgagc 1860
cgcccgcgca cgtctcaacc gtgcggctgc atgaaatcct ggccggtttg tctgatgcca 1920
agctggcggc ctggccggcc agcttggccg ctgaagaaac cgagcgccgc cgtctaaaaa 1980
ggtgatgtgt atttgagtaa aacagcttgc gtcatgcggt cgctgcgtat atgatgcgat 2040
gagtaaataa acaaatacgc aaggggaacg catgaaggtt atcgctgtac ttaaccagaa 2100
aggcgggtca ggcaagacga ccatcgcaac ccatctagcc cgcgccctgc aactcgccgg 2160

ggccgatgtt ctgttagtcg attccgatcc ccagggcagt gcccgcgatt gggcggccgt 2220
gcgggaagat caaccgctaa ccgttgtcgg catcgaccgc ccgacgattg accgcgacgt 2280
gaaggccatc ggccggcgcg acttcgtagt gatcgacgga gcgccccagg cggcggactt 2340
ggctgtgtcc gcgatcaagg cagccgactt cgtgctgatt ccggtgcagc caagccctta 2400
cgacatatgg gccaccgccg acctggtgga gctggttaag cagcgcattg aggtcacgga 2460
tggaaggcta caagcggcct ttgtcgtgtc gcgggcgatc aaaggcacgc gcatcggcgg 2520
tgaggttgcc gaggcgctgg ccgggtacga gctgcccatt cttgagtccc gtatcacgca 2580
gcgcgtgagc tacccaggca ctgccgccgc cggcacaacc gttcttgaat cagaacccga 2640
gggcgacgct gcccgcgagg tccaggcgct ggccgctgaa attaaatcaa aactcatttg 2700
agttaatgag gtaaagagaa aatgagcaaa agcacaaaca cgctaagtgc cggccgtccg 2760
agcgcacgca gcagcaaggc tgcaacgttg gccagcctgg cagacacgcc agccatgaag 2820
cgggtcaact ttcagttgcc ggcggaggat cacaccaagc tgaagatgta cgcggtacgc 2880
caaggcaaga ccattaccga gctgctatct gaatacatcg cgcagctacc agagtaaatg 2940
agcaaatgaa taaatgagta gatgaatttt agcggctaaa ggaggcggca tggaaaatca 3000
agaacaacca ggcaccgacg ccgtggaatg ccccatgtgt ggaggaacgg gcggttggcc 3060
aggcgtaagc ggctgggttg tctgccggcc ctgcaatggc actggaaccc ccaagcccga 3120
ggaatcggcg tgacggtcgc aaaccatccg gcccggtaca aatcggcgcg gcgctgggtg 3180
atgacctggt ggagaagttg aaggccgcgc aggccgccca gcggcaacgc atcgaggcag 3240
aagcacgccc cggtgaatcg tggcaagcgg ccgctgatcg aatccgcaaa gaatcccggc 3300
aaccgccggc agccggtgcg ccgtcgatta ggaagccgcc caagggcgac gagcaaccag 3360
atttttcgt tccgatgctc tatgacgtgg gcacccgcga tagtcgcagc atcatggacg 3420
tggccgtttt ccgtctgtcg aagcgtgacc gacgagctgg cgaggtgatc cgctacgagc 3480
ttccagacgg gcacgtagag gtttccgcag ggccggccgg catggccagt gtgtgggatt 3540
acgacctggt actgatggcg gtttcccatc taaccgaatc catgaaccga taccgggaag 3600
ggaagggaga caagcccggc cgcgtgttcc gtccacacgt tgcggacgta ctcaagttct 3660
gccggcgagc cgatggcgga aagcagaaag acgacctggt agaaacctgc attcggttaa 3720
acaccacgca cgttgccatg cagcgtacga agaaggccaa gaacggccgc ctggtgacgg 3780
tatccgaggg tgaagccttg attagccgct acaagatcgt aaagagcgaa accgggcggc 3840
cggagtacat cgagatcgag ctagctgatt ggatgtaccg cgagatcaca gaaggcaaga 3900
acccggacgt gctgacggtt caccccgatt actttttgat cgatcccggc atcggccgtt 3960
ttctctaccg cctggcacgc cgcgccgcag gcaaggcaga agccagatgg ttgttcaaga 4020
cgatctacga acgcagtggc agcgccggag agttcaagaa gttctgtttc accgtgcgca 4080
agctgatcgg gtcaaatgac ctgccggagt acgatttgaa ggaggaggcg gggcaggctg 4140
gcccgatcct agtcatgcgc taccgcaacc tgatcgaggg cgaagcatcc gccggttcct 4200
aatgtacgga gcagatgcta gggcaaattg ccctagcagg ggaaaaaggt cgaaaaggtc 4260
tctttcctgt ggatagcacg tacattggga acccaaagcc gtacattggg aaccggaacc 4320
cgtacattgg gaacccaaag ccgtacattg ggaaccggtc acacatgtaa gtgactgata 4380
taaaagagaa aaaaggcgat ttttccgcct aaaactcttt aaaacttatt aaaactctta 4440
aaacccgcct ggcctgtgca taactgtctg gccagcgcac agccgaagag ctgcaaaaag 4500
cgcctaccct tcggtcgctg cgctccctac gccccgccgc ttcgcgtcgg cctatcgcgg 4560
ccgctggccg ctcaaaaatg gctggcctac ggccaggcaa tctaccaggg cgcggacaag 4620
ccgcgccgtc gccactcgac cgccggcgcc cacatcaagg caccctgcct cgcgcgtttc 4680
ggtgatgacg gtgaaaacct ctgacacatg cagctcccgg agacggtcac agcttgtctg 4740
taagcggatg ccgggagcag acaagcccgt cagggcgcgt cagcgggtgt tggcgggtgt 4800
cggggcgcag ccatgaccca gtcacgtagc gatagcggag tgtatactgg cttaactatg 4860

cggcatcaga gcagattgta ctgagagtgc accatatgcg gtgtgaaata ccgcacagat 4920
gcgtaaggag aaaataccgc atcaggcgct cttccgcttc ctcgctcact gactcgctgc 4980
gctcggtcgt tcggctgcgg cgagcggtat cagctcactc aaaggcggta atacggttat 5040
ccacagaatc aggggataac gcaggaaaga acatgtgagc aaaaggccag caaaaggcca 5100
ggaaccgtaa aaaggccgcg ttgctggcgt ttttccatag gctccgcccc cctgacgagc 5160
atcacaaaaa tcgacgctca agtcagaggt ggcgaaaccc gacaggacta taaagatacc 5220
aggcgtttcc ccctggaagc tccctcgtgc gctctcctgt tccgaccctg ccgcttaccg 5280
gatacctgtc cgcctttctc ccttcgggaa gcgtggcgct ttctcatagc tcacgctgta 5340
ggtatctcag ttcggtgtag gtcgttcgct ccaagctggg ctgtgtgcac gaaccccccg 5400
ttcagcccga ccgctgcgcc ttatccggta actatcgtct tgagtccaac ccggtaagac 5460
acgacttatc gccactggca gcagccactg gtaacaggat tagcagagcg aggtatgtag 5520
gcggtgctac agagttcttg aagtggtggc ctaactacgg ctacactaga aggacagtat 5580
ttggtatctg cgctctgctg aagccagtta ccttcggaaa aagagttggt agctcttgat 5640
ccggcaaaca aaccaccgct ggtagcggtg gtttttttgt ttgcaagcag cagattacgc 5700
gcagaaaaaa aggatctcaa gaagatcctt tgatcttttc tacggggtct gacgctcagt 5760
ggaacgaaaa ctcacgttaa gggattttgg tcatgcattc taggtactaa aacaattcat 5820
ccagtaaaat ataatatttt attttctccc aatcaggctt gatccccagt aagtcaaaaa 5880
atagctcgac atactgttct tccccgatat cctccctgat cgaccggacg cagaaggcaa 5940
tgtcatacca cttgtccgcc ctgccgcttc tcccaagatc aataaagcca cttactttgc 6000
catctttcac aaagatgttg ctgtctccca ggtcgccgtg ggaaaagaca agttcctctt 6060
cgggcttttc cgtctttaaa aaatcataca gctcgcgcgg atctttaaat ggagtgtctt 6120
cttcccagtt ttcgcaatcc acatcggcca gatcgttatt cagtaagtaa tccaattcgg 6180
ctaagcggct gtctaagcta ttcgtatagg gacaatccga tatgtcgatg gagtgaaaga 6240
gcctgatgca ctccgcatac agctcgataa tcttttcagg gctttgttca tcttcatact 6300
cttccgagca aaggacgcca tcggcctcac tcatgagcag attgctccag ccatcatgcc 6360
gttcaaagtg caggaccttt ggaacaggca gctttccttc cagccatagc atcatgtcct 6420
tttcccgttc cacatcatag gtggtccctt tataccggct gtccgtcatt tttaaatata 6480
ggttttcatt ttctcccacc agcttatata ccttagcagg agacattcct tccgtatctt 6540
ttacgcagcg gtatttttcg atcagttttt tcaattccgg tgatattctc attttagcca 6600
tttattattt ccttcctctt ttctacagta tttaaagata ccccaagaag ctaattataa 6660
caagacgaac tccaattcac tgttccttgc attctaaaac cttaaatacc agaaaacagc 6720
tttttcaaag ttgttttcaa agttggcgta taacatagta tcgacggagc cgattttgaa 6780
accgcggtga tcacaggcag caacgctctg tcatcgttac aatcaacatg ctaccctccg 6840
cgagatcatc cgtgtttcaa acccggcagc ttagttgccg ttcttccgaa tagcatcggt 6900
aacatgagca aagtctgccg ccttacaacg gctctcccgc tgacgccgtc ccggactgat 6960
gggctgcctg tatcgagtgg tgattttgtg ccgagctgcc ggtcggggag ctgttggctg 7020
gctggtggca ggatatattg tggtgtaaac aaattgacgc ttagacaact taataacaca 7080
ttgcggacgt ttttaatgta ctgaattaac gccgaattaa gcttggacaa tcagtaaatt 7140
gaacggagaa tattattcat aaaaatacga tagtaacggg tgatatattc attagaatga 7200
accgaaaccg gcggtaagga tctgagctac acatgctcag gtttttacaa acgtgcacaa 7260
cagaattgaa agcaaatatc atgcgatcat aggcgtctcg catatctcat taaagcaggg 7320
catgccggtc gagtcaaatc tcggtgacgg gcaggaccgg acggggccgt accggcaggc 7380
tgaagtccag ctgccagaaa cccacgtcat gccagttccc gtgcttgaag ccggccgccc 7440
gcagcatgcc gcggggggca tatccgagcg cctcgtgcat gcgcacgctc gggtcgttgg 7500
gcagcccgat gacagcgacc acgctcttga agccctgtgc ctccagggac ttcagcaggt 7560

```
gggtgtagag cgtggagccc agtcccgtcc gctggtggcg gggggagacg tacacgctcg   7620
actcggccgt ccagtcgtag gcgttgcgtg ccttccaggg gcccgcgtag gcgatgccgg   7680
cgacctcgcc gtccacctcg gcgacgagcc agggatagcg ctcccgcaga cggacgaggt   7740
cgtccgtcca ctcctgcggt tcctgcggct cggtacggaa gttgaccgtg cttgtctcga   7800
tgtagtggtt gacgatggtg cagaccgccg gcatgtccgc ctcggtggca cggcggatgt   7860
cggccgggcg tcgttctggg ctcatggtag actcgacgga tccacgtgtg gaagatatga   7920
atttttttga gaaactagat aagattaatg aatatcggtg ttttggtttt ttcttgtggc   7980
cgtctttgtt tatattgaga tttttcaaat cagtgcgcaa gacgtgacgt aagtatccga   8040
gtcagttttt atttttctac taatttggtc gaagctttgg gcggatcctc tagagaattc   8100
gaatccaaaa attacggata tgaatatagg catatccgta tccgaattat ccgtttgaca   8160
gctagcaacg attatacaat tgcttcttta aaaaaggaag aaagaaagaa agaaaagaat   8220
caacatcagc gttaacaaac ggccccgtta cggcccaaac ggtcatatag agtaacggcg   8280
ttaagcgttg aaagactcct atcgaaatac gtaaccgcaa acgtgtcata gtcagatccc   8340
ctcttccttc accgcctcaa acacaaaaat aatcttctac agcctatata tacaaccccc   8400
ccttctatct ctcctttctc acaattcatc atctttcttt ctctaccccc aattttaaga   8460
aatcctctct tctcctcttc attttcaagg taaatctctc tctctctctc tctctctgtt   8520
attccttgtt ttaattaggt atgtattatt gctagtttgt taatctgctt atcttatgta   8580
tgccttatgt gaatatcttt atcttgttca tctcatccgt ttagaagcta taaatttgtt   8640
gatttgactg tgtatctaca cgtggttatg tttatatcta atcagatatg aatttcttca   8700
tattgttgcg tttgtgtgta ccaatccgaa atcgttgatt tttttcattt aatcgtgtag   8760
ctaattgtac gtatacatat ggatctacgt atcaattgtt catctgtttg tgtttgtatg   8820
tatacagatc tgaaaacatc acttctctca tctgattgtg ttgttacata catagatata   8880
gatctgttat atcatttttt ttattaattg tgtatatata tatgtgcata gatctggatt   8940
acatgattgt gattatttac atgattttgt tatttacgta tgtatatatg tagatctgga   9000
ctttttggag ttgttgactt gattgtattt gtgtgtgtat atgtgtgttc tgatcttgat   9060
atgttatgta tgtgcagccc                                                9080
```

## Description

## Abstract

The present invention is in the field of genetics, especially plant genetics, and provides agents capable of gene-specific silencing. The present invention specifically provides polycistronic RNA molecules capable to generate double-stranded RNA (dsRNA) agents, methods for utilizing such molecules and cells and organism, especially plants, containing such molecules. The polycistronic RNA molecules, compositions and methods of the invention can be used to enhance performance of agricultural relevant crops and for therapy, prophylaxis, research and diagnostics in diseases and disorders which afflict mammalian species, in which the goal is to reduce or inhibit the function of a selected target polynucleotide sequence. These compositions and methods have utility both in vitro and in vivo.

## Description

### INCORPORATION OF SEQUENCE LISTING

A paper copy of the Sequence Listing and a computer readable form of the sequence listing on diskette, containing the file named " AE20040971.ST25.txt", which is 43,540 bytes in size (measured in MS-DOS), and which was created on 17.01.2005, are herein incorporated by reference.

### FIELD OF THE INVENTION

The present invention is in the field of genetics, especially plant genetics, and provides agents capable of gene-specific silencing. The present invention specifically provides polycicstronic RNA molecules capable to generate double-stranded RNA (dsRNA) agents, methods for utilizing such molecules and cells and organism, especially plants, containing such molecules.

### BACKGROUND OF THE INVENTION

Silencing of genes in plants occurs at both the transcriptional level and post-transcriptional level. Certain of these mechanisms are associated with nucleic acid homology at the DNA or RNA level (Matzke *et al.*, Current Opinion in Genetics and Development, 11:221-227 (2001)).

In 1986 Ecker and Davies reported a method called "antisense" for repressing genes. They expressed a gene in opposite orientation relative to the promoter and found in some transgenic lines repression of the respective endogenous gene. It was speculated, that the antisense mRNA might hybridize with the respective sense mRNA forming a double stranded RNA molecule which is then triggering the breakdown of homologous mRNA (Ecker JR, Davis RW, Poc.Natl.Acad.Sci.USA 83, 5372-5376 (1986)). In 1990 van der Krol and Coworkers reported that over expression of a petunia gene in petunia lead to down regulation of the respective genes both the transgene and the endogenous gene (van der Krol AR *et al.*, Plant Cell 2, 291-299 (1990)). As antisense this method named cosuppression was found not to be very effective for repressing gene expression and many plants had to be screened to find those exhibiting repression.

Double-stranded RNA (dsRNA) molecules can induce sequence-specific posttranscriptional gene silencing, referred to as RNA interference or RNAi (Fire *et al.*, Nature, 391:806-811 (1988)). This term has come to generalize all forms of gene silencing involving dsRNA leading to the sequence-specific reduction of endogenous targeted mRNA levels; unlike co-suppression, in which transgenic DNA leads to silencing of both the transgene and the endogenous gene. Montgomery *et al*. suggests that the primary interference affects of dsRNA are post-transcriptional. (Montgomery *et al.*,

Proc. Natl. Acad. Sci. USA, 1998, 95, 15502-15507). The RNAi phenomenon has its natural role presumably in viral defense and transposon silencing mechanisms.

The first clear evidence that dsRNA could lead to gene silencing in animals came from work in the nematode, *Caenorhabditis elegans.* Injection of just a few molecules of dsRNA per cell was sufficient to completely silence the homologous gene's expression. Furthermore, injection of dsRNA into the gut of the worm caused gene silencing not only throughout the worm, but also in first generation offspring (Fire *et al.*, Nature, 1998, 391, 806-811; Timmons and Fire, Nature 1998, 395, 854; Timmons *et al.*, Gene, 2001, 263, 103-112; PCT publication WO 01/48183). Further work showed that soaking worms in dsRNA was also able to induce silencing (Tabara *et al.*, Science, 1998, 282, 430-431). The same effect was shown in plants that expressed a construct comprising sense and antisense strand under control of the same promoter leading to a RNA molecule which is able to form dsRNA (Waterhouse PM *et al.*, Plant Biology 95, 13959-13964 (1998)). To stabilize dsRNA forming constructs in E. coli and to facilitate cloning of inverted repeats, spacers of random DNA or non repeated regions of the target gene were introduced between the repeated regions.

During RNAi, long dsRNA molecules are cleaved by an RNase III family nuclease call Dicer into 19- to 23-nt RNAs known as short-interfering RNAs (siRNAs). These siRNAs are incorporated into a multicomponent nuclease complex, RISC (the effector nuclease of RNAi), which identifies mRNA substrates through their homology to siRNAs and targets these cognate mRNAs for destruction. In addition, siRNAs can function as primers for an RNA-dependent RNA polymerase that synthesizes additional dsRNA, which in turn is processed into siRNAs, amplifying the effects of the original siRNAs.

Tuschl *et al.* demonstrated that 21- and 22-nt RNA fragments are the sequence-specific mediators of RNAi (Tuschl T, *et al.*, Genes & Dev., 13:3191-3197 (1999)). These fragments, which they termed short interfering RNAs (siRNAs), were shown to be generated by an RNase III-like processing reaction from long dsRNA. They also showed that chemically synthesized siRNA duplexes with overhanging 3' ends mediate efficient target RNA cleavage, and that the cleavage site is located near the center of the region spanned by the guiding siRNA. In addition, they suggest that the direction of dsRNA processing determines whether sense or antisense target RNA can be cleaved by the siRNA-protein complex (Elbashir *et al.*, Genes Dev., 2001, 15, 188-200). Further characterization of the suppression of expression of endogenous and heterologous genes caused by the 21-23 nucleotide siRNAs have been investigated in several mammalian cell lines, including human embryonic kidney (293) and HeLa cells (Elbashir *et al.*, Nature, 2001, 411, 494-498).

mRNA-dependent siRNA incorporation to form dsRNA is carried out by an RNA-dependent RNA polymerase activity (RdRP) (Lipardi *et al.*, Cell, 2001, 107, 297-307). The involvement of an RNA-directed RNA polymerase and siRNA primers is reported by Lipardi *et al.* (Lipardi *et al.*, Cell, 2001, 107, 297-307). This suggests an apparent catalytic nature to the phenomenon. New biochemical and genetic evidence reported by Nishikura *et al.* also shows that an RNA-directed RNA polymerase chain reaction, primed by siRNA, amplifies the interference caused by a small amount of "trigger" dsRNA (Nishikura, Cell, 2001, 107, 415-418). Investigating the role of "trigger" RNA amplification during RNA interference (RNAi) in *Caenorhabditis elegans,* Sijen *et al* revealed a substantial fraction of siRNAs that cannot derive directly from input dsRNA. Instead, a population of siRNAs (termed secondary siRNAs) appeared to derive from the action of the previously reported cellular RNA-directed RNA polymerase (RdRP) on mRNAs that are being targeted by the RNAi mechanism. The distribution of secondary siRNAs exhibited a distinct polarity (5'-3'; on the antisense strand), suggesting a cyclic amplification process in which RdRP is primed by existing siRNAs. This amplification mechanism substantially augmented the potency of RNAi-based surveillance, while ensuring that the RNAi machinery will focus on expressed mRNAs (Sijen *et al.*, Cell, 2001, 107, 465-476).

Several recent publications have described the structural requirements for the dsRNA trigger required for RNAi activity. Recent reports have indicated that ideal dsRNA sequences are 21 nt in length containing 2 nt 3'-end overhangs (Elbashir *et al,* EMBO (2001), 20, 6877-6887, Brantl, Biochimica et Biophysica Acta, 2002, 1575, 15-25.) The inclusion of a 5'-phosphate moiety was shown to enhance activity of siRNA's in vivo in Drosophila embryos (Boutla, *et al.*, Curr. Biol., 2001, 11, 1776-1780). In another study, it was reported that the 5'-phosphate was required for siRNA function in human HeLa cells (Schwarz *et al.*, Molecular Cell, 2002, 10, 537-548). Furthermore, it was shown that the 5'-hydroxyl group of the siRNA is essential as it is phosphorylated for activity while the 3'-hydroxyl group is not essential and tolerates substitute groups such as biotin (Chiu *et al.*, Molecular Cell, 2002, 10, 549-561). It was further shown that bulge structures in one or both of the sense or antisense strands either abolished or severely lowered the activity relative to the unmodified siRNA duplex. Also shown was severe lowering of activity when psoralen was used to cross link an siRNA duplex.

A number of PCT applications have been published that relate to the RNAi phenomenon. These include: PCT publication WO 00/44895; PCT publication WO 00/49035; PCT publication WO 00/63364; PCT publication WO 01/36641; PCT publication WO 01/36646; PCT publication WO 99/32619; PCT publication WO 00/44914; PCT publication WO 01/29058; and PCT publication WO 01/75164.

Recent studies have identified a group of small RNAs known generically as short temporal RNAs (stRNAs) and more broadly as micro-RNAs (miRNAs) in Drosophila, C. *elegans,* and mammals. The miRNAs appear to be transcribed as hairpin RNA precursors, which are processed to their mature, about 21 nt forms by Dicer (Lee RD, and Ambros, V. Science 294: 862-864 (2001)). Thus, it was realized that small, endogenously encoded hairpin RNAs could stably regulate gene expression via elements of the RNAi machinery. Neither stRNAs nor the broader group of miRNAs that has recently been discovered form perfect hairpin structures. Rather, each of these RNAs is predicted to contain several bulged nucleotides within their rather short (about 30 nt) stem structures. Only the let-7 and lin-4 miRNAs have known mRNA targets. In both cases, pairing to binding sites within the regulated transcripts is imperfect, and in the case of lin-4, the presence of a bulged nucleotide is critical to suppression (Ha, I., *et al.*, Genes Dev. 10:3041-3050 (1996)).

Yu et *al.* (PNAS 99:6047-6052 (2002)) demonstrated that short hairpin siRNAs can function like siRNA duplexes to inhibit gene expression in a sequence-specific manner. Inhibition was observed both by the in vitro transcribed hairpin siRNAs by using transfection into mouse P19 cells, and by expression from the U6 promoter. The hairpin siRNAs effectively inhibited RNAs complementary to either the sense or antisense siRNA sequences.

Paddison *et al.* (Genes Dev. 16:948-958 (2002)) have shown that shRNAs can induce sequence-specific gene silencing in mammalian cells. It was found that silencing could be provoked by transfecting exogenously synthesized hairpins into cells, and could also be triggered by endogenous expression of shRNAs. The shRNAs were designed to include bulges within the shRNA stem, and contained nucleotide loops or varying sizes. Paddison *et al.* found that the stem lengths could range anywhere from 25 to 29 nucleotides and loop size could range from 4 to 23 nucleotides without affecting silencing activity.

McManus *et al.* (RNA 8:842-850 (2002)) also studied miRNA mimics containing 19 nucleotides of uninterrupted RNA duplex, a 12-nucleotide loop length and one asymmetric stem-loop bulge composed of a single uridine opposing a double uridine. The loop sequence was chosen from the loop of the mir-26a gene, except that a single C residue was omitted to prevent a predicted alternative nonhairpin structure. The loop was placed on the 5' or 3' end of the antisense strand. McManus *et al*. also showed that gene silencing could be provoked by transfecting exogenously synthesized hairpins into cells, and could also be triggered by endogenous expression of shRNAs.

A number of groups have developed expression vectors to continually express siRNAs in transiently and stably transfected mammalian cells (see, for example, Brummelkamp *et al.* (Science, 296:550-553 (2002)). Some of these vectors have been engineered to express short hairpin RNAs (shRNAs), which get processed in vivo into siRNA-like molecules capable of carrying out gene-specific silencing. The transcript folds into a stem-loop structure with 3' UU-overhangs. The ends of the shRNAs are processed in vivo, converting the shRNAs into about 21 nucleotide siRNA-like molecules, which in turn initiate RNAi.

United States Patent Application 20040198690, United States Patent Application 20040058886 describe the use of dsRNA and siRNA molecules as long as chemically modified variants (comprising polyethers such as polyethylene glycol) and their use in pharmaceutical use.

The method of using dsRNA forming constructs for repression of gene expression in plants was improved by using introns as spacers between the sense and antisense strand instead of random DNA (Varsha Wesley, S. *et al.*, The Plant Journal 27, 581-590 (2001)). The authors discuss the presence of the intron as such to be responsible for increasing the efficiency of silencing as a construct for silencing GUS in rice having an intron 5' prime of the inverted repeat also exhibited increased silencing efficiency compared to a similar construct without intron. United States Patent Application No. 20040214330 discloses that inclusion of an *intron* sequence in the chimeric DNA genes encoding an RNA molecule comprising the hairpin RNA, preferably in the spacer region, and preferably in sense orientation, enhances the efficiency of reduction of expression of the target nucleic acid. In a particularly preferred embodiment, the *intron* is essentially identical in sequence to the *Flaveria trinervia* pyruvate orthophosphate dikinase 2 *intron* 2. United States Patent Application No. 20040029283 also describes intron comprising double stranded RNA constructs and uses thereof.

However, intron sequences are generally quite large and often comprise restriction cleavage sites, which make handling in cloning procedures difficult and often laborious. In addition, splicing of introns leads to joining of the remaining DNA strands leaving a hairpin structure, which is not the natural form of dsRNA. Accordingly, there is a need for alternatives, which provide the gene silencing enhancing effects of introns but do not have their negative features. It is the objective of the present invention to fulfill this need.

## SUMMARY OF THE INVENTION

A first embodiment of the invention relates to a polycistronic RNA molecule capable to provide in an eukaryotic cell an at least partially double-stranded RNA molecule, said polycistronic RNA molecule comprising

a) at least one first ribonucleotide sequence that is substantially identical to at least a part of a target nucleotide sequence of at least one target gene, and

b) at least one second ribonucleotide sequence, which is substantially complementary to said first nucleotide sequence and is capable to hybridize to said first nucleotide sequence to form a double-stranded RNA structure, and

c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

In a preferred embodiment said first and said second ribonucleotide sequence of the polycistronic RNA molecule of the invention are arranged in an antiparallel structure capable to form in an eukaryotic cell a hairpin structure.

Various sequences are suitable for the removable RNA element. Preferably, said third ribonucleotide sequence of the polycistronic RNA molecule of the invention comprises a sequence, which is at least 60%, preferably 80%, more preferably, 90%, most preferably 95% identical to at least one transcribed RNA selected from the group consisting of tRNAs, rRNAs and snoRNAs and their precursor molecules. Preferably, said transcribed RNA is a transcribed RNA of an eukaryotic organism, more preferably of a plant or mammalian organism. Most preferably, said third ribonucleotide sequence comprises a native tRNAs, rRNA or snoRNAs or a precursor molecule thereof from a native plant or a mammal. Most preferred the third ribonucleotide sequence comprises at least one sequences encoded by a sequences selected from the group of

a) the sequences as described by SEQ ID NO: 1, 2, 3, 4, 5, and 6, and

b) a sequences having a identity of at least 60%, preferably 80%, more preferably, 90%, most preferably 95% to a sequence as described by SEQ ID NO: 1, 2, 3, 4, 5, or 6 and comprising the functional elements necessary for RNA processing, and

c) a sequences capable to hybridize (preferably under high stringency conditions) with a sequence as described by SEQ ID NO: 1, 2, 3, 4, 5, or 6 or the complement thereof and comprising the functional elements necessary for RNA processing.

Preferably, the first ribonucleotide sequence is substantially identical to a target gene is selected from the group consisting of eukaryotic endogenous genes, genes of pathogens and transgenes. More preferably, said gene of a pathogen is from a pathogen capable to infect an eukaryotic organism. Most preferably, said pathogen is selected from the group of virus, bacteria, fungi and nematodes.

In one preferred embodiment of the invention, said first ribonucleotide sequence of the polycistronic RNA molecule of the invention hybridizes (preferably under stringent conditions, more preferably under low stringency conditions, most preferably under high stringency conditions) to at least one sequence of the target gene.

In another preferred embodiment, said first ribonucleotide sequence of said polycistronic RNA of the invention has a length of at least 15 nucleotides, preferably at least 19 nucleotides, more preferably a size of about 20 to 500 nucleotides. Preferably, said first nucleotide sequence of the polycistronic RNA of the invention is at least 65% identical to at least one mammalian, nematode, fungal or plant gene (or the complement thereof). More preferably, said first nucleotide sequence is substantially identical to a nucleotide sequence corresponding to the coding sequence or the non-coding of at least one mammalian or plant gene. Said non-coding sequence may be a non-transcribed sequence (such as the promoter sequence).

In one preferred embodiment of the invention, the polycistronic RNA molecule of the invention generates after removal of said third ribonucleotide sequence a short interfering double-stranded RNA, which does not produce a significant PKR-dependent response in the host cells at concentrations effective for attenuating expression of the target gene. Preferably, said generated short-interfering double-stranded RNA is about 15 to about 45 nucleotides in length, preferably 19 to about 50 nucleotides.

Another subject of the inventions relates to a composition for attenuating expression of a target gene, comprising at least one polycistronic RNA of the invention.

Yet another embodiment of the invention relates to a pharmaceutically preparation comprising at least one polycistronic RNA of the invention. Preferably, said preparation gives rise to at least one short interfering double stranded RNA (siRNA) in a mammalian cell and attenuates expression of at least one target gene, which polycistronic RNA does not produce a significant PKR-dependent response in the mammalian cell at concentrations effective for attenuating expression of the target gene.

Another embodiment of the invention relates to an expression construct comprising

a) a promoter functional in eukaryotic cells, and
b) a nucleic acid sequence encoding a polycistronic RNA molecule of any of claim 1 to 14,

wherein said promoter a) is operably linked and heterologous to said nucleic acid sequence b).

Preferably, the expression construct is a DNA molecule, more preferably the expression construct is in a plasmid.

Another embodiment of the invention relates to an expression vector comprising an expression construct of the invention. Preferably, the expression vector is an eukaryotic expression vector. More preferably the eukaryotic expression vector is a viral vector or a plasmid vector.

Yet another embodiment of the invention relates to a transformed cell or non-human organism comprising an expression construct or an expression vector of the invention. Preferably, said expression construct or expression vector is inserted into the genome (preferably the chromosomal or plastid DNA) of said cell or organism. Preferably, said cell or organism is selected from the group of mammalian, bacterial, fungal, nematode or plant cells and organism.

Another embodiment of the invention relates to a method for attenuating (or reducing or suppressing) expression of at least one target gene in an eukaryotic cell, comprising introducing a polycistronic RNA molecule of the invention (or an expression construct or vector encoding the same) into the cells in an amount sufficient to attenuate expression of the target gene, wherein the polycistronic RNA molecule comprises at least one ribonucleotide sequence that is substantially identical to at least a part of the nucleotide sequence of the target gene. Preferably the target gene is an endogenous gene of the cell or a heterologous gene relative to the genome of the cell, such as a pathogen gene. More preferably the heterologous gene is a transgene or a pathogen gene such as a viral, nematode or fungal gene. The polycistronic RNA may be produced outside the cell, or may be recombinantly produced by an expression construct or expression vector within the cell. The cell is preferably eukaryotic cell, more preferably a nematode, mammalian cell or a plant cell. The identity of the first ribonucleic acid sequence of the polycistronic RNA employed in the method of the invention to the target gene is at least about 65%. In one preferred embodiment of the method of the invention, the polycistronic RNA is processed to an short-interfering double-stranded RNA (siRNA) molecule by said host cells. Preferably, the target gene expression is attenuated (or reduced or suppressed) by at least about 10%, preferably at least about 30%, more preferably at least about 50%, even more preferably at least about 70%, most preferably at least about 90%.

Another embodiment of the invention relates to a method for generating short interfering double-stranded RNA (siRNA), comprising:

(i) providing an in vitro transcription system including an expression construct of the invention (i.e., for expression of a polycistronic RNA of the invention), and
(ii) isolating said short interfering double-stranded RNA (siRNA) from said in vitro transcription system.

Any transcription system can be used. For in vitro transcription systems preferably the employed RNA polymerase is a bacteriophage RNA polymerase, more preferably selected from the group consisting of T3 polymerase, T7 polymerase and SP6 polymerase.

Another embodiment of the invention related to the use of the compositions of the invention (i.e., the polycistronic RNA molecule, the expression cassettes, vectors, and recombinant cells or non-human organism of the invention) in the manufacture of a medicament for attenuating expression of one or more genes in vivo. Yet another embodiment of the invention relates to a method for reducing the susceptibility of host cells or host organisms to infection by pathogen, comprising introducing a polycistronic RNA of the invention into said host cells or host organisms in an amount sufficient to attenuate expression of one or more genes necessary for expression by said pathogen. Preferably the pathogen is a virus, a fungus or a nematode.

Another embodiment of the invention related to a method for obtaining pathogen resistant organisms, comprising the steps of providing cells of the organism with an polycistronic RNA molecule of the invention, said polycistronic RNA molecule capable to provide in an eukaryotic cell an at least partially double-stranded RNA molecule, said polycistronic RNA molecule comprising

a) at least one first ribonucleotide sequence that is substantially identical to at least a part of a target nucleotide sequence of at least one target gene, and
b) at least one second ribonucleotide sequence which is substantially complementary to said first nucleotide sequence and is capable to hybridize to said first nucleotide sequence to form a double-stranded RNA structure, and
c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

Preferably in this method said first ribonucleotide sequence has between 65 and 100% sequence identity with at least part of the nucleotide sequence of the genome of a pathogen. More preferably the pathogen is selected from the group of virus, bacteria, fungi, and nematodes.

Additional advantages and novel features of this invention shall be set forth in part in the description and examples that follow, and in part will become apparent to those skilled in the art upon examination of the following or may be learned by the practice of the invention. The objects and the advantages of the invention may be realized and attained by means of the instrumentalities and in combinations particularly pointed out in the appended claims.

## BRIEF DESCRIPTION OF THE DRAWINGS

Fig. 1: Example for the basic principle of the invention. For example the polycistronic RNA molecule may have the schematic structure, 5'-F(1)-(RE)-S(1)-3', wherein F(1) is the first ribonucleotide sequence, S(1) is the second ribonucleotide sequence, and RE is the removable RNA element. This molecule is capable to form a hairpin secondary structure (see Fig. 1 left part) and is processed by removal of the removable RNA into a two-strand double-stranded RNA molecule (without be restricted to the order of the various steps i.e. whether RNA processing (step II in Fig.1) or formation of the double-stranded structure (step I in Fig. 1) occurs first) as schematically shown in Fig. 1.

Fig.2A It is possible first to add the first ribonucleotide sequences (F) of the individual subunits to one another, which is followed by a sequential arrangement of the essentially complementary second ribonucleotide sequences (S). The number of the units n is greater than or equal to two. This gives rise to a structure with a single hairpin. At least one of the linker between said first and said second ribonucleotide sequence is an removable RNA element as described herein. The primary structure of the polycistronic RNA can have for example the basic structure 5'-F(1)-F(2)-.....-F(n)-(RE)-S(n)-....-S(2)-S(1)-3'. These molecules are capable to form a single hairpin secondary structure (see Fig.2A left part). The molecule is processed by removal of the removable RNA into a multiple doublestranded RNA molecules (without be restricted to the order of the various steps i.e. whether RNA processing (step II in Fig.2A) or formation of the double-stranded structure (step I in Fig.2A) occurs first) as schematically shown in Fig. 2A.

Fig.2B It is possible first to add the first ribonucleotide sequence (F) and the essentially complementary second ribonucleotide sequences (S) of the first subunits to one another, which is followed by the sequential arrangement of "sense" and "antisense" ribonucleotide sequences of the further subunits. The number of the units n is greater than or equal to two. This gives rise to a structure with several hairpins. The primary structure of the polycistronic RNA can have for example the basic structure 5'-F(1)-(RE1)-S(1)-(RE2)-F(2)-(RE3)-S(2)-(RE4)-.....-F(n)-(REn)-S(n)-3'. These molecules are capable to form multiple hairpin secondary structures (see Fig. 2B left part). In both cases the molecule is processed by removal of the removable RNA into a multiple double-stranded RNA molecules (without be restricted to the order of the various steps i.e. whether RNA processing (step II in Fig. 2B) or formation of the double-stranded structure (step I in Fig. 2B) occurs first) as schematically shown in Fig. 2B.

Fig.3 Schematic drawing of binary plasmid vector 1bxPcUbiLic (SEQ ID NO: 17).

Fig.4 Schematic drawing of binary plasmid vector 1bxPcUbitRNA (SEQ ID NO: 19).

Fig.5 Schematic drawing of binary plasmid vector 1 bxPcUbisnoRNA (SEQ ID NO: 18).

**DEFINITIONS**

Abbreviations: BAP - 6-benzylaminopurine; 2,4-D - 2,4-dichlorophenoxyacetic acid; MS - Mura-shige and Skoog medium; NAA - 1-naphtaleneacetic acid; MES, 2-(N-morpholino-ethanesulfonic acid, IAA indole acetic acid; Kan: Kanamycin sulfate; GA3 - Gibberellic acid; Timentin™: ticarcillin disodium / clavulanate potassium.

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth. The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 per-cent up or down (higher or lower). As used herein, the word "or" means any one member of a particular list and also includes any combination of members of that list. The words "comprise," "comprising," "include," "including," and "includes" when used in this specification and in the following claims are intended to specify the presence of one or more stated features, integers, components, or steps, but they do not preclude the presence or addition of one or more other features, integers, components, steps, or groups thereof. For clarity, certain terms used in the specification are defined and used as follows:

Agronomically valuable trait: The term "agronomically valuable trait" refers to any phenotype in a plant organism that is useful or advantageous for food production or food products, including plant parts and plant products. Non-food agricultural products such as paper, etc. are also included. A partial list of agronomically valuable traits includes pest resistance, vigor, development time (time to harvest), enhanced nutrient content, novel growth patterns, flavors or colors, salt, heat, drought and cold tolerance, and the like. Preferably, agronomically valuable traits do not include selectable marker genes (e.g., genes encoding herbicide or antibiotic resistance used only to facilitate detection or selection of transformed cells), hormone biosynthesis genes leading to the production of a plant hormone (e.g., auxins, gibberllins, cytokinins, abscisic acid and ethylene that are used only for selection), or reporter genes (*e.g.* luciferase, glucuronidase, chloramphenicol acetyl transferase (CAT, *etc.*). Such agronomically valuable important traits may include improvement of pest resistance (e.g., Melchers *et al.* (2000) Curr Opin Plant Biol 3(2):147-52), vigor, development time (time to harvest), enhanced nutrient content, novel growth patterns, flavors or colors, salt, heat, drought, and cold tolerance (e.g., Sakamoto *et al.* (2000) J Exp Bot 51(342):81-8; Saijo *et al.* (2000) Plant J 23(3): 319-327; Yeo *et al.*(2000) Mol Cells 10(3):263-8; Cushman *et al.* (2000) Curr Opin Plant Biol 3(2):117-24), and the like. Those of skill will recognize that there are numerous polynucleotides from which to choose to confer these and other agronomically valuable traits.

Alter: To "alter" or "modulate" the expression of a nucleotide sequence in a cell (e.g., a plant cell) means that the level of expression of the nucleotide sequence in a cell after applying a method of the present invention is different from its expression in the cell before applying the method. In a preferred embodiment, to alter expression means that the expression of the nucleotide sequence in the plant is reduced after applying a method of the present invention as compared to before applying the method. "Reduction of or "to reduce" the expression of a target gene is to be understood in the broad sense and comprises the partial or essentially complete prevention or blocking of the expression of the target gene or the RNA, mRNA, rRNA, tRNA derived therefrom and/or of the protein product encoded by it in a cell, an organism or a part, tissue, organ, cell or seed thereof, which prevention or blockage may be based on different cell-biological mechanisms. The term "reduced" means herein lower, preferably significantly lower, more preferably the expression of the nucleotide sequence is not detectable. As used herein, "a reduction" of the level of an agent such as a protein or mRNA means that the level is reduced relative to a cell or organism lacking a polycistronic RNA molecule of the invention capable of reducing the agent. As used herein, "at least a partial reduction" of the level of an agent (such as a RNA, mRNA, rRNA, tRNA expressed by the target gene and/or of the protein product encoded by it) means that the level is reduced at least 25%, preferably at least 50%, relative to a cell or organism lacking a polycistronic RNA molecule of the invention capable of reducing said agent. As used herein, "a substantial reduction" of the level of an agent such as a protein or mRNA means that the level is reduced relative to a cell or organism lacking a polycistronic RNA molecule of the invention capable of reducing the agent, where the reduction of the level of the agent is at least 75%, preferably at least 85%,. As used herein, "an effective elimination" of an agent such as a protein or mRNA is relative to a cell or organism lacking a polycistronic RNA molecule of the invention capable of reducing the agent, where the reduction of the level of the agent is greater than 95%, preferably greater than 98%. The reduction can be determined by methods with which the skilled worker is familiar. Thus, the reduction of the protein quantity can be determined for example by an immunological detection of the protein. Moreover, biochemical techniques such as Northern hybridization, nuclease protection assay, reverse transcription (quantitative RT-PCR), ELISA (enzyme-linked immunosorbent assay), Western blotting, radioimmunoassay (RIA) or other immunoassays and fluorescence-activated cell analysis (FACS) can be employed. Depending on the type of the reduced protein product, its activity or the effect on the phenotype of the organism or the cell may also be determined. Methods for determining the protein quantity are known to the skilled worker. Examples, which may be mentioned, are: the micro-Biuret method (Goa J (1953) Scand J Clin Lab Invest 5: 218-222), the Folin-Ciocalteau method (Lowry OH *et al.* (1951) J Biol Chem 193:265-275) or measuring the absorption of CBB G-250 (Bradford MM (1976) Analyt Biochem 72:248-254). In another preferred embodiment, to alter expression means that the expression of the nucleotide sequence in the plant is increased after applying a method of the present invention as compared to before applying the method.

Amino acid sequence: As used herein, the term "amino acid sequence" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

Animal: The terms "animal" or "animal organism" refer to nonhuman vertebrates or invertebrates. Preferred vertebrates comprise, for example, fish species, nonhuman mammals such as cattle, horse, sheep, goat, mouse, rat or pig, and birds such as chicken or goose. Preferred animal cells comprise CHO, COS, HEK293 cells. Invertebrates comprise nematodes or other worms, and insects. Invertebrates comprise insect cells such as Drosophila S2 and Spodoptera Sf9 or Sf21 cells. Furthermore preferred are nematodes, which are capable of attacking animals or humans, such as those of the genera *Ancylostoma, Ascaridia, Ascaris, Bunostomum, Caenorhabditis, Capillaria, Chabertia, Cooperia, Dictyocaulus, Haemonchus, Heterakis, Nematodirus, Oesophagostomum, Ostertagia, Oxyuris, Parascaris, Strongylus, Toxascaris, Trichuris, Trichostrongylus, Tfhchonema, Toxocara or Uncinana.* Furthermore preferred are those which are capable of attacking plant organisms such as, for example, *Bursaphalenchus, Criconemella, Diiylenchus, Ditylenchus, Globodera, Helicotylenchus, Heterodera, Longidorus, Melodoigyne, Nacobbus, Paratylenchus, Pratylenchus, Radopholus, Rotelynchus, Tylenchus* or *Xiphinema.* Preferred insects comprise those of the genera *Coleoptera, Diptera, Lepidoptera* and *Homoptera*.

Antiparallel: "Antiparallel" refers herein to two nucleotide sequences paired through hydrogen bonds between complementary base residues with phosphodiester bonds running in the 5'-3' direction in one nucleotide sequence and in the 3'-5' direction in the other nucleotide sequence.

Antisense: The term "antisense" refers to a nucleotide sequence that is inverted relative to its normal orientation for transcription and so expresses an RNA transcript that is complementary to a target gene mRNA molecule expressed within the host cell (e.g., it can hybridize to the target gene mRNA molecule through Watson-Crick base pairing). An antisense strand may be constructed in a number of different ways, provided that it is capable of interfering with the expression of a target gene. For example, the antisense strand can be constructed by inverting the coding region (or a portion thereof) of the target gene relative to its normal orientation for transcription to allow the transcription of its complement, (e.g., RNAs encoded by the antisense and sense gene may be complementary). Furthermore, the antisense oligonucleotide strand need not have the same intron or exon pattern as the target gene, and noncoding segments of the target gene may be equally effective in achieving antisense suppression of target gene expression as coding segments. In the context of gene silencing the term "antisense" is understood to mean a nucleic acid having a sequence complementary to a target sequence, for example a messenger RNA (mRNA) sequence the blocking of whose expression is sought to be initiated by hybridization with the target sequence.

Cell: The term "cell" or "plant cell" as used herein refers preferably to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronized or not synchronized. A cell within the meaning of this invention may be isolated (e.g., in suspension culture) or comprised in a tissue, organ or organism at any developmental stage.

Coding region: As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'-side by the nucleotide triplet "ATG" which encodes the initiator methionine and on the 3'-side by one of the three triplets which specify stop codons (*i.e.*, TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, post-transcriptional cleavage and polyadenylation.

Complementary: "Complementary" or "complementarity" refers to two nucleotide sequences which comprise antiparallel nucleotide sequences capable of pairing with one another (by the base-pairing rules) upon formation of hydrogen bonds between the complementary base residues in the antiparallel nucleotide sequences. For example, the sequence 5'-AGT-3' is complementary to the sequence 5'-ACT-3'. Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. A "complement" of a nucleic acid sequence as used herein refers to a nucleotide sequence whose nucleic acids show total complementarity to the nucleic acids of the nucleic acid sequence.

Chromosomal DNA: The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like e.g., polymerase chain reaction (PCR) analysis, Southern blot analysis, fluorescence *in situ* hybridization (FISH), and *in situ* PCR.

DNA shuffling: DNA shuffling is a method to rapidly, easily and efficiently introduce mutations or rearrangements, preferably randomly, in a DNA molecule or to generate exchanges of DNA sequences between two or more DNA molecules, preferably randomly. The DNA molecule resulting from DNA shuffling is a shuffled DNA molecule that is a non-naturally occurring DNA molecule derived from at least one template DNA molecule. The shuffled DNA encodes an enzyme modified with respect to the enzyme encoded by the template DNA, and preferably has an altered biological activity with respect to the enzyme encoded by the template DNA.

Double-stranded RNA: A "double-stranded RNA" molecule, "RNAi molecule", or "dsRNA" molecule comprises a sense RNA fragment of a nucleotide sequence and an antisense RNA fragment of the nucleotide sequence, which both comprise nucleotide sequences complementary to one another, thereby allowing the sense and antisense RNA fragments to pair and form a double-stranded RNA molecule. Preferably the terms refer to a double-stranded RNA molecule capable, when introduced into a cell or organism, of at least partially reducing the level of an mRNA species present in a cell or a cell of an organism. As used herein, "RNA interference", "RNAi, and "dsRNAi" refer to gene-specific silencing that is induced by the introduction of a double-stranded RNA molecule.

Endogenous: An "endogenous" nucleotide sequence refers to a nucleotide sequence which is present in the genome of the untransformed cell (e.g., a plant or mammalian cell).

Essential: An "essential" gene is a gene encoding a protein such as e.g. a biosynthetic enzyme, receptor, signal transduction protein, structural gene product, or transport protein that is essential to the growth or survival of the organism or cell (e.g., a plant).

Exon: The term "exon" as used herein refers to the normal sense of the term as meaning a segment of nucleic acid molecules, usually DNA, that encodes part of or all of an expressed protein.

Expression: "Expression" refers to the biosynthesis of a gene product, preferably to the transcription and/or translation of a nucleotide sequence, for example an endogenous gene or a heterologous gene, in a cell. For example, in the case of a structural gene, expression involves transcription of the structural gene into mRNA and - optionally - the subsequent translation of mRNA into one or more polypeptides. In the case of antisense constructs, for example, expression may refer to the transcription of the antisense DNA only.

Expression cassette / expression construct: "Expression cassette" and "expression construct" as used herein are synonyms and mean a DNA sequence capable of directing expression of a particular nucleotide sequence in an appropriate host cell (e.g., a plant pr mammalian cell), comprising a promoter functional in said host cell into which it will be introduced, operatively linked to the nucleotide sequence of interest which is - optionally - operatively linked to termination signals. If translation is required, it also typically comprises sequences required for proper translation of the nucleotide sequence. The coding regiori may code for a protein of interest but may also code for a functional RNA of interest, for example antisense RNA, dsRNA, or a nontranslated RNA, in the sense or antisense direction. The expression cassette comprising the nucleotide sequence of interest may be chimeric, meaning that at least one of its components is heterologous with respect to at least one of its other components. The expression cassette may also be one, which is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into the host cell or an ancestor of the host cell by a transformation event. The expression of the nucleotide sequence in the expression cassette may be under the control of a constitutive promoter or of an inducible promoter which initiates transcription only when the host cell is exposed to some particular external stimulus. In the case of a multicellular organism, such as a plant, the promoter can also be specific to a particular tissue or organ or stage of development.

Foreign gene: The term "foreign gene" refers to any nucleic acid (e.g., gene sequence) which is introduced into the genome of a cell by experimental manipulations and may include gene sequences found in that cell so long as the introduced gene contains some modification (e.g., a point mutation, the presence of a selectable marker gene, etc.) relative to the naturally-occurring gene.

Gene: The term "gene" refers to a coding region operably joined to appropriate regulatory sequences capable of regulating the expression of the gene product (e.g., a polypeptide or a functional RNA) in some manner. A gene includes untranslated regulatory regions of DNA (e.g., promoters, enhancers, repressors, etc.) preceding (up-stream) and following (downstream) the coding region (open reading frame, ORF) as well as,

where applicable, intervening sequences (*i.e.*, introns) between individual coding regions (*i.e.*, exons). The term "structural gene" as used herein is intended to mean a DNA sequence that is transcribed into mRNA which is then translated into a sequence of amino acids characteristic of a specific polypeptide.

Genetically-modified organism: The term "genetically-modified organism" or "GMO" refers to any organism that comprises heterologous DNA or a transgene. Exemplary organisms include plants, animals and microorganisms.

Genome and genomic DNA: The terms "genome" or "genomic DNA" is referring to the heritable genetic information of a host organism. Said genomic DNA comprises the DNA of the nucleus (also referred to as chromosomal DNA) but also the DNA of the plastids (e.g., chloroplasts) and other cellular organelles (e.g., mitochondria). Preferably the terms genome or genomic DNA is referring to the chromosomal DNA of the nucleus.

Hairpin RNA: As used herein "hairpin RNA" refers to any self-annealing double stranded RNA molecule. In its simplest representation, a hairpin RNA consists of a double stranded stem made up by the annealing RNA strands, connected by a single stranded RNA loop, and is also referred to as a "pan-handle RNA". However, the term "hairpin RNA" is also intended to encompass more complicated secondary RNA structures comprising self-annealing double stranded RNA sequences, but also internal bulges and loops. The specific secondary structure adapted will be determined by the free energy of the RNA molecule, and can be predicted for different situations using appropriate software such as FOLDRNA (Zuker and Stiegler (1981) Nucleic Acids Res 9(1):133-48; Zuker, M. (1989) Methods Enzymol. 180, 262-288).

Heterologous: The terms "heterologous" with respect to a nucleic acid or DNA refer to a nucleotide sequence which is ligated to, or is manipulated to become ligated to, a nucleic acid sequence to which it is not ligated in nature, or to which it is ligated at a different location in nature. A heterologous expression construct comprising a nucleic acid sequence and at least one regulatory sequence (such as an promoter or an transcription termination signal) linked thereto for example is a constructs originating by experimental manipulations in which either a) said nucleic acid sequence, or b) said regulatory sequence or c) both (i.e.(a) and (b)) is not located in its natural (native) genetic environment or has been modified by experimental manipulations, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1,000 bp, very especially preferably at least 5,000 bp, in length. A naturally occurring expression construct - for example the naturally occurring combination of a promoter with the corresponding gene - becomes a transgenic expression construct when it is modified by non-natural, synthetic "artificial" methods such as, for example, mutagenization. Such methods have been described (US 5,565,350; WO 00/15815). For example a protein encoding nucleic acid sequence operably lined to a promoter, which is not the native promoter of this sequence, is considered to be heterologous with respect to the promoter. Preferably, heterologous DNA is not endogenous to or not naturally associated with the cell into which it is introduced, but has been obtained from another cell. Heterologous DNA also includes an endogenous DNA sequence, which contains some modification, non-naturally occurring multiple copies of a endogenous DNA sequence, or a DNA sequence which is not naturally associated with another DNA sequence physically linked thereto. Generally, although not necessarily, heterologous DNA encodes RNA and proteins that are not normally produced by the cell into which it is expressed.

Homologous DNA Sequence: a DNA sequence naturally associated with a host cell or another DNA sequence.

Hybridization : The term "hybridization" as used herein includes "any process by which a strand of nucleic acid joins with a complementary strand through base pairing." (J. Coombs (1994) Dictionary of Biotechnology, Stockton Press, New York). Hybridization and the strength of hybridization (*i.e.*, the strength of the association between the nucleic acids) is impacted by such factors as the degree of complementarity between the nucleic acids, stringency of the conditions involved, the Tm of the formed hybrid, and the G:C ratio within the nucleic acids. As used herein, the term "Tm" is used in reference to the "melting temperature." The melting temperature is the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0.41 (% G+C), when a nucleic acid is in aqueous solution at 1 M NaCl [see e.g., Anderson and Young, Quantitative Filter Hybridization, in Nucleic Acid Hybridization (1985)]. Other references include more sophisticated computations, which take structural as well as sequence characteristics into account for the calculation of Tm. Stringent conditions, are known to those skilled in the art and can be found in Current Protocols in Molecular Biology, John Wiley & Sons, N.Y. (1989), 6.3.1-6.3.6. Low stringency conditions when used in reference to nucleic acid hybridization comprise conditions equivalent to binding or hybridization at 68°C in a solution consisting of 5x SSPE (43.8 g/L NaCl, 6.9 g/L $NaH_2PO_4.H_2O$ and 1.85 g/L EDTA, pH adjusted to 7.4 with NaOH), 1% SDS, 5x Denhardt's reagent [50x Denhardt's contains the following per 500 mL 5 g Ficoll (Type 400, Pharmacia), 5 g BSA (Fraction V; Sigma)] and 100 μg/mL denatured salmon sperm DNA followed by washing (preferably for one times 15 minutes, more preferably two times 15 minutes, more preferably three time 15 minutes) in a solution comprising 1xSSC (1× SSC is 0.15 M NaCl plus 0.015 M sodium citrate) and 0.1% SDS at room temperature or - preferably 37°C - when a DNA probe of preferably about 100 to about 1,000 nucleotides in length is employed. Medium stringency conditions when used in reference to nucleic acid hybridization comprise conditions equivalent to binding or hybridization at 68°C in a solution consisting of 5x SSPE (43.8 g/L NaCl, 6.9 g/L $NaH_2PO_4.H_2O$ and 1.85 g/L EDTA, pH adjusted to 7.4 with NaOH), 1% SDS, 5x Denhardt's reagent [50x Denhardt's contains the following per 500 mL 5 g Ficoll (Type 400, Pharmacia), 5 g BSA (Fraction V; Sigma)] and 100 μg/mL denatured salmon sperm DNA followed by washing (preferably for one times 15 minutes, more preferably two times 15 minutes, more preferably three time 15 minutes) in a solution comprising 0.1xSSC (1× SSC is 0.15 M NaCl plus 0.015 M sodium citrate) and 1% SDS at room temperature or - preferably 37°C - when a DNA probe of preferably about 100 to about 1,000 nucleotides in length is employed. High stringency conditions when used in reference to nucleic acid hybridization comprise conditions equivalent to binding or hybridization at 68°C in a solution consisting of 5x SSPE, 1% SDS, 5x Denhardt's reagent and 100 μg/mL denatured salmon sperm DNA followed by washing (preferably for one times 15 minutes, more preferably two times 15 minutes, more preferably three time 15 minutes) in a solution comprising 0.1x SSC, and 1% SDS at 68°C, when a probe of preferably about 100 to about 1,000 nucleotides in length is employed. The term "equivalent" when made in reference to a hybridization condition as it relates to a hybridization condition of interest means that the hybridization condition and the hybridization condition of interest result in hybridization of nucleic acid sequences which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence. When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, etc.) and the concentration of the salts and other components (e.g., the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding, lower stringencies may be preferred to detect a larger number of nucleic acid sequences having different homologies.

"Identity": The term "identity" is a relationship between two or more polypeptide sequences or two or more nucleic acid molecule sequences, as determined by comparing the sequences. In the art, "identity" also means the degree of sequence relatedness between polypeptide or nucleic acid molecule sequences, as determined by the match between strings of such sequences. "Identity" as used herein can be measured between nucleic acid sequences of the same ribonucleic-type (such as between DNA and DNA sequences) or between different types (such as between RNA and DNA sequences). It should be understood that in comparing an RNA sequence to a DNA sequence, an "identical" RNA sequence will contain ribonucleotides where the DNA sequence contains deoxyribonucleotides, and further that the RNA sequence will contain a uracil at positions where the DNA sequence contains thymidine. In case an identity is measured between RNA and DNA sequences, uracil bases of RNA sequences are considered to be identical to thymidine bases of DNA sequences. "Identity" can be readily calculated by known methods including, but not limited to, those described in Computational Molecular Biology, Lesk, A. M., ed., Oxford University Press, New York (1988); Biocomputing: Informatics and Genome Projects, Smith, D. W., ed., Academic Press, New York, 1993; Computer Analysis of Sequence Data, Part I, Griffin, A. M. and Griffin, H. G., eds., Humana Press, New Jersey (1994); Sequence Analysis in Molecular Biology, von Heinje, G., Academic Press (1987); Sequence Analysis Primer, Gribskov, M. and Devereux, J., eds., Stockton Press, New York (1991); and Carillo, H., and Lipman, D., SIAM J. Applied Math, 48:1073 (1988). Methods to determine identity are designed to give the largest match between the sequences tested. Moreover, methods to determine identity are codified in publicly available programs. Computer programs which can be used to determine identity between two sequences include, but are not limited to, GCG (Devereux, J., *et al.*, Nucleic Acids Research 12(1):387 (1984); suite of five BLAST programs, three designed for nucleotide sequences queries (BLASTN, BLASTX, and TBLASTX) and two designed for protein sequence queries (BLASTP and TBLASTN) (Coulson, Trends in Biotechnology, 12:76-80 (1994); Birren *et al.*, Genome Analysis, 1:543-559 (1997)). The BLASTX program is publicly available from NCBI and other sources (BLAST Manual, Altschul, S., et *al.*, NCBI NLM NIH, Bethesda, Md. 20894; Altschul, S., *et al.*, J. Mol. Biol., 215: 403-410 (1990)). The well-known Smith Waterman algorithm can also be used to determine identity. Parameters for polypeptide sequence comparison typically include the following:

- Algorithm: Needleman and Wunsch, J. Mol. Biol., 48:443-453 (1970)
- Comparison matrix: BLOSUM62 from Hentikoff and Hentikoff, Proc. Natl. Acad. Sci. USA, 89:10915-10919 (1992)
- Gap Penalty: 12
- Gap Length Penalty: 4

A program, which can be used with these parameters, is publicly available as the "gap" program from Genetics Computer Group, Madison, Wis. The above parameters along with no penalty for end gap are the default parameters for peptide comparisons. Parameters for nucleic acid molecule sequence comparison include the following:

- Algorithm: Needleman and Wunsch, J. Mol. Bio., 48:443-453 (1970)
- Comparison matrix: matches-+10; mismatches=0
- Gap Penalty: 50
- Gap Length Penalty: 3 As used herein, "% identity" is determined using the above parameters as the default parameters for nucleic acid molecule sequence comparisons and the "gap" program from GCG, version 10.2.

Infecting: The terms "infecting" and "infection" with a bacterium or virus refer to co-incubation of a target biological sample, (e.g., cell, tissue, etc.) with the bacterium or virus under conditions such that nucleic acid sequences contained within the bacterium or virus are introduced into one or more cells of the target biological sample.

Intron: The term "intron" as used herein refers to the normal sense of the term as meaning a segment of nucleic acid molecules, usually DNA, that does not encode part of or all of an expressed protein, and which, in endogenous conditions, is transcribed into RNA molecules, but which is spliced out of the endogenous RNA before the RNA is translated into a protein. The splicing, i.e., intron removal, occurs at a defined splice site, e.g., typically at least about 4 nucleotides, between cDNA and intron sequence. For example, without limitation, the sense and antisense intron segments illustrated herein, which form a double-stranded RNA contained no splice sites.

Isogenic: organisms (e.g., plants), which are genetically identical, except that they may differ by the presence or absence of a heterologous DNA sequence.

Isolated: The term "isolated" as used herein means that a material has been removed by the hand of man and exists apart from its original, native environment and is therefore not a product of nature. An isolated material or molecule (such as a DNA molecule or enzyme) may exist in a purified form or may exist in a non-native environment such as, for example, in a transgenic host cell. For example, a naturally occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment. Preferably, the term "isolated" when used in relation to a nucleic acid, as in "an isolated nucleic acid sequence" refers to a nucleic acid sequence that is identified and separated from at least one contaminant nucleic acid with which it is ordinarily associated in its natural source. Isolated nucleic acid is nucleic acid present in a form or setting that is different from that in which it is found in nature. In contrast, non-isolated nucleic acids are nucleic acids such as DNA and RNA, which are found in the state they exist in nature. For example, a given DNA sequence (e.g., a gene) is found on the host cell chromosome in proximity to neighboring genes; RNA sequences, such as a specific mRNA sequence encoding a specific protein, are found in the cell as a mixture with numerous other mRNAs, which encode a multitude of proteins. However, an isolated nucleic acid sequence comprising for example SEQ ID NO: 1 includes, by way of example, such nucleic acid sequences in cells which ordinarily contain SEQ ID NO:1 where the nucleic acid sequence is in a chromosomal or extrachromosomal location different from that of natural cells, or is otherwise flanked by a different nucleic acid sequence than that found in nature. The isolated nucleic acid sequence may be present in single-stranded or double-stranded form. When an isolated nucleic acid sequence is to be utilized to express a protein, the nucleic acid sequence will contain at a minimum at least a portion of the sense or coding strand (*i.e.*, the nucleic acid sequence may be single-stranded). Alternatively, it may contain both the sense and anti-sense strands (i.e., the nucleic acid sequence may be double-stranded).

Mammal: The terms "mammal" or "mammalian" are intended to encompass their normal meaning. While the invention is most desirably intended for efficacy in humans, it may also be employed in domestic mammals such as canines, felines, and equines, as well as in mammals of particular interest, e.g., zoo animals, farmstock and the like.

Mature protein: protein which is normally targeted to a cellular organelle, such as a chloroplast, and from which the transit peptide has been removed.

Minimal Promoter: promoter elements, particularly a TATA element, that are inactive or that have greatly reduced promoter activity in the absence of upstream activation. In the presence of a suitable transcription factor, the minimal promoter functions to permit transcription.

Non-coding: The term "non-coding" refers to sequences of nucleic acid molecules that do not encode part or all of an expressed protein. Non-coding sequences include but are not limited to introns, promoter regions, 3' untranslated regions, and 5' untranslated regions.

Nucleic acids and nucleotides: The terms "Nucleic Acids" and "Nucleotides" refer to naturally occurring or synthetic or artificial nucleic acid or nucleotides. The terms "nucleic acids" and "nucleotides" comprise deoxyribonucleotides or ribonucleotides or any nucleotide analogue and polymers or hybrids thereof in either single- or double-stranded, sense or antisense form. Unless otherwise indicated, a particular nucleic acid sequence also implicitly encompasses conservatively modified variants thereof (e.g., degenerate codon substitutions) and complementary sequences, as well as the sequence explicitly indicated. The term "nucleic acid" is used inter-changeably herein with "gene", "cDNA, "mRNA", "oligonucleotide," and "polynucleotide". Nucleotide analogues include nucleotides having modifications in the chemical structure of the base, sugar and/or phosphate, including, but not limited to, 5-position pyrimidine modifications, 8-position purine modifications, modifications at cytosine exocyclic amines, substitution of 5-bromo-uracil, and the like; and 2'-position sugar modifications, including but not limited to, sugar-modified ribonucleotides in which the 2'-OH is replaced by a group selected from H, OR, R, halo, SH, SR, NH2, NHR, NR2, or CN. shRNAs also can comprise non-natural elements such as non-natural bases, e.g., ionosin and xanthine, non-natural sugars, e.g., 2'-methoxy ribose, or non-natural phosphodiester linkages, e.g., methylphosphonates, phosphorothioates and peptides.

Nucleic acid sequence: The phrase "nucleic acid sequence" refers to a single or double-stranded polymer of deoxyribonucleotide or ribonucleotide bases read from the 5'-to the 3'-end. It includes chromosomal DNA, self-replicating plasmids, infectious polymers of DNA or RNA and DNA or RNA that performs a primarily structural role. "Nucleic acid sequence" also refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid, which is transcribed and translated in a sequence-specific manner to produce into a particular polypeptide or protein when placed under the control of appropriate regulatory sequences. The coding region is said to encode such a polypeptide or protein.

Nucleotide sequence of interest: The term "nucleotide sequence of interest" refers to any nucleotide sequence, the manipulation of which may be deemed desirable for any reason (e.g., confer improved qualities), by one of ordinary skill in the art. Such nucleotide sequences include, but are not limited to, coding sequences of structural genes (e.g., reporter genes, selection marker genes, drug resistance genes, growth factors, etc.), and non-coding regulatory sequences which do not encode an mRNA or protein product, (*e.g.*, promoter sequence, polyadenylation sequence, termination sequence, enhancer sequence, *etc.*). A nucleic acid sequence of interest may preferably encode for an agronomically valuable trait.

Oligonucleotide: The term "oligonucleotide" refers to an oligomer or polymer of ribonucleic acid (RNA) or deoxyribonucleic acid (DNA) or mimetics thereof, as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. An oligonucleotide preferably includes two or more nucleomonomers covalently coupled to each other by linkages (e.g., phosphodiesters) or substitute linkages.

Operable linkage: The term "operable linkage" or "operably linked" is to be understood as meaning, for example, the sequential arrangement of a regulatory element (e.g. a promoter) with a nucleic acid sequence to be expressed and, if appropriate, further regulatory elements (such as e.g., a terminator) in such a way that each of the regulatory elements can fulfill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. In a preferred embodiment, the nucleic acid sequence to be transcribed is located behind the promoter in such a way that the transcription start is identical with the desired beginning of the polycistronic RNA of the invention. Operable linkage, and an expression construct, can be generated by means of customary recombination and cloning techniques as described (*e.g.*, in Maniatis T, Fritsch EF and Sambrook J (1989) Molecular Cloning: A Laboratory Manual, 2nd Ed., Cold Spring Harbor Laboratory, Cold Spring Harbor (NY); Silhavy *et al.* (1984) Experiments with Gene Fusions, Cold Spring Harbor Laboratory, Cold Spring Harbor (NY); Ausubel *et al.* (1987) Current Protocols in Molecular Biology, Greene Publishing Assoc. and Wiley Interscience; Gelvin *et al.* (Eds) (1990) Plant Molecular Biology Manual; Kluwer Academic Publisher, Dordrecht, The Netherlands). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of promoter and nucleic acid sequence to be expressed, can exist in a vector-integrated form and be inserted into a plant genome, for example by transformation.

Organ: The term "organ" with respect to a plant (or "plant organ") means parts of a plant and may include (but shall not limited to) for example roots, fruits, shoots, stem, leaves, anthers, sepals, petals, pollen, seeds, etc. The term "organ" with respect to an animal ("animal organ") means parts of an animal and may include (but shall not limited to) for example external organs (such as arms, legs, head, etc.) or internal organs (such as heart, kidney, liver, stomach, etc.).

Overhang: An "overhang" is a relatively short single-stranded nucleotide sequence on the 5'- or 3'-hydroxyl end of a double-stranded oligonucleotide molecule (also referred to as an "extension," "protruding end," or "sticky end").

Plant: The terms "plant" or "plant organism" refer to any organism, which is capable of photosynthesis, and the cells, tissues, parts or propagation material (such as seeds or fruits) derived therefrom. Encompassed within the scope of the invention are all genera and species of higher and lower plants of the Plant Kingdom. Annual, perennial, monocotyledonous and dicotyledonous plants and gymnosperms are preferred. A "plant" refers to any plant or part of a plant at any stage of development. Mature plants refer to plants at any developmental stage beyond the seedling stage. Encompassed are mature plant, seed, shoots and seedlings, and parts, propagation material (for example tubers, seeds or fruits) and cultures, for example cell cultures or callus cultures,) derived therefrom. Seedling refers to a young, immature plant at an early developmental stage. Therein are also included cuttings, cell or tissue cultures and seeds. As used in conjunction with the present invention, the term "plant tissue" includes, but is not limited to, whole plants, plant cells, plant organs, plant seeds, protoplasts, callus, cell cultures, and any groups of plant cells organized into structural and/or functional units. Preferably, the term "plant" as used herein refers to a plurality of plant cells, which are largely differentiated into a structure that is present at any stage of a plant's development. Such structures include one or more plant organs including, but are not limited to, fruit, shoot, stem, leaf, flower petal, *etc.* More preferably, the term "plant" includes whole plants, shoot vegetative organs/structures (*e.g.* leaves, stems and tubers), roots, flowers and floral organs/structures (*e.g.* bracts, sepals, petals, stamens, carpels, anthers and ovules), seeds (including embryo, endosperm, and seed coat) and fruits (the mature ovary), plant tissues (e.g. vascular tissue, ground tissue, and the like) and cells (e.g. guard cells, egg cells, trichomes and the like), and progeny of same. The class of plants that can be used in the method of the invention is generally as broad as the class of higher and lower plants amenable to transformation techniques, including an-giosperms (monocotyledonous and dicotyledonous plants), gymnosperms, ferns, and multicellular algae. It includes plants of a variety of ploidy levels, including aneuploid, polyploid, diploid, haploid and hemizygous. Included within the scope of the invention are all genera and species of higher and lower plants of the plant kingdom. Included are furthermore the mature plants, seed, shoots and seedlings, and parts, propagation material (for example seeds and fruit) and cultures, for example cell cultures, derived therefrom. Preferred are plants and plant materials of the following plant families: *Amaranthaceae, Brassicaceae, Carophyllaceae, Chenopodiaceae, Compositae, Cucurbitaceae, Labiatae, Leguminosae, Papilionoideae, Liliaceae, Linaceae, Malvaceae, Rosaceae, Saxifragaceae, Scrophulariaceae, Solanaceae, Tetragoniaceae.* Annual, perennial, monocotyledonous and dicotyledonous plants are preferred host organisms for the generation of transgenic plants. The use of the recombination system, or method according to the invention is furthermore advantageous in all ornamental plants, forestry, fruit, or ornamental trees, flowers, cut flowers, shrubs or turf. Said plant may include - but shall not be limited to - bryophytes such as, for example, Hepaticae (hepaticas) and Musci (mosses); pteridophytes such as ferns, horsetail and clubmosses; gymnosperms such as conifers, cycads, ginkgo and Gnetaeae; algae such as *Chlorophyceae, Phaeophpyceae, Rhodophyceae, Myxophyceae, Xanthophyceae, Bacillariophyceae* (diatoms) and *Euglenophyceae.* Plants for the purposes of the invention may comprise the families of the *Rosaceae* such as rose, Ericaceae such as rhododendrons and azaleas, *Euphorbiaceae* such as poinsettias and croton, Caryophyllaceae such as pinks, *Solanaceae* such as petunias, *Gesneriaceae* such as African violet, *Balsaminaceae* such as touch-me-not, *Orchidaceae* such as orchids, Iridaceae such as gladioli, iris, freesia and crocus, Compositae such as marigold, Geraniaceae such as geraniums, *Liliaceae* such as *Drachaena,* Moraceae such as ficus, Araceae such as philodendron and many others. The transgenic plants according to the invention are furthermore selected in particular from among dicotyledonous crop plants such as, for example, from the families of the *Leguminosae* such as pea, alfalfa and soybean; the family of the *Umbelliferae,* particularly the genus *Daucus* (very particularly the species carota (carrot)) and *Apium* (very particularly the species *graveolens* var. dulce (celery)) and many others; the family of the Solanaceae, particularly the genus *Lycopersicon,* very particularly the species *esculentum* (tomato) and the genus So*lanum,* very particularly the species *tuberosum* (potato) and *melongena* (aubergine), tobacco and many others; and the genus *Capsicum,* very particularly the species *annum* (pepper) and many others; the family of the Leguminosae, particularly the genus *Glycine,* very particularly the species *max* (soybean) and many others; and the family of the Cruciferae, particularly the genus *Brassica,* very particularly the species *napus* (oilseed rape), *campestris* (beet), *oleracea* cv Tastie (cabbage), oleracea cv Snowball Y (cauliflower) and *oleracea* cv Emperor (broccoli); and the genus *Arabidopsis,* very particularly the species *thaliana* and many others; the family of the Compositae, particularly the genus *Lactuca,* very particularly the species *sativa* (lettuce) and many others. The transgenic plants according to the invention are selected in particular among monocotyledonous crop plants, such as, for example, cereals such as wheat, barley, sorghum and millet, rye, triticale, maize, rice or oats, and sugarcane. Further preferred are trees such as apple, pear, quince, plum, cherry, peach, nectarine, apricot, papaya, mango, and other woody species including coniferous and deciduous trees such as poplar, pine, sequoia, cedar, oak, *etc.* Especially preferred are *Arabidopsis thaliana, Nicotiana tabacum,* oilseed rape, soybean, com (maize), wheat, linseed, potato and tagetes.

Polynucleotide construct. The term "polynucleotide construct" refers to a nucleic acid at least partly created by recombinant methods. The term "DNA construct" is referring to a polynucleotide construct consisting of deoxyribonucleotides. The construct may be single- or - preferably - double stranded. The construct may be circular or linear. The skilled worker is familiar with a variety of ways to obtain one of a DNA construct. Constructs can be prepared by means of customary recombination and cloning techniques as are described, for example, in Maniatis T, Fritsch EF and Sambrook J (1989) Molecular Cloning: A Laboratory Manual, 2nd Ed., Cold Spring Harbor Laboratory, Cold Spring Harbor (NY); Silhavy *et al*. (1984) Experiments with Gene Fusions, Cold Spring Harbor Laboratory, Cold Spring Harbor (NY); Ausubel *et al*. (1987) Current Protocols in Molecular Biology, Greene Publishing Assoc. and Wiley Interscience; Gelvin *et al*. (Eds) (1990) Plant Molecular Biology Manual; Kluwer Academic Pub-lisher, Dordrecht, The Netherlands.

Polypeptide: The terms "polypeptide", "peptide", "oligopeptide", "polypeptide", "gene product", "expression product" and "protein" are used interchangeably herein to refer to a polymer or oligomer of consecutive amino acid residues.

Pre-protein: Protein, which is normally targeted to a cellular organelle, such as a chloroplast, and still comprising its transit peptide.

Promoter: The terms "promoter," "promoter element," or "promoter sequence" are equivalents and as used herein, refers to a DNA sequence which when ligated to a nucleotide sequence of interest is capable of controlling the transcription of the nucleotide sequence of interest into mRNA. A promoter is typically, though not necessarily, located 5' (*i.e.*, upstream) of a nucleotide sequence of interest (e.g., proximal to the transcriptional start site of a structural gene) whose transcription into mRNA it controls, and provides a site for specific binding by RNA polymerase and other transcription factors for initiation of transcription. A polynucleotide sequence is "heterologous to" an organism or a second polynucleotide sequence if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is not naturally associated with the promoter (e.g. a genetically engineered coding sequence or an allele from a different ecotype or variety). Suitable promoters can be derived from genes of the host cells where expression should occur or from pathogens for this host cells (e.g., plants or plant pathogens like plant viruses). If a promoter is an inducible promoter, then the rate of transcription increases in response to an inducing agent. In contrast, the rate of transcription is not regulated by an inducing agent if the promoter is a constitutive promoter. Also, the promoter may be regulated in a tissue-specific or tissue preferred manner such that it is only active in transcribing the associated coding region in a specific tissue type(s) such as leaves, roots or meristem. The term "tissue specific" as it applies to a promoter refers to a promoter that is capable of directing selective expression of a nucleotide sequence of interest to a specific type of tissue (e.g., petals) in the relative absence of expression of the same nucleotide sequence of interest in a different type of tissue (e.g., roots). Tissue specificity of a promoter may be evaluated by, for example, operably linking a reporter gene to the promoter sequence to generate a reporter construct, introducing the reporter construct into the genome of a plant such that the reporter construct is integrated into every tissue of the resulting transgenic plant, and detecting the expression of the reporter gene (e.g., detecting mRNA, protein, or the activity of a protein encoded by the reporter gene) in different tissues of the transgenic plant. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter, which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, e.g., GUS activity staining or immunohistochemical staining. The term "constitutive" when made in reference to a promoter means that the promoter is capable of directing transcription of an operably linked nucleic acid sequence in the absence of a stimulus (e.g., heat shock, chemicals, light, etc.). Typically, constitutive promoters are capable of directing expression of a transgene in substantially any cell and any tissue. In contrast, a "regulatable" promoter is one which is capable of directing a level of transcription of an operably linked nuclei acid sequence in the presence of a stimulus (e.g., heat shock, chemicals, light, etc.) which is different from the level of transcription of the operably linked nucleic acid sequence in the absence of the stimulus.

Purified: As used herein, the term "purified" refers to molecules, either nucleic or amino acid sequences that are removed from their natural environment, isolated or separated. "Substantially purified" molecules are at least 60% free, preferably at least 75% free, and more preferably at least 90% free from other components with which they are naturally associated. An purified nucleic acid sequence may be an isolated nucleic acid sequence.

Recombinant: The term "recombinant" with respect to polypeptides or proteins refer to polypeptides or proteins produced by recombinant DNA techniques, *i.e.*, produced from cells transformed by an exogenous recombinant DNA construct encoding the desired polypeptide or protein. Recombinant nucleic acids and polypeptide may also comprise molecules, which as such does not exist in nature but are modified, changed, mutated or otherwise manipulated by man. Preferably, a "recombinant polypeptide" is a non-naturally occurring polypeptide that differs in sequence from a naturally occurring polypeptide by at least one amino acid residue. Preferred methods for producing said recombinant polypeptide and/or nucleic acid may comprise directed or non-directed mutagenesis, DNA shuffling or other methods of recursive recombination.

Sense: The term "sense" is understood to mean a nucleic acid having a sequence which is homologous or identical to a target sequence, for example a sequence which binds to a protein transcription factor and which is involved in the expression of a given gene. According to a preferred embodiment, the nucleic acid comprises a gene of interest and elements allowing the expression of the said gene of interest.

Significant Increase or Decrease: An increase or decrease, for example in enzymatic activity or in gene expression, that is larger than the margin of error inherent in the measurement technique, preferably an increase or decrease by about 2-fold or greater of the activity of the control enzyme or expression in the control cell, more preferably an increase or decrease by about 5-fold or greater, and most preferably an increase or decrease by about 10-fold or greater.

Stabilize: To "stabilize" the expression of a nucleotide sequence in a plant cell means that the level of expression of the nucleotide sequence after applying a method of the present invention is approximately the same in cells from the same tissue in different plants from the same generation or throughout multiple generations when the plants are grown under the same or comparable conditions.

Substantially complementary: In its broadest sense, the term "substantially complementary", when used herein with respect to a nucleotide sequence in relation to a reference or target nucleotide sequence, means a nucleotide sequence having a percentage of identity between the substantially complementary nucleotide sequence and the exact complementary sequence of said reference or target nucleotide sequence of at least 60%, more desirably at least 70%, more desirably at least 80% or 85%, preferably at least 90%, more preferably at least 93%, still more preferably at least 95% or 96%, yet still more preferably at least 97% or 98%, yet still more preferably at least 99% or most preferably 100% (the later being equivalent to the term "identical" in this context). Preferably identity is assessed over a length of at least 19 nucleotides, preferably at least 50 nucleotides, more preferably the entire length of the nucleic acid sequence to said reference sequence (if not specified otherwise below). Sequence comparisons are carried out using default GAP analysis with the University of Wisconsin GCG, SEQWEB application of GAP, based on the algorithm of Needleman and Wunsch (Needleman and Wunsch (1970) J Mol. Biol. 48: 443-453; as defined above). A nucleotide sequence "substantially complementary " to a reference nucleotide sequence hybridizes to the reference nucleotide sequence under low stringency conditions, preferably medium stringency conditions, most preferably high stringency conditions (as defined above).

Substantially identical: In its broadest sense, the term "substantially identical", when used herein with respect to a nucleotide sequence, means a nucleotide sequence corresponding to a reference or target nucleotide sequence, wherein the percentage of identity between the substantially identical nucleotide sequence and the reference or target nucleotide sequence is desirably at least 60%, more desirably at least 70%, more desirably at least 80% or 85%, preferably at least 90%, more preferably at least 93%, still more preferably at least 95% or 96%, yet still more preferably at least 97% or 98%, yet still more preferably at least 99% or most preferably 100% (the later being equivalent to the term "identical" in this context). Preferably identity is assessed over a length of at least 19 nucleotides, preferably at least 50 nucleotides, more preferably the entire length of the nucleic acid sequence to said reference sequence (if not specified otherwise below). Sequence comparisons are carried out using default GAP analysis with the University of Wisconsin GCG, SEQWEB application of GAP, based on the algorithm of Needleman and Wunsch (Needleman and Wunsch (1970) J Mol. Biol. 48: 443-453; as defined above). A nucleotide sequence "substantially identical" to a reference nucleotide sequence hybridizes to the exact complementary sequence of the reference nucleotide sequence (i.e. its corresponding strand in a double-stranded molecule) under low stringency conditions, preferably medium stringency conditions, most preferably high stringency conditions (as defined above). Homologes of a specific nucleotide sequence include nucleotide sequences that encode an amino acid sequence that is at least 24% identical, more preferably at least 35% identical, yet more preferably at least 50% identical, yet more preferably at least 65% identical to the reference amino acid sequence, as measured using the parameters described above, wherein the amino acid sequence encoded by the homolog has the same biological activity as the protein encoded by the specific nucleotide. The term "substantially identical", when used herein with respect to a polypeptide, means a protein corresponding to a reference polypeptide, wherein the polypeptide has substantially the same structure and function as the reference protein, e.g. where only changes in amino acids sequence not affecting the polypeptide function occur. When used for a polypeptide or an amino acid sequence the percentage of identity between the substantially similar and the reference polypeptide or amino acid sequence desirably is at least 24%, more desirably at least 30%, more desirably at least 45%, preferably at least 60%, more preferably at least 75%, still more preferably at least 90%, yet still more preferably at least 95%, yet still more preferably at least 99%, using default GAP analysis parameters as described above. Homologes are amino acid sequences that are at least 24% identical, more preferably at least 35% identical, yet more preferably at least 50% identical, yet more preferably at least 65% identical to the reference polypeptide or amino acid sequence, as measured using the parameters described above, wherein the amino acid sequence encoded by the homolog has the same biological activity as the reference polypeptide.

Synthetic: As used herein, "synthetic" means made wholly by chemical means, e.g. through the annealing of chemically-synthesized complementary oligonucleotides rather than by biological means, e.g. through the amplification of a chemically-synthesized template using the polymerase chain reaction (PCR) or other enzyme-mediated biological reactions such as ligation or phosphorylation. In preferred embodiments, the oligonucleotides are synthesized using commercial oligonucleotide synthesis machines, including but not limited to the ABI 394 and ABI 3900 DNA/RNA Synthesizers available from Applied Biosystems, Inc. or other commercially-equivalent synthesizers.

Target gene: The terms "target", "target gene" and "target nucleotide sequence" are used equivalently. As used herein, a target gene can be any gene of interest present in an eukaryotic organism (such as a plant). A target gene may be endogenous or introduced. For example, a target gene is a gene of known function or is a gene whose function is unknown, but whose total or partial nucleotide sequence is known. Alternatively, the function of a target gene and its nucleotide sequence are both unknown. A target gene is a native gene of the eukaryotic cell or is a heterologous gene which has previously been introduced into the eukaryotic cell or a parent cell of said eukaryotic cell, for example by genetic transformation. A heterologous target gene is stably integrated in the genome of the eukaryotic cell or is present in the eukaryotic cell as an extrachromosomal molecule, e.g. as an autonomously replicating extrachromosomal molecule. A target gene may include polynucleotides comprising a region that encodes a polypeptide or polynucleotide region that regulates replication, transcription, translation, or other process important in expression of the target protein; or a polynucleotide comprising a region that encodes the target polypeptide and a region that regulates expression of the target polypeptide; or non-coding regions such as the 5' or 3' UTR or introns. A target gene may refer to, for example, an mRNA molecule produced by transcription a gene of interest. Furthermore, the term "correspond," as in "an polycistronic RNA comprising a sequence that corresponds to a target gene sequence," means that the two sequences are complementary or homologous or bear such other biologically rational relationship to each other (e.g., based on the sequence of nucleomonomers and their base-pairing properties). The "target gene" to which an polycistronic RNA molecule of the invention is directed may be associated with a pathological condition. For example, the gene may be a pathogen-associated gene, e.g., a viral gene, a tumor-associated gene, a defective gene (e.g., an abnormal cancer-causing gene), or an autoimmune disease-associated gene. The target gene may also be a heterologous gene expressed in a recombinant cell or a genetically altered organism. By determining or modulating (e.g., inhibiting) the function of such a gene, valuable information and therapeutic benefits in medicine, veterinary medicine, and biology may be obtained.

Tissue: The term "tissue" with respect to an organism (e.g., a plant; "plant tissue") means arrangement of multiple cells including differentiated and undifferentiated tissues of the organism. Tissues may constitute part of an organ (e.g., the epidermis of a plant leaf or an animal skin) but may also constitute tumor tissues (e.g., callus tissue) and various types of cells in culture (e.g., single cells, protoplasts, embryos, calli, protocorm-like bodies, *etc.).* The tissue may be *in vivo (e.g., in planta)*, in organ culture, tissue culture, or cell culture.

Transformation: The term "transformation" as used herein refers to the introduction of genetic material (*e.g.*, a transgene or heterologous nucleic acid molecules) into a cell, tissue or organism. Transformation of a cell may be stable or transient. The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA), which detects the presence of a polypeptide encoded by one or more of the transgenes. Alternatively, transient transformation may be detected by detecting the activity of the protein (e.g., β-glucuronidase) encoded by the transgene (e.g., the uid A gene). The term "transient transformant" refers to a cell which has transiently incorporated one or more transgenes. In contrast, the term "stable transformation" or "stably transformed" refers to the introduction and integration of one or more transgenes into the genome of a cell, preferably resulting in chromosomal integration and stable heritability through meiosis. Stable transformation of a cell may be detected by Southern blot hybridization of genomic DNA of the cell with nucleic acid sequences, which are capable of binding to one or more of the transgenes. Alternatively, stable transformation of a cell may also be detected by the polymerase chain reaction of genomic DNA of the cell to amplify transgene sequences. The term "stable transformant" refers to a cell, which has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene. Transformation also includes introduction of genetic material into plant cells in the form of plant viral vectors involving epichromosomal replication and gene expression, which may exhibit variable properties with respect to meiotic stability. Transformed cells, tissues, or plants are understood to encompass not only the end product of a transformation process, but also transgenic progeny thereof.

Transgene: The term "transgene" as used herein refers to any nucleic acid sequence, which is introduced into the genome of a cell by experimental manipulations. A transgene may be an "endogenous DNA sequence," or a "heterologous DNA sequence" (i.e., "foreign DNA"). The term "endogenous DNA sequence" refers to a nucleotide sequence, which is naturally found in the cell into which it is introduced so long as it does not contain some modification (e.g., a point mutation, the presence of a selectable marker gene, *etc.*) relative to the naturally-occurring sequence.

Transgenic: The term transgenic when referring to a cell, tissue or organisms means transformed, preferably stably transformed, with a recombinant DNA molecule that preferably comprises a suitable promoter operatively linked to a DNA sequence of interest.

Unaffected: As used herein, "essentially unaffected" refers to a level of an agent such as a protein or mRNA transcript that is either not altered by a particular event or altered only to an extent that does not affect the physiological function of that agent. In a preferred aspect, the level of the agent that is essentially unaffected is within 20%, more preferably within 10%, and even more preferably within 5% of the level at which it is found in a cell or organism that lacks a nucleic acid molecule capable of selectively reducing another agent. As used herein, "substantially unaffected" refers to a level of an agent such as a protein or mRNA transcript in which the level of the agent that is substantially unaffected is within 49%, more preferably within 35%, and even more preferably within 24% of the level at which it is found in a cell or organism that lacks a nucleic acid molecule capable of selectively reducing another agent. As used herein, "partially unaffected" refers to a level of an agent such as a protein or mRNA transcript in which the level of the agent that is partially unaffected is within 80%, more preferably within 65%, and even more preferably within 50% of the level at which it is found in a cell or organism that lacks a nucleic acid molecule capable of selectively reducing another agent.

Vector: As used herein, the term "vector" refers to a nucleic acid molecule capable of transporting another nucleic acid to which it has been linked. One type of vector is a genomic integrated vector, or "integrated vector", which can become integrated into the chromosomal DNA of the host cell. Another type of vector is an episomal vector, i.e., a nucleic acid capable of extra-chromosomal replication. Vectors capable of directing the expression of genes to which they are operatively linked are referred to herein as "expression vectors". In the present specification, "plasmid" and "vector" are used interchangeably unless otherwise clear from the context. Expression vectors designed to produce RNAs as described herein in vitro or in vivo may contain sequences under the control of any RNA polymerase, including mitochondrial RNA polymerase; RNA pol I, RNA pol II; and RNA pol III. These vectors can be used to transcribe the desired RNA molecule in the cell according to this invention. Vectors may be desirably designed to utilize an endogenous mitochondrial RNA polymerase (e.g., human mitochondrial RNA polymerase, in which case such vectors may utilize the corresponding human mitochondrial promoter). Mitochondrial polymerases may be used to generate capped (through expression of a capping enzyme) or uncapped messages in vivo. RNA pol I, RNA pol II, and RNA pol III transcripts may also be generated in vivo. Such RNAs may be capped or not, and if desired, cytoplasmic capping may be accomplished by various means including use of a capping enzyme such as a vaccinia capping enzyme or an alphavirus capping enzyme. The DNA vector is designed to contain one of the promoters or multiple promoters in combination (mitochondrial, RNA polI, II, or polIII, or viral, bacterial or bacteriophage promoters along with the cognate polymerases). Preferably, where the promoter is RNA pol II, the sequence encoding the RNA molecule has an open reading frame greater than about 300 nts to avoid degradation in the nucleus. Such plasmids or vectors can include plasmid sequences from bacteria, viruses or phages. Such vectors include chromosomal, episomal and virus-derived vectors e.g., vectors derived from bacterial plasmids, bacteriophages, yeast episomes, yeast chromosomal elements, and viruses, vectors derived from combinations thereof, such as those derived from plasmid and bacteriophage genetic elements, cosmids and phagemids. Thus, one exemplary vector is a single or double-stranded phage vector. Another exemplary vector is a single or double-stranded RNA or DNA viral vector. Such vectors may be introduced into cells as polynucleotides, preferably DNA, by well known techniques for introducing DNA and RNA into cells. The vectors, in the case of phage and viral vectors may also be and preferably are introduced into cells as packaged or encapsidated virus by well known techniques for infection and transduction. Viral vectors may be replication competent or replication defective. In the latter case, viral propagation generally occurs only in complementing host cells.

Wild-type: The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

## DETAILED DESCRIPTION OF THE INVENTION

A first embodiment of the invention relates to a polycistronic RNA molecule capable to generate in an eukaryotic cell an at least partially double-stranded RNA molecule, said polycistronic RNA molecule comprising

a) at least one first ribonucleotide sequence that is substantially identical to at least a part of a target nucleotide sequence of at least one target gene, and

b) at least one second ribonucleotide sequence, which is substantially complementary to said first nucleotide sequence and is capable to hybridize to said first nucleotide sequence to form a double-stranded RNA structure, and

c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

The methods of the invention will lead to better results and/or higher efficiencies when compared to the methods using conventional sense, antisense, or double-stranded RNA nucleotide sequences and it is believed that other sequence-specific mechanisms regulating the phenotypic expression of target nucleic acids might be involved and/or triggered by the presence of the dsRNA molecules described in this specification.

### 1. Structure of the polycistronic RNA of the invention

The polycistronic RNA molecule of the invention has at least two important features:

1. The RNA molecule is polycistronic: The term "polycistronic RNA" or "polycistronic RNA molecule" as used herein refers to a ribonucleotide molecule that contains two or more ribonucleotide sequence regions ("cistrons") within the same RNA transcript. Each cistron encodes a functional RNA molecule (such as tRNA, rRNA, or a first or second ribonucleic acid sequence as defined herein) or a single polypeptide. The cistrons are released by posttranscriptional processing from the polycistronic RNA molecule. As the result of this processing the polycistronic RNA molecule is endonucleolytically cleaved into two or more RNA molecules. Preferably this cleavage results in excision of the removable RNA element from said third ribonucleic acid without subsequently covalently joining the sequences comprising said first and said second ribonucleotide sequence, respectively. For example in a linear polycistronic RNA molecule comprising only one first ribonucleic acid, one second ribonucleic acid, and one third ribonucleic acid with one removable RNA element, the processing will result in three subsequent RNA molecules.
2. The RNA molecule is capable to generate in an eukaryotic cell an at least partially double-stranded RNA molecule. The term to "generate" is to be understood in the broad sense it may comprise a direct formation of a double-stranded RNA molecule (which may subsequently be processed by excision of said third ribonucleic acid) but also excision of said third ribonucleic acid and a subsequent hybridization of the separate first and second ribonucleic acid to a double-stranded RNA molecule.

**1.1 The removable RNA element**

The third ribonucleotide sequence comprised in the polycistronic RNA of the invention comprises at least one removable RNA element.

The term "removable RNA element" as used herein means a ribonucleotide sequence which can be removed or excised from the third ribonucleotide sequence by the RNA processing mechanism of an eukaryotic cell without subsequently (i.e. after removal) covalently joining the resulting sequences comprising said first and said second ribonucleotide sequence, respectively. The term "remove" is to be understood in the broad sense and may comprise various biochemical or cellular mechanism (see e.g., Leader et al. (1997) The EMBO Journal 18, 5742-5751). In the context of this application the term "remove" or "removal" has the same meaning than the terms "excision" or "processing".

In consequence of the removal of the removable RNA element, the polycistronic RNA molecule of the invention is processed within an eukaryotic host cell resulting in at least two, preferably three ribonucleotide molecules (e.g., one comprising the first ribonucleotide sequence and one comprising the second ribonucleotide sequence, and - preferably - one comprising the removable RNA element). Said resulting ribonucleotide molecules comprising said first and said second ribonucleotide sequence, respectively, are capable to form in an eukaryotic host cell a dsRNA molecule by intermolecular hybridization. In contrast to intron sequences, which are described in the art to be incorporated into dsRNA molecules and which in consequence of the cellular intron-exon splicing mechanism result in a covalent linkage of its flanking sequences after its removal from the original RNA molecule, the polycistronic RNA molecule of the invention results in separate strands. After removal of the RNA element the remaining ends (i.e. of the sequences comprising said first and said second ribonucleotide sequence, respectively) are not covalently linked. They may however be associated by base-pairing to form a double-stranded RNA molecule.

Various elements are known in the art, which are suitable for use as removable RNA element, i.e. for removal by the RNA processing machinery of host cells. Preferably, the third ribonucleotide sequence comprises a RNA sequence, which is endonucleolyticaly cleaved at least at one (preferably two or more) defined site. Preferably at least two sites are flanking the removable RNA element thereby mediating its excision from the polycistronic RNA.

All RNAs that are processed after transcription to release individual sequences (i.e., individual genes or smaller fragment, e.g., by defined "tailoring" of the RNA termini) are suitable to be inserted into said third ribonucleotide sequence as a source (hereinafter "pre-RNA-element") for the removable RNA element (i.e., as the "spacer" between said first and said second ribonucleotide sequence). Preferably such pre-RNA-elements can be derived from naturally occurring eukaryotic polycistronic RNA molecules or from RNA molecules, which otherwise undergo defined endonucleolytic RNA processing (such as certain monocistronic tRNA molecules). More preferably, the pre-RNA-element is derived from a plant polycistronic RNA or a plant RNA, which otherwise undergoes defined endonucleolytic RNA processing. Plant polycistronic RNAs are for example described for Arabidopsis and com (Leader et al. (1997) EMBO J 18, 5742-5751; Brown, JWS et al. (2001) RNA 7, 1817-1832; Liang-Hu, Q. et al (2001) Nucleic Acid Research 29 (7), 1623-1630; Kruszka, K et al. (2003) EMBO J 22 (3), 5742-5751). The processing of polycistronic RNA in plants is mechanistically not yet fully understood. The process has been shown to be independent of splicing.

Preferably, the removable RNA element is a non-messenger RNA (nmRNA), more preferably a small non-messenger RNA (snmRNA) (Hüttenhofer A, et al., (2002) Current Opinion in Chemical Biology 6:835-843; Eddy (2001) Nature Reviews Genetics 2:919-929). Known classes of snmRNAs are, for example, tRNAs that serve as essential components of the protein-synthesizing machinery, small nuclear RNAs (snRNAs) required for splicing of pre-mRNAs, or small nucleolar RNA (snoRNAs), which are involved in modification of other RNAs. The corresponding pre-RNA-elemebt is the respective pre molecule (e.g., the pre-snoRNA, pre-tRNA) as expressed and present prior to the post-transcriptional RNA processing. The removable RNA element (or its pre-RNA-element) can be selected - without limitation - from the group comprising

1. snoRNAs: Small nucleolar RNAs (snoRNAs) directing modification of ribosomal RNAs (rRNA), U RNAs and tRNAs have been identified in all eukaryotes and in Archaea (for reviews see Kruszka et al. (2003) EMBO J 22(3): 621-632; Hüttenhofer A, et al., (2002) Current Opinion in Chemical Biology 6:835-843). They fall into two large sub-families, box C/D and H/ACA snoRNAs that direct site-specific 2' -O- ribose methylation and pseudouridylation, respectively, of rRNAs or spliceosomal snRNAs. Both snoRNA types function through specific base-pairing at the modification site, in both cases as small ribonucleoprotein particles. The C/D snoRNAs have two phylogenetically conserved C (RUGAUGA) and D (CUGA) motifs flanked by short inverted repeats at the 5' and 3' termini of the snoRNA, respectively. In their central region, they contain an additional, less conserved copy of box D and box C, termed box D' and C', respectively. Immediately upstream from box D or/and box D', they exhibit one or two antisense elements (AEs) (i.e. 10 to 21 nt sequence(s) complementary to the cognate RNA target). Each AE guides a 2'-O-methylation onto the nucleotide located 5 bp upstream from the D/D' box in the bimolecular RNA duplex (centre). Schematic structure of box H/ACA snoRNAs and pseudouridylation guide RNA duplex. Box H/ACA share a common secondary structure consisting of two large hairpins linked by a hinge and followed by a short tail. Boxes H (ANANNA) and ACA are located in the hinge and tail, respectively. The internal loop of one or both hairpin(s) contains a bipartite AE (i.e. a pair of 3-10 nt sequences complementary to nucleotides flanking the substrate uridine in target RNA (center)). The bipartite guide duplex is at least 9 bp and the distance between the pseudouridylation site and the downstream H or ACA box is 13-16 nt. Conserved box motifs, stem structures and sequence complementarities to the target RNA provide the basis for computer search for both snoRNA families. Searches has be performed and published for the yeast *Saccharomyces cerevisiae* (Lowe TM & Eddy SR (1999) *Science,* 283:1168-117116) and the plant *Arabidopsis thaliana* (Bameche F et al. (2001) J Mol Biol 311:57-73; Brown JW et al. (2001) RNA, 7: 1817-1832; Liang-Hu Q et al. (2001) Nucleic Acids Res, 29:1623-163017-19), and other specimen (see Hüttenhofer A, et al., (2002) Current Opinion in Chemical Biology 6:835-843 and the references cited therein for a review).
117 different C/D snoRNA genes are expressed in Arabidopsis thaliana (Bameche F et al. (2001) J Mol Biol 311: 57-73; Brown JW et al. (2001) RNA, 7:1817-1832; Liang-Hu Q et al. (2001) Nucleic Acids Res, 29:1623-163017-19). Moreover, nearly 50% of these snoRNAs display multiple isoforms resulting from gene and chromosomal duplication events, which generated a massive expansion of snoRNA genes, a situation unique to plants (Barneche et al., 2001; Brown et al., 2001). The plant snoRNAs are also distinguished by their modes of expression. Processing from polycistronic snoRNA clusters is widespread in plants (Bameche F et al. (2001) J Mol Biol 311:57-73; Brown JW et al. (2001) RNA, 7:1817-1832; Liang-Hu Q et al. (2001) Nucleic Acids Res, 29:1623-163017-19). Most Arabidopsis snoRNA genes are organized in independent clusters transcribed from a single promoter producing a polycistronic precursor, as first described for the C/D snoRNA U14 (Leader et al., 1997). The polycistronic precursor is then processed by endonucleolytic cleavages and exonucleolytic trimming to release the mature snoRNAs (Leader et al., 1999). Intronic snoRNAs also exist in Arabidopsis but are not frequent. Remarkably, three clusters were found within introns of protein coding genes (Barneche et al., 2001; Brown et al., (2001). These intronic clusters are unique to plants and occur frequently in rice, a monocotyledonous species (Liang et al., 2002).

2. miRNA: A large number of different miRNAs (MicroRNA) have been identified so far in different species. Northern blot analyses have been done for many of these RNAs, and generally show both a 21-24- nucleotide form (presumably the active miRNA) and, often, a less abundant ~70-nucleotide form (presumably the precursor stem-loop). The miRNA genes are often clustered in the genome (Lau, NC et al. (2001) Science 294, 858-862; Lagos-Quintana, M et al. (2001) Science 294, 853-858) and are presumably coexpressed in polycistronic precursor transcripts (Eddy (2001) Nature Reviews Genetics 2:919-929).

3. tRNA: The term tRNAs as used herein refers to noncoding RNAs that are not translated in protein but function as RNA molecules. tRNAs have two functions, they are chemically linked to a particular amino acid and they recognize a codon in mRNA, so that the amino acid can be added at the correct place in a growing peptide chain in the protein synthesis. tRNAs share a specific secondary structure which is necessary for that functions. The structure is formed by stem loop arrangements within the respective tRNA molecule and form a structure with 4 arms. The four stems are short double helices stabilized by Watson-Crick base pairing, three stems bear loops comprising seven to eight bases at their ends. The amino acid binding arm, the acceptor arm is not forming a loop. The opposing stem-loop forms the anticodon stem-loop necessary for correct recognition of the codon in the mRNA. The right arm is the TΨC stem-loop, the left arm is the D stem-loop. In addition, a variable arm may be located between anticodon stem-loop and TΨC stem-loop. The three dimensional structure of tRNAs has an L shape with the anticodon stem-loop and the acceptor stem-loop forming the ends of the two arms. tRNAs may be expressed in monocistronic or polycistronic forms. In the monocistronic case the tRNAs are synthesized as pre-tRNAs about 100-150 nucleotides in length. The pre-tRNA is subsequently processed to the final tRNA of about 70-80 nucleotides. Processing of pre tRNA to the final tRNA is a complex process and involves a series of proteins and RNA/protein complexes. In a first step the RNA/protein complex RNaseP binds the pre tRNA and cleaves endonucleolyticaly the 5' end. The processing of the 3' also comprises an endonucleolytic cleavage. In both cases, the binding and processing of the pre-tRNA is dependent on the secondary structure of the RNA rather than the sequence (Frank DN, Pace NR Annu.Rev.Biochem. 67, 153-180 (1998); Schiffer et al. (2001) Biochemistry 40, 8264-8272). The processing includes exonucleolytic processing of the 5'and 3'ends, the addition of CCA to the 3'end and several typical modifications of the nucleotides within the tRNA molecule. Also from polycistronic tRNA clusters the final tRNA is released by endonucleolytic processing. In case of Arabidopsis, some 630 tRNA genes have been identified in the genome (published for example on the web sites of the INRA institute (France, http://www.inra.fr/Internet/Produits/TAARSAT/english.html) and University of California Santa Cruz UCSC (http://lowelab.ucsc.edu/GtRNAdb/Athal/)). The tRNA prediction can be performed by software such as *tRNAscan-SE 1.21* which is publicly available (http://lowelab.ucsc.edu/tRNAscan-SEI).

Other ribonucleotide sequences such as, for example multimeric versions or combinations of the above mentioned preferred snmRNAs (e.g., multiple snoRNAs or combined genes for tRNAs and snoRNAs) are also suitable.

Various sequences are suitable for the removable RNA element. Preferably, said third ribonucleotide sequence of the polycistronic RNA molecule of the invention comprises a sequence, which is at least 60%, preferably 80%, more preferably, 90%, most preferably 95% identical to at least one transcribed RNA selected from the group consisting of tRNAs, rRNAs and snoRNAs and their precursor molecules. Preferably, said transcribed RNA is a transcribed RNA of an eukaryotic organism, more preferably of a plant or mammalian organism. In another preferred embodiment the third ribonucleotide sequence and/or the removable RNA element is heterologous to the first and/or the second ribonucleotide sequence (i.e. not linked to said sequences in its natural occurrence).

The removable RNA element (or the corresponding pre-RNA-element) may have deletions in comparison to its natural occurring counterpart. Sequences, which are not essential for the removal may be deleted without affecting functionality of the removable RNA element. More specifically, sequences may be deleted to an extent that efficiency of the removal (i.e. excision) remains substantially unaffected. Preferably by the deletion efficiency of the removal is reduced by not more than 80%, preferably, not more than 50%, more preferably not more than 30%, most preferably not more than 10%. Ideally efficiency of removable is unchanged in comparison to the naturally occurring RNA sequence under otherwise unchanged conditions. Preferably, the pre-RNA-element comprises all elements required for RNA processing. In case of a tRNA or snoRNA such elements may include sequences localized upstream and downstream of the endonucleolytic cleavage site (i.e. not comprised in the removed removable RNA element). For example, the 3'processing step of a tRNA needs at least the acceptor stem and the two adjacent stem-loops, whereas the anticodon stem is not necessary (Schiffer et al. (2001) Biochemistry 40, 8264-8272). 5' processing of tRNAs by Ribonuclease P needs at least the acceptor arm, the TψC stem-loop and at least one of the other stems of tRNAs (Schon, A. (1999) FEMS Microbiol. Rev. 23, pp 391 - 406). Therefore, also deletions of tRNA genes lacking the respective stem-loops are suitable as spacers in RNAi constructs. For the snoRNAs it is preferred to include the entire cluster (i.e. the entire pre-RNA) into the polycistronic RNA (e.g., the whole cluster 9 comprising the genes z43, snR74 (z4) and snR60 (z15) was cloned; see Examples for details).

One or more of the elements required for removal of the removable RNA element may remain attached to the sequences comprising said first and said second ribonucleotide sequences, respectively. Accordingly, the polycistronic RNA of the invention may comprise additional elements necessary for correct and efficient processing, which after said processing remain attached to said first or said second ribonucleotide sequence. Said additional elements within the polycistronic RNA may for example enable, enhance, facilitate or otherwise influence removal of said removable RNA element. Said additional elements may flank directly the removable RNA element but can be also localized in other locations within the polycistronic RNA molecule.

Preferably, the pre-RNA-element (e.g., a pre-tRNA or pre-snoRNA) contains at least the functional RNA processing sites at each end. For example, without limitation, in the constructs illustrated herein, removable RNA elements have been used to form the hairpin loop connecting two antiparallel RNA strands. Preferably, the removable RNA element specified under c) is a tRNAs or snoRNAs of a native plant or mammal.

Nucleic acid sequences of such removable RNA elements can be derived from a multitude of sources, including, without limitation, databases such as EMBL and Genbank found at www-ebi.ac.uk/swisprot/; www-expasy.ch/; www-embl-heidelberg.de/; and www-ncbi.nlm.nih.gov. Nucleic acid sequences of such removable RNA elements can also be derived, without limitation, from sources such as the GENSCAN program found at //genes.mit.edu/GENSCAN.html. In a further embodiment, additional removable RNA elements may be obtained by any method by which additional removable RNA elements may be identified. In a preferred embodiment, additional removable RNA elements may be obtained by screening a genomic library with a probe of either known removable RNA element sequences. Additional removable RNA elements may, for example without limitation, be amplified by PCR and used in an embodiment of the present invention.

The following sequences (or functionally equivalent fragments thereof) are preferably used as spacers in polycistronic RNA molecules of the invention:

a) Arabidopsis snoRNA gene cluster 9 (SEQ ID NO: 1), comprising the snoRNA genes z43 (bp 47-155), snR74(z4) (bp 233-388) and snR60(z15) (bp 496-563):

5'-**ttcatcttcttcttctggtgttaaaacttttgtttttggtaaaagt**aatggacagtgacgattgatattaaggtctc
gttcgtcgtaaaaacggccgtaaaaagcctatttgccgaccattctgttatatcaccacccatattctgagtcctatt**ga**
**ttttgtttttcaatctttttctaatcgatttttggccaatgatttaatcaaaattgacaagcatatgtctga**attaat
ccttgtttgataaactctaatcttctctatgaggcctttttcaattacctttcaaatccgatttcgtatttatttctttggttctgttt
ttggttgatttagtggcctatgatttaaatgattatagacaagcatatgtctgaattaat**ccttgttgataaatctaatctt**
**ctctatgaggtctaaatccgattttgtttttggttattggttttaaaattgtcttggatttatttgatgagaaaaga**
**ggaaatt**gaaaggcatcaagtgatgattgataacgcatagttcagatgataaattctattatgattaacatcttc**agc**
**atagtctttcgctcctatctgatgaccctttcctctctattttcatctattatccaaatttttcaatctcaaattgttc**
**cttaagt**-3'

The sequence described above (SEQ ID NO: 1) represents the sequence encoding the pre-snoRNA. From resulting transcript the regions underlined and in bold letters are deleted. The regions in between said deleted regions form the individual, mature snoRNA molecules. The entire cluster can be employed as sequence encoding the third ribonucleotide sequence. Alternatively parts of said sequence are used, which at least release one snoRNA sequence. Preferably, said parts comprise at least one mature snoRNA encoding region (preferably selected from the group consisting of base 47-155, base 233-388, and base 496-563 of SEQ ID NO: 1, respectively) flanked on each side by sequences suitable to allow correct RNA processing (preferably the respective underlines regions in bold letters). For example such fragments may comprise base 1 to 232, or base 156 to 495, or base 389 to 652 of the sequence described above (SEQ ID NO: 1)

b) Sequence of Arabidopsis snoRNA cluster 2, comprising the snoRNA genes z44a (bp 41-134), z44b (bp 218-313) and u24 (bp 362-460) (SEQ ID NO: 2):

5'-**ttggttgaatattggaacttttgtggagcagtattgatat**tgatggtgaggatgacgaaaaaatcattcggatt
ccctttgaattcctccggaaacatgtgattatataatcagctacttctgactcatcatca**tcatcataacaccttttttgtt**
**tttcgccgatttagaaaacgtttttgaggtcttttatgacattttctttgataaattttt**tgatggtaaagatgacga
aaaaaatcgttcggattccctttgaattccctcgggaaagatgtgatcacaaaccagctacttctgattttaccatca**t**
**cgatatagatttcccaaaataagttccacttttgaattgtttttgaa**tgaggccagtgatgtaataaaaatatttgct
actcttgatgaagaactttgttctttcagttgatgattggtttaccaccaagatctctgagggcctta**gttttctactgttta**
**tct**-3'

The sequence described above (SEQ ID NO: 2) represents the sequence encoding the pre-snoRNA. From resulting transcript the regions underlined and in bold letters are deleted. The regions in between said deleted regions form the individual, mature snoRNA molecules. The entire cluster can be employed as sequence encoding the third ribonucleotide sequence. Alternatively parts of said sequence are used, which at least release one snoRNA sequence. Preferably, said parts comprise at least one mature snoRNA encoding region (preferably selected from the group consisting of base 41-134, base 218-313, and base 362-460 of SEQ ID NO: 2, respectively) flanked on each side by sequences suitable to allow correct RNA processing (preferably the respective underlines regions in bold letters). For example such fragments may comprise base 1 to 217, or base 135 to 361, or base 314 to 478 of the sequence described above (SEQ ID NO: 2).

c) Sequence encoding Arabidopsis thaliana $tRNA^{Gln}$ (see also GenBank Acc.No: D50935) (SEQ ID NO: 3) with the mature tRNA from base 129 to 200:

**5'-agacaaagaacactgcaatctgtggtgctgctggactgctgatgttgaatctattcattggccgtgtcta**
**aaatacaatattttggattagattctaaactggtacatgcaacagaacaagcacctga**ggttctatggtgtagt
ggttagcactctggactttgaatccagcgacctgggttcgactcccggtaggacct**ctcatgc-3'**

The sequence described above (SEQ ID NO: 3) represents the sequence encoding the pre-tRNA. From resulting transcript the regions underlined and in bold letters are deleted. The region in between said deleted regions forms the mature tRNA molecule. The entire gene can be employed as sequence encoding the third ribonucleotide sequence. Alternatively parts of said sequence are used, which at least release the mature tRNA sequence. Preferably, said parts comprise at least the mature tRNA encoding region (base 129-200 of SEQ ID NO: 3) flanked on each side by sequences suitable to allow correct RNA processing (preferably the respective underlines regions in bold letters).

d) Sequence encoding Arabidopsis thaliana $tRNA^{His}$ (SEQ ID NO: 4) with the mature tRNA from base 51 to 122:

5'-**<u>TTATTAAATCTTTGGTTAATTAAATACTAAGGGAAAGAAATAACACAAAA</u>**
GTGGTTGTAGTATAGCGGTTAGTATCCCACGTTGTGGCCGTGGGGACCCGG
GCTCGAATCCCGGCAGCCACA**<u>CTTTTTGTTACCTCTTCTTTTTTAT</u>**-3'

The sequence described above (SEQ ID NO: 4) represents the sequence encoding the pre-tRNA. From resulting transcript the regions underlined and in bold letters are deleted. The region in between said deleted regions forms the mature tRNA molecule. The entire gene can be employed as sequence encoding the third ribonucleotide sequence. Alternatively parts of said sequence are used, which at least release the mature tRNA sequence. Preferably, said parts comprise at least the mature tRNA encoding region (base 51-122 of SEQ ID NO: 4) flanked on each side by sequences suitable to allow correct RNA processing (preferably the respective underlines regions in bold letters).

e) Sequence encoding Arabidopsis thaliana $tRNA^{Met}$ (SEQ ID NO: 5) with the mature tRNA from base 51 to 135:

**<u>5'-ATGGAAAATAGGGGTAAAAAATACATAATATTACGGTACTCAACAACATT</u>**
GGGGTGGTGGCGCAGTTGGCTAGCGCGTAGGTCTCATAGCTAGTGAGTGAT
CCTGAGGTCGAGAGTTCGAGCCTCTCTCACCCCA**<u>ATATTTCTTTTTGCATTG-</u>**
**<u>TTTTTAT</u>**-3'

The sequence described above (SEQ ID NO: 5) represents the sequence encoding the pre-tRNA. From resulting transcript the regions underlined and in bold letters are deleted. The region in between said deleted regions forms the mature tRNA molecule. The entire gene can be employed as sequence encoding the third ribonucleotide sequence. Alternatively parts of said sequence are used, which at least release the mature tRNA sequence. Preferably, said parts comprise at least the mature tRNA encoding region (base 51-135 of SEQ ID NO: 5) flanked on each side by sequences suitable to allow correct RNA processing (preferably the respective underlines regions in bold letters).

f) Sequence of Arabidopsis dicistronic tRNA snoRNA gene (GenBank Acc.-No. AJ506163) comprising a $tRNA^{Gly}$ gene (bp 14-84) and the snoRNA gene R43.1 (bp 85-168) (SEQ ID NO: 6):

5'-**<u>aaggttcaacaaa</u>**gcaccagtggtctagtggtagaatagtaccctgccacggtacagacccgggttcgattc
ccggctggtgc***at***gagctgtgatgaaattggctttagtattggttgggtccaatgcattctcctgaatatcagatgatttc
gcctactctgagctc**<u>tatttttaatttttatttt</u>**-3'

The sequence described above (SEQ ID NO: 6) represents the sequence encoding the pre-RNA. From resulting transcript the regions underlined and in bold letters are deleted. The region in between said deleted regions forms the mature tRNA and snoRNA molecules. The restriction site to separate the tRNA and snoRNA is given in bold cursive letters. The entire cluster can be employed as sequence encoding the third ribonucleotide sequence. Alternatively parts of said sequence are used, which at least release the mature tRNA or mature snoRNA sequence. Preferably, said parts comprise at least the mature tRNA encoding region (base 85-168 of SEQ ID NO: 6) or the mature snoRNA encoding region (base 14-84 of SEQ ID NO: 6) flanked on each side by sequences suitable to allow correct RNA processing (preferably the respective underlines regions in bold letters).

Any of the above-described sequences (or their respective homologues or orthologues from other species, preferably plant species) is useful to be employed as sequence encoding said third ribonucleotide sequence (e.g., as a spacer sequence between the self-complementary sequences in a RNAi construct).

As described above, not the entire sequence of the pre-RNA described above is necessary. Parts, which are not required for the RNA processing, may be deleted, without affecting the functionality of the sequence. For example a functional equivalent fragment suitable as a spacer sequence should comprise at least the sequence of a tRNA, and - in order to enable processing - preferably at least 10bp upstream of the 5' and downstream of the 3' end of the tRNA respectively.

Preferably, said third ribonucleotide sequence comprises a native tRNA, rRNA or snoRNA or a precursor molecule thereof from a native plant or a mammal. Most preferred the third ribonucleotide sequence comprises at least one sequences encoded by a sequence selected from the group of

a) the sequences as described by SEQ ID NO: 1, 2, 3, 4, 5, and 6, and

b) a sequence having an identity of at least 60%, preferably 80%, more preferably, 90%, most preferably 95% to a sequence as described by SEQ ID NO: 1, 2, 3, 4, 5, or 6 and comprising the functional elements necessary for RNA processing, and

c) a sequences capable to hybridize (preferably under high stringency conditions) with a sequence as described by SEQ ID NO: 1, 2, 3, 4, 5, or 6 or the complement thereof and comprising the functional elements necessary for RNA processing.

### 1.2. Structure of the polycistronic RNA of the invention

The polycistronic RNA molecule of the invention is - beside the third ribonucleotide sequence and the removable RNA element - comprising

a) at least one first ribonucleotide sequence that is substantially identical to at least a part of a target nucleotide sequence of at least one target gene, and

b) at least one second ribonucleotide sequence which is substantially complementary to said first nucleotide sequence and is capable to hybridizes to said first nucleotide sequence to form a double-stranded RNA structure.

The order of the first and the second ribonucleotide sequence in the polycistronic RNA molecule is thought not to be critical.

Because said first and said second ribonucleotide sequence are substantially complementary (as defined above) they can hybridize to each other, thus forming an at least partially double-stranded RNA (dsRNA) structure. The dsRNA structure can be formed prior or after removal of the removable RNA element. In an preferred embodiment, the first and the second ribonucleotide sequence are capable to hybridize to each other in an eukaryotic cell, thus forming an at least partially double-stranded RNA (dsRNA) structure. The capability of said first and said second ribonucleotide sequence to hybridize with each other may be assessed under various conditions, preferably under conditions which simulate the conditions in an eukaryotic cell environment. Alternatively and preferably the sequences hybridize to each other under low stringency, more preferably high stringency conditions (as defined above).

As described above, prior to the RNA processing (i.e., removal of the removable RNA element) the polycistronic RNA molecule of the invention is forming hairpin dsRNA structure, wherein the loop of the hairpin is formed by the removable RNA element. Thus, in a preferred embodiment, the first and said second ribonucleotide sequences are arranged in an antiparallel structure (an "inverted repeat") capable to form (preferably in an eukaryotic cell) a hairpin structure. After RNA processing in the eukaryotic host cell, the at least partially dsRNA structure consists of at least two ribonucleotide strands, which are not covalently linked to each other but hybridize with each other to form a dsRNA molecule. In an preferred embodiment of the invention, removal of said removable RNA element provides (or generates) at least one short interfering double stranded RNA (siRNA), which - preferably - does not produce a significant PKR-dependent response in the host cells at concentrations effective for attenuating expression of the target gene. More preferably, said provided siRNA is 15 to 45 base pairs in length. Preferably this siRNA is at least partially double-stranded.

For example the polycistronic RNA molecule may have the following schematic structure:

$$5'\text{-F(1)-(RE)-S(1)-3'}$$

wherein F(1) is the first ribonucleotide sequence, S(1) is the second ribonucleotide sequence, and RE is the removable RNA element. This molecule is capable to form a hairpin secondary structure (see Fig. 1 left part) and is processed by removal of the removable RNA into a two-strand double-stranded RNA molecule (without be restricted to the order of the various steps i.e. whether RNA processing (step II in Fig. 1) or formation of the double-stranded structure (step I in Fig. 1) occurs first) as schematically shown in Fig. 1.

Preferably, the first ribonucleotide sequence and/or the second ribonucleotide sequence have a length of at least 19 nucleotides, preferably a length from about 20 to about 500 nucleotides. In a preferred embodiment, each of the first and/or the second ribonucleotide sequences has a length of at least 19 base pairs, preferably at least 50 base pairs, especially preferably at least 100 base pairs, very especially preferably at least 250 base pairs. In a furthermore preferred embodiment, each of the first and/or the second ribonucleotide sequences has a length of an integer multiple of 21 or 22 base pairs, that is to say, for example, 21, 22, 42, 43, 44, 63, 64, 65, 66, 84, 85, 86, 87, 88, 105, 106, 107, 108, 109, 110, 126, 127, 128, 129, .131, 132, 147, 148, 149, 150, 151, 152, 153, 154, 168, 169, 170, 171, 172, 173, 174, 175, 176, 189, 190, 191, 192, 193, 194, 195, 196, 197, 198, 210, 211, 212, 213, 214, 215, 216, 217, 218, 219 or 220 base pairs, preferably 21, 22, 42, 44, 63, 66, 84, 88, 105, 110, 126, 132, 147, 154, 168, 176, 189, 198, 210 or 220 base pairs, very especially preferably 21, 42, 63, 84, 105, 126, 147, 168, 189 or 210 base pairs, most preferably 180 or 210 base pairs.

The polycistronic RNA molecule (or the dsRNA molecule resulting therefrom after removal of the removable RNA element) is at least partially double-stranded. The part being double-stranded has preferably a length sufficient to keep the RNA molecule stable. Minimally, to keep the RNA molecule stable, it has a minimum of 11 to 30 nucleotides involved in a double-stranded sequence, depending upon the composition of the polynucleotide sequence and a $\Delta G$ of about -9.2 kcal/mol. As known in the art, $\Delta G$ defines the state of minimal external energy required to keep a molecular configuration stable [see, e.g., Jaeger *et al.*, Proc. Nati. Acad. Sci., USA, 20:7706-7710 (1989); and Soler and Jankowski, Math. Biosci., 2:167-190 (1991)]. Based on this minimum, preferably at least 10% of this partially double-stranded RNA molecule sequence is double-stranded. Alternatively, the double stranded portion of these RNA molecules can be at least 30% of the sequence. In another embodiment, the double-stranded portion of these molecules can be at least 50% of the sequence. In still another embodiment, the double stranded portion of these molecules can be at least 70% of the sequence. In another embodiment, the double stranded portion of these molecules can be at least 90% of the sequence. In another embodiment, the entire sequence can be double stranded. Alternatively, the double-stranded portion of these molecules can occur at either or both termini, or in some middle portion of the sequence, if the molecule is linear.

Preferably, the sequence of the polycistronic RNA molecule (or the dsRNA molecule resulting therefrom after removal of the removable RNA element) is chosen in such a way that the desired dsRNA structure has in each case the least free energy after formation of the duplex in comparison with other possible folding variants of the primary structure of the double-stranded RNA. This can be ensured for example by avoiding sequence duplications and the like. The specific secondary structure can be predicted and optimized for example using suitable computer programs (for example FOLDRNA; Zuker and Stiegler (1981) Nucleic Acids Res 9(1):133-48; Zuker, M. (1989) Methods

Enzymol. 180, 262-288).

There are various possibilities for the primary structure of the polycistronic RNA of the invention. Furthermore more than one first sequence and second sequence can be comprised in the polycistronic molecule, wherein at least one of these pairs is separated by a removable RNA element. Thus, the polycistronic RNA molecule may comprise several artificial hairpin structures, which may be designed to reduce the phenotypic expression of different nucleic acids of interest. The person skilled in the art is aware of various forms and structures for such a polycistronic RNA molecule. Those listed herein below are to be understood as examples, but not by way of limitation:

a) It is possible first to add the first ribonucleotide sequences (F) of the individual subunits to one another, which is followed by a sequential arrangement of the essentially complementary second ribonucleotide sequences (S). The number of the units n is greater than or equal to two. This gives rise to a structure with a single hairpin. At least one of the linker between said first and said second ribonucleotide sequence is an removable RNA element as described above. The primary structure of the polycistronic RNA here can be for example as in the following scheme (see also Fig. 2A):

**5'-F(1)-F(2)-.....-F(n)-(RE)-S(n)-....-S(2)-S(1)-3'**

b) It is possible first to add the first ribonucleotide sequence (F) and the essentially complementary second ribonucleotide sequences (S) of the first subunits to one another, which is followed by the sequential arrangement of "sense" and "antisense" ribonucleotide sequences of the further subunits. The number of the units n is greater than or equal to two. This gives rise to a structure with several hairpins. The primary structure of the polycistronic RNA here can be for example as in the following scheme (see also Fig. 2B):

5'-F(1)-(RE1)-S(1)-(RE2)-F(2)-(RE3)-S(2)-(RE4)-.....-F(n)-(REn)-S(n)-3'

The secondary structure and processing of these molecules is shown schematically in Fig. 2 A +B. These molecules are capable to form a single hairpin secondary structure (see Fig. 2A left part) or multiple hairpin secondary structures (see Fig. 2B left part). In both cases the molecule is processed by removal of the removable RNA into a multiple double-stranded RNA molecules (without be restricted to the order of the various steps i.e. whether RNA processing (step II in Fig. 2 A + B) or formation of the double-stranded structure (step I in Fig. 2 A+ B) occurs first) as schematically shown in Fig. 2.

Alternatively (especially in molecules as depicted in Fig. 2B) one or more of the removable RNA elements (RE) may be replaced by other sequences (e.g., which may or may not be removed by RNA processing), wherein however at least one removable RNA element remains present in the polycistronic RNA molecule.

In cases were more than one first ribonucleic acid is comprised in the polycistronic RNA molecule of the invention, the target genes corresponding to said two or more first ribonucleic acid sequences may be identical and/or different. Different means preferably that said target genes not identical, more preferably that they have a percentage of identity of less 60%, preferably less than 50%, more preferably less than 40%.

Beside at least one removable RNA element the polycistronic RNA may include additional elements and linkers, which are preferentially located between the individual ribonucleotide sequences (such as the first and the second ribonucleotide sequence or between two sets of sequences). The RNA molecule resulting from the transcription of the chimeric DNA may comprise a spacer nucleotide sequence located between the sense and antisense nucleotide sequence. In the absence of such a spacer sequence, the RNA molecule will still be able to form a double-stranded RNA, particularly if the sense and antisense nucleotide sequence is larger than about 10 nucleotides and part of the sense and/or antisense nucleotide sequence will be used to form the loop allowing the base-pairing between the regions with sense and antisense nucleotide sequence and formation of a dsRNA molecule. It is expected that there are no length limits or sequence requirements associated with the spacer region, as long as these parameters do not interfere with the capability of the RNA regions with the sense and antisense nucleotide sequence to form a dsRNA. In a preferred embodiment, the spacer region varies in length from 4 to about 200 bp, but as previously mentioned, it may be absent.

Such additional elements may for example comprise introns, especially preferably an intron in sense orientation with respect to the 5'-3' orientation of the polycistronic RNA. The intron is preferably an intron of a plant gene, such as - for example - the intron 3 of the maize alcohol dehydrogenase 1 (Adh1) (GenBank Acc.-No.: AF044293; GI: 2828164), the intron 4 of the soya beta-conglycinin alpha subunit (GenBank Acc.-No.: AB051865); one of the introns of the pea rbcS-3A gene for the ribulose-1,5-bisphosphate carboxylase (RBC) small subunit (GenBank Acc.-No.: X04333). The skilled worker is familiar with these and further suitable introns (McCullough AJ & Schuler MA (1997) Nuc Acids Res 25: 1071-1077). For the application in the method according to the invention, the intron is preferably employed in combination with splice acceptor and splice donor sequences, which make it possible that the dsRNA is spliced out at a later point in time.

In another embodiment of the invention the polycistronic RNA molecule (or the dsRNA molecule resulting therefrom after removal of the removable RNA element) does not produce a functional protein, or alternatively, is not translated. The polycistronic RNA molecule (or the dsRNA molecule resulting therefrom after removal of the removable RNA element or the expression construct or vector for their expression) can be engineered in a variety of known ways, so as to optionally not express a functional protein or to optionally not interact with cellular factors involved in translation. Thus, for example, the molecules, preferably whether it be a synthesized RNA molecule or a molecule which becomes an RNA molecule in vivo, lacks a poly-adenylation sequence. Similarly, the polycistronic RNA molecule (or the dsRNA molecule resulting therefrom after removal of the removable RNA element) can lack a Kozak region necessary for protein translation. In another embodiment, the RNA molecule can also lack the native initiating methionine codon. In still another embodiment, the RNA molecule polynucleotide sequence lacks a cap structure. In yet a further embodiment, the RNA molecule has no signals for protein synthesis. In still another embodiment, the RNA molecule contains no coding sequence or a functionally inoperative coding sequence. In still another embodiment, the RNA sequence can be punctuated with intronic sequences. In yet a further embodiment, a hairpin sequence can be placed before the native initiation codon, if present. All such embodiments can be designed by resort to the known teachings of, e.g., such texts as cited herein.

In one embodiment, polynucleotides in the form of "lariats" may be utilized. Lariats contain a 2'-5' phosphodiester linkage as opposed to the usual 3'-5' linkage. Such structures are formed in splicing reactions catalyzed by spliceosomes and self-cleaving ribozymes. These structures are either intermediates or by-products of splicing reactions. They can be prepared in vivo through expression (transcription) in a cell or prepared in vitro. Lariats form when a free 5' phosphoryl group of either a ribose or deoxyribose becomes linked to the 2'-OH of a ribose in a loop back fashion. The lariats may contain 10 or more nucleotides in the loop or may be a complete circle, with the loop back linkage in each case being 2'-5'. A lariat linking the terminal nucleotides produces a circle-like structure. The loops and/or the stem can contain either the sense and the antisense sequences in tandem in a single molecule, or each single lariat contains either a sense or an antisense sequence. The lariats that contain sense and antisense in separate molecules may be administered together as a double-stranded form or the antisense lariat may be used singly to form a double strand with the mRNA in the cell. Lariats may be RNA or a DNA hybrid, with the 2'-5' linkage effected through the 2'--OH of the RNA portion of the hybrid [Rees C and Song Q. Nucl. Acid Res., 27, 2672-2681 (1999); Dame E *et al*, Biochemistry, 38, 3157-3167, 1999; Clement JQ et al, RNA, 5, 206-220, 1999; Block T and Hill J. J. Neurovirol., 3 313-321, 1997; Schindewolf C A and Domdey H., Nucl. Acid Res., 23, 1133-1139 (1995)]. In another embodiment, a circular RNA (or circular RNA-DNA hybrid) can be generated through a 2'-5' or a 3'-5' linkage of the termini. These may be generated enzymatically through RNA ligase reactions using a splinter to bring the ends in proximity in vitro, or through the use of self splicing ribozymes (in vivo and in vitro).

In yet a farther embodiment, the polycistronic RNA molecule of the invention is a capped linear RNA. In circumstances where cytoplasmic expression would not ordinarily result in capping of the RNA molecule, capping may be accomplished by various means including use of a capping enzyme, such as a vaccinia capping enzyme or an alphavirus capping enzyme. Capped RNA may be prepared in vitro or in vivo. RNA made in the nucleus by RNA pol II ordinarily is capped. Cytoplasmically expressed RNA may or may not be capped. Capping can be achieved by expressing capping enzymes of cytoplasmic viruses.

Still another embodiment of the synthetic RNA molecule is a circular RNA molecule that optionally forms a rod structure [see, e.g., K-S. Wang *et al*, Nature, 323:508-514 (1986)] or is partially double-stranded, and can be prepared according to the techniques described in Wang *et al*, Nucl. Acids Res., 22(12):2326-33 (1994); Matsumoto *et al*, Proc. Natl. Acad. Sci. USA. 87(19)7628-32 (1990); Proc. Natl. Acad. Sci., USA, 91(8):3117-21 (1994); Tsagris *et al*, Nucl. Acids Res., 19(7):1605-12 (1991); S. Braun *et al*, Nucl. Acids Res., 24(21):4152-7 (1996); Pasman *et al*, RNA, 2(6): 603-10 (1996); Zaphiropoulos, Proc. Natl. Acad. Sci., USA, 9(13):6536-41 (1996); Beaudry *et al*, Nucl. Acids Res., 23 (15):3064-6 (1995), all incorporated herein by reference.

### 3. Production and processing of the polycistronic RNA of the invention

The polycistronic RNA molecule (or the dsRNA molecule resulting therefrom after removal of the removable RNA element) can be produced and applied to the host cell or organism by various means, all familiar to the person skilled in the art. The polycistronic RNA molecules of the invention can be produced or synthesized by any method known in the art, e.g., using recombinant expression, enzymatic synthesis or chemical synthesis. The RNA molecules can be synthesized *in vitro* (e.g., using enzymatic synthesis and chemical synthesis) or *in vivo* (using recombinant DNA technology well known in the art).

For example, polycistronic RNA may be produced outside the eukaryotic target cell or may be produced recombinantly (e.g., by an expression construct) within the target cell. In one embodiment, the polycistronic RNA molecule of the invention can be produced by enzymatic synthetic methods or chemical synthetic methods in vitro. In another embodiment, the polycistronic RNA molecule may be generated in a recombinant culture, e.g., bacterial cells, isolated therefrom, and used in the methods discussed below. In another embodiment the another agent (such as an expression cassette or vector) generates the polycistronic RNA molecule in vivo after delivery to the target cell. The target cell is preferably a nematode, mammalian cell or a plant cell. For example the polycistronic RNA molecule (or a dsRNA molecule generated therefrom) can be

a) expressed from an expression construct or an expression vector in the target cell or organism, wherein the polycistronic RNA molecule is preferably processed into the dsRNA molecule by the internal RNA processing mechanism of the host cell, or

b) expressed from an expression construct in an in vivo or in vitro transcription system, wherein the polycistronic RNA molecule or the dsRNA molecule derived therefrom is purified from said transcription system and introduced into the host cell or organism (e.g., by feeding or injection), or

c) chemical synthesis of the polycistronic RNA molecule introduced into the host cell or organism (e.g., by feeding or injection), wherein the polycistronic RNA molecule is processed into the dsRNA molecule by the internal RNA processing mechanism of the host cell.

The polycistronic RNA of the invention can be processed by the RNA processing machinery of an eukaryotic cell, preferably the cell in which gene silencing should be achieved. However, processing can also be achieved in a first eukaryotic system (either in vitro or in -vivo) and the resulting dsRNA is subsequently introduced into the target eukaryotic cell. Processing in the context mean the correct removal of the removable RNA element. Processing of the invention results in providing one or more double stranded RNA molecules. Because of the removal of the removable RNA element, one or more of these double stranded RNA molecules may not be representing hairpin dsRNA but dsRNA with separate sense and antisense strands. More preferably the polycistronic RNA is processed to an siRNA species by said host cells. The duplex formation of the polycistronic RNA (or the dsRNA derived therefrom) can be initiated either outside (i.e. prior to the application to the cell) or within the cell.

### 3.2 Production of the polycistronic RNA of the invention by recombinant expression

The polycistronic RNA molecule of the invention can be made by recombinant expression. Thus, in one embodiment of the invention the polycistronic RNA is produced in the cell (preferably the eukaryotic cell where gene silencing should be achieved) by an expression construct or expression vector. The polycistronic RNA molecule can be made (e.g., expressed) directly in the eukaryotic target cell, where it can directly fulfill its function without the need of further introduction. Alternatively the polycistronic RNA molecule can be expressed in another cell, optionally purified, and subsequently delivered into the target cell. Thus, the RNA molecule of this invention can be made in a recombinant microorganism, e.g., bacteria and yeast or in a recombinant host cell or organism, e.g., plant or mammalian cells, and - optionally - isolated from the cultures thereof by conventional techniques. See, e.g., the techniques described in Sambrook *et al*, MOLECULAR CLONING, A LABORATORY MANUAL, 2nd Ed.; Cold Spring Harbor Laboratory Press, Cold Spring Harbor, N.Y., 1989, which is exemplary of laboratory manuals that detail these techniques, and the techniques described in US 5,824,538; 5,877,159 and 65,643,771, incorporated herein by reference.

Where the RNA molecules of the invention are formed *in vivo* they are preferably produced employing an expression construct or expression vector. More preferably the expression construct or vector is comprising a nucleic acid sequence, preferably a double stranded DNA molecule, encoding at least one of the above-described polycistronic RNA molecules of the invention, operably linked to a transcription regulating sequence (a promoter) which is capable to realize transcription of said nucleic acid sequence in the chosen host or target cell to produce a polycistronic RNA of the invention. Thus, another embodiment of the invention related to an expression construct comprising

a) a promoter functional in host cells (preferably in eukaryotic cells), and
b) a nucleic acid sequence encoding a polycistronic RNA molecule of the invention,

wherein said promoter a) is operably linked to said nucleic acid sequence b). Preferably the promoter a) is heterologous to said nucleic acid sequence b).

The use and production of an expression construct are known in the art (see also WO 97/32016; U.S. Pat. Nos. 5,593,874, 5,698,425, 5,712,135, 5,789,214, and 5,804,693; and the references cited therein).

For transcription from a transgene in vivo or an expression construct, a regulatory region (e.g., promoter, enhancer, silencer, splice donor and acceptor, polyadenylation) may be used to transcribe the polycistronic RNA. Inhibition may be targeted by specific transcription in an organ, tissue, or cell type; stimulation of an environmental condition (e.g., infection, stress, temperature, chemical inducers); and/or engineering transcription at a developmental stage or age. The RNA strands may or may not be polyadenylated; the RNA strands may or may not be capable of being translated into a polypeptide by a cell's translational apparatus. Examples for preferred expression constructs are described below for the specific application. However, it is desirable that the sequences which enable protein expression, e.g., Kozak regions, etc., are not included in these expression constructs of the invention.

Preferably, the expression construct is a DNA molecule, more preferably the expression construct is in a plasmid.

The expression construct is preferably comprised in an expression vector. Thus another embodiment of the invention relates to an expression vetor comprising an expression construct of the invention. The expression vector can be a DNA or RNA molecule, can be single stranded or double stranded, can be a plasmid or other type of vector (as defined above and specified for the various application and technical field below in detail). More preferably the expression vector is a double-stranded, circular plasmid DNA vector. A further embodiment of the invention relates to an expression vector comprising an expression construct of the invention. Examples of vectors (see above in the DEFINITION section for details) can be plasmids, cosmids, phages, viruses or else *Agrobacteria.* Preferably, the vector is a eukaryotic expression vector. More preferably, the eukaryotic expression vector is a viral vector or plasmid vector. In certain embodiments, the expression constructs or vectors are episomal, e.g., and transfection is transient. In other embodiments, the expression constructs or vectors are chromosomally integrated, e.g., to produce a stably transfected cell line. Preferred vectors for forming such stable cell lines are described in US 6,025,192 and WO/9812339, which are incorporated by reference herein. Vectors for expression in E.coli are preferably pQE70, pQE60 and pQE-9 (QIAGEN, Inc.); pBluescript vectors, Phagescript vectors, pNH8A, pNH16a, pNH18A, pNH46A (Stratagene Cloning Systems, Inc.); ptrc99a, pKK223-3, pKK233-3, pDR54.0, pRIT5 (Pharmacia Biotech, Inc.).

As described above (and for specific organisms and cells below in more detail), the expression construct and vector may be introduced into organisms or cells. Yet another embodiment of the invention relates to a transformed cell or non-human organism comprising an expression construct or an expression vector of the invention. Preferably, said expression construct or expression vector is inserted into the genome (preferably the chromosomal or plastid DNA) of said cell or organism. Preferably, said cell or organism is selected from the group of mammalian, bacterial, fungal, nematode or plant cells and organism.

The transgenic expression cassettes can be inserted into the vector (preferably a plasmid vector) via a suitable restriction cleavage site. The resulting vector is first introduced into *E.coli*. Correctly transformed *E.coli* are selected, grown, and the recombinant vector is obtained by methods with which the skilled worker is familiar. Restriction analysis and sequencing can be employed for verifying the cloning step. Preferred vectors are those, which make possible a stable integration of the expression cassette into the host genome. Suitable promoters and vector constructs are described in United States Patent Application No. 20040220130.

The vectors designed to produce the polycistronic RNA of the invention may desirably be designed to generate two or more, including a number of different polycistronic RNAs homologous and complementary to a target sequence. This approach is desirable in that a single vector may produce many, independently operative dsRNAs rather than a single dsRNA molecule from a single transcription unit and by producing a multiplicity of different dsRNAs, it is possible to self select for optimum effectiveness. Various means may be employed to achieve this, including autocatalytic sequences as well as sequences for cleavage to create random and/or predetermined splice sites.

The construction of polynucleotide constructs generally requires the use of vectors able to replicate in bacteria. A plethora of kits are commercially available for the purification of plasmids from bacteria. For their proper use, follow the manufacturer's instructions (see, for example, EasyPrep™, FlexiPrep™, both from Pharmacia Biotech; StrataClean™, from Stratagene; and, QIAprep™, Qiagen). The isolated and purified plasmids can then be further manipulated to produce other plasmids, used to transfect cells or incorporated into other vector systems (e.g., *Agrobacterium tumefaciens)* to infect and transform target cells or organism (preferably plants).

Still other suitable vector (or delivery agents) for introducing a polycistronic RNA of the invention into a target cell include live, attenuated or killed, inactivated viruses, and particularly recombinant viruses carrying the required RNA polynucleotide sequence discussed above. Such viruses may be designed similarly to recombinant viruses presently used to deliver genes to cells for gene therapy and the like, but preferably do not have the ability to express a protein or functional fragment of a protein. Among useful viruses or viral sequences which may be manipulated to provide the required RNA molecule to the mammalian cell in vivo are, without limitation, alphavirus, adenovirus, adeno-associated virus, baculoviruses, delta virus, pox viruses, hepatitis viruses, herpes viruses, papova viruses (such as SV40), poliovirus, pseudorabies viruses, retroviruses, vaccinia viruses, positive and negative stranded RNA viruses, viroids, and virusoids, or portions thereof. These various viral delivery agents may be designed by applying conventional techniques such as described in M. Di Nocola *et al*, Cancer Gene Ther., 5(6):350-6 (1998), among others, with the teachings of the present invention. A viral construct packaged into a viral particle would accomplish both efficient introduction of an expression construct into the cell and transcription of polycistronic RNA construct encoded by the expression construct.

Another delivery agent for providing the polycistronic RNA molecules of the invention in the target cell or organism include live, attenuated or killed, inactivated donor cells which have been transfected or infected in vitro with a synthetic RNA molecule or an expression cassette or vector as described above. These donor cells may then be administered or feed to the target organism (e.g., a mammal or a pathogen such as a nematode), as described in detail below, to stimulate the mechanism in the target organism which mediates this inhibitory effect. These donor cells are desirably eukaryotic cells, such as mammalian cells C127, 3T3, CHO, HeLa, human kidney 293, BHK cell lines, and COS-7 cells, and preferably are of the same mammalian species as the mammalian recipient, or plant cells. Such donor cells can be made using techniques similar to those described in, e.g., Emerich *et al*, J. Neurosci., 16: 5168-81 (1996). Even more preferred, the donor cells may be harvested from the specific mammal to be treated and made into donor cells by ex vivo manipulation, akin to adoptive transfer techniques, such as those described in D. B. Kohn *et al*, Nature Med. 4(7):775-80 (1998). Donor cells may also be from non-mammalian species, if desired.

Finally, the composition of this invention can also include one or more of the selected agents which are described above. The composition can contain a mixture of synthetic RNA molecules described above, synthetic DNA delivery molecules described above, and any of the other delivery agents described above, such as recombinant bacteria, cells, and viruses. The identity of the composition mixture may be readily selected by one of skill in the art.

### 3.2 Production of the polycistronic RNA of the invention by enzymatic synthesis

The polycistronic RNA molecule according to this invention (or the dsRNA molecule resulting therefrom after removal of the removable RNA element) may be delivered to the target cell or organism (e.g., a pathogen (such as a nematode) or a mammalian) as a molecule, which was made in vitro by enzymatic synthesis.

Thus, another embodiment of the invention relates to a method for generating a polycistronic RNA of the invention comprising:

(i) providing an in vitro transcription system including an expression construct for the polycistronic RNA of the invention, and

(ii) isolating said polycistronic RNA of the invention.

Thus, another embodiment of the invention relates to a method for generating short interfering double-stranded RNA (siRNA) comprising:

(i) providing an in vitro transcription system including an expression construct for a polycistronic RNA of the invention, and
(ii) isolating said short interfering double-stranded RNA (siRNA) from said in vitro transcription system.

Preferably, the employed transcription system is a eukaryotic system allowing for processing of the polycistronic RNA molecule of the invention (i.e. excision of the removable RNA element). More preferably, the transcription system is a eukaryotic cell in which said expression construct is introduced to mediate transgenic expression of the polycistronic RNA molecule.
Prokaryotic and - preferably - eukaryotic systems can be employed. Furthermore, systems based on isolated enzymes and systems based on cellular extracts can be utilized. Eukaryotic, prokaryotic or bacteriophage RNA polymerases (such as, for example, T3, T7 or SP6 RNA polymerase) can be used for this purpose. Suitable methods for the *in-vitro* expression of RNA are described (WO 97/32016; US 5,593,874; US 5,698,425, US 5,712,135, US 5,789,214, US 5,804,693). Enzymatic systems based on isolated enzymes can be used, for example, the bacteriophage T7, T3 or SP6 RNA polymerases according to the conventional methods described by such texts as the Promega Protocols and Applications Guide, (3rd ed. 1996), eds. Doyle, ISBN No. 1-882274-57-1.
Accordingly, the invention also provides a kit that includes reagents for attenuating the expression of a target gene in a cell. The kit contains a DNA template comprising a promoter (preferably a T7 promoter, a T3 promoter or an SP6 promoter) operably linked to a nucleotide sequence encoding a polycistronic RNA of the invention. The kit optionally contains amplification primers for amplifying the DNA sequence from the DNA template and nucleotide triphosphates (i.e., ATP, GTP, CTP and UTP) for forming RNA. Also optionally, the kit contains a RNA polymerase, capable of binding to the promoter on the DNA template and causing transcription of the nucleotide sequence to which the promoter is operably linked; a purification column for purifying single stranded RNA, such as a size exclusion column; one or more buffers, for example a buffer for annealing single stranded RNAs to yield double stranded RNA; and RNAse A or RNAse T for purifying double stranded RNA.
In cases where an eukaryotic transcription system is employed (such as lysates from rabbit reticulocytes or wheat germ; see Movahedzadeh *et al*., "In vitro transcription and translation," in Methods in Molecular Biology, V. 235, N. Casali, A. Preston, Eds., Totowa, NJ: Humana Press, p. 247-55; Lamla *et al*., Acta Biochim Pol, 48:453-65, 2001) correct removal of the removable RNA element is expected resulting in release of the dsRNA or siRNA, which may be purified from the system.
Prior to introduction into a cell, tissue or organism, a polycistronic RNA (or a dsRNA derived therefrom) which has been synthesized *in vitro,* either chemically or enzymatically, can be purified either completely or in part from the reaction mixture, for example by extraction, precipitation, electrophoresis, chromatography or combinations of these methods.

### 3.3 Production of the polycistronic RNA of the invention by chemical synthesis

The polycistronic RNA molecules of the invention can also be synthesized - entirely or in part - by chemical synthesis. Chemical synthesis of linear oligonucleotides is well known in the art and can be achieved by solution or solid phase techniques. Preferably, synthesis is by solid phase methods. Suitable synthetic procedures include but are not limited to phosphoramidite, phosphite triester, H-phosphonate, and phosphotriester methods, typically by automated synthesis methods. Such oligonucleotide synthesis protocols can be found, e.g., in US 5,830,653; WO 98/13526; Stec *et al*. 1984. J. Am. Chem. Soc. 106:6077; Stec *et al*. 1985. J. Org. Chem. 50:3908; Stec et *al*. J. Chromatog. 1985. 326: 263; LaPlanche *et al*. 1986. Nuc. Acid. Res. 1986. 14:9081; Fasman G. D., 1989. Practical Handbook of Biochemistry and Molecular Biology. 1989. CRC Press, Boca Raton, Fla.; Lamone. 1993. Biochem. Soc. Trans. 21:1; US 5,013,830; US 5,214,135; US 5,525,719; Kawasaki *et al*. 1993. J. Med. Chem. 36:831; WO 92/03568; US 5,276,019; US 5,264,423. Alternative methods for in vitro chemical synthetis of the RNA molecules of the invention are described [see, e.g., Xu *et al,* Nucl. Acids Res., 24(18):3643-4 (1996); Naryshkin *et al*, Bioorg. Khim., 22(9):691-8 (1996); Grasby *et al,*
Nucl. Acids Res., 21(19):4444-50 (1993); Chaix el al, Nucl. Acids Res., 17(18):7381-93 (1989); Chou *et al,* Biochem., 2(6):2422-35 (1989); Odai *et al,* Nucl. Acids Symp, Ser., 21:105-6 (1989); Naryshkin *et al,* Bioorg. Khim, 22 (9):691-8 (1996); Sun *et al,* RNA, 3(11):1352-1363 (1997); X. Zhang *et al,* Nucl. Acids Res., 25(20):3980-3 (1997); Grvaznov *et al,* Nucl. Acids Res., 26 (18):4160-7 (1998); Kadokura *et al,* Nucl. Acids

Symp Ser, 37:77-8 (1997); Davison *et al,* Biomed. Pept. Proteins, Nucl. Acids, 2(1):1-6 (1996); Mudrakovskaia *et al*, Bioorg. Khim., 17(6):819-22 (1991)].

The synthesis method selected can depend on the length of the desired oligonucleotide and such choice is within the skill of the ordinary artisan. For example, the phosphoramidite and phosphite triester method can produce oligonucleotides having 175 or more nucleotides while the H-phosphonate method works well for oligonucleotides of less than 100 nucleotides. If modified bases are incorporated into the oligonucleotide, and particularly if modified phosphodiester linkages are used, then the synthetic procedures are altered as needed according to known procedures. In this regard, Uhlmann *et al.* (1990, Chemical Reviews 90:543-584) provide references and outline procedures for making oligonucleotides with modified bases and modified phosphodiester linkages. Other exemplary methods for making oligonucleotides are taught in Sonveaux. 1994. "Protecting Groups in Oligonucleotide Synthesis"; Agrawal. Methods in Molecular Biology 26:1. Exemplary synthesis methods are also taught in "Oligonucleotide SynthesisA Practical Approach" (Gait, M. J. IRL Press at Oxford University Press. 1984). Moreover, linear oligonucleotides of defined sequence, including some sequences with modified nucleotides, are readily available from several commercial sources.

The polycistronic RNA molecule of the invention (or the dsRNA derived therefrom) may include modifications to either the phosphate-sugar backbone or the nucleoside. For example, the phosphodiester linkages of natural RNA may be modified to include at least one of a nitrogen or sulfur heteroatom. Modifications in RNA structure may be tailored to allow specific genetic inhibition while avoiding a general panic response in some organisms which is generated by dsRNA. Likewise, bases may be modified to block the activity of adenosine deaminase. In one embodiment of the invention the polycistronic RNA molecule has end-blocks on one or both ends.

The polycistronic RNA of the invention (or the dsRNA derived therefrom) may include "morpholino oligonucleotides." Morpholino oligonucleotides are non-ionic and function by an RNase H-independent mechanism. Each of the 4 genetic bases (Adenine, Cytosine, Guanine, and Thymine/Uracil) of the morpholino oligonucleotides is linked to a 6-membered morpholine ring. Morpholino oligonucleotides are made by joining the 4 different subunit types by, e.g., non-ionic phosphorodiamidate inter-subunit linkages. Morpholino oligonucleotides have many advantages including: complete resistance to nucleases (Antisense & Nuc. Acid Drug Dev. 1996. 6:267); predictable targeting (Biochemica Biophysica Acta. 1999. 1489:141); reliable activity in cells (Antisense & Nuc. Acid Drug Dev. 1997. 7:63); excellent sequence specificity (Antisense & Nuc. Acid Drug Dev. 1997. 7:151); minimal non-antisense activity (Biochemica Biophysica Acta. 1999. 1489:141); and simple osmotic or scrape delivery (Antisense & Nuc. Acid Drug Dev. 1997. 7:291). Morpholino oligonucleotides are also preferred because of their non-toxicity at high doses. A discussion of the preparation of morpholino oligonucleotides can be found in Antisense & Nuc. Acid Drug Dev. 1997. 7:187.

Another embodiment of the invention includes duplexes in which nucleomonomernucleomonomer mismatches are present in a sense 2'-O-methly strand (and are thought to be easier to unwind). As a further example, the use of 2'-O-methyl RNA may beneficially be used in circumstances in which it is desirable to minimize cellular stress responses. RNA having 2'-O-methyl nucleomonomers may not be recognized by cellular machinery that is thought to recognize unmodified RNA. The use of 2'-O-methylated or partially 2'-O-methylated RNA may avoid the interferon response to double-stranded nucleic acids, while maintaining target RNA inhibition. This RNA interference ("stealth RNAi") is useful for avoiding the interferon or other cellular stress responses, both in short RNAi (e.g., siRNA) sequences that induce the interferon response, and in longer RNAi sequences that may induce the interferon response. Other chemical modifications in addition to 2'-O-methylation may also achieve this effect.

In certain embodiments, the polycistronic RNA molecules of the invention (or the dsRNA molecule resulting therefrom after removal of the removable RNA element) comprise 3' and 5' termini (except for circular molecules): In one embodiment, the 3' and 5' termini can be substantially protected from nucleases e.g., by modifying the 3' or 5' linkages (e.g., US 5,849,902 and WO 98/13526). For example, oligonucleotides can be made resistant by the inclusion of a "blocking group." The term "blocking group" as used herein refers to substituents (e.g., other than OH groups) that can be attached to oligonucleotides or nucleomonomers, either as protecting groups or coupling groups for synthesis (e.g., FITC, propyl, phosphate, hydrogen phosphonate, or phosphoramidite). "Blocking groups" also include "end blocking groups" or "exonuclease blocking groups" which protect the 5' and 3' termini of the oligonucleotide, including modified nucleotides and non-nucleotide exonuclease resistant structures. Exemplary end-blocking groups include cap structures (e.g., a 7-methylguanosine cap), inverted nucleomonomers, e.g., with 3'-3' or 5'-5' end inversions (see, e.g., Ortiagao *et al.* 1992. Antisense Res. Dev. 2:129), methylphosphonate, phosphoramidite, non-nucleotide groups (e.g., non-nucleotide linkers, amino linkers, conjugates) and the like. The 3' terminal nucleomonomer can comprise a modified sugar moiety. The 3' terminal nucleomonomer comprises a 3'-O that can optionally be substituted by a blocking group that prevents 3'-exonuclease degradation of the oligonucleotide. For example, the 3'-hydroxyl can be esterified to a nucleotide through a 3'-3' internucleotide linkage. For example, the alkyloxy radical can be methoxy, ethoxy, or isopropoxy, and preferably, ethoxy. Optionally, the 3'-3' linked nucleotide at the 3' terminus can be linked by a substitute linkage. To reduce nuclease degradation, the 5' most 3'-5' linkage can be a modified linkage, e.g., a phosphorothioate or a P-alkyloxyphosphotriester linkage. Optionally, the 5' terminal hydroxy moiety can be esterified with a phosphorus containing moiety, e.g., phosphate, phosphorothioate, or P-ethoxyphosphate.

In another embodiment of the invention the polycistronic RNA molecule of the invention may comprise one or more flexible linker. Such linkers may be used to combine or fuse two or more smaller polycistronic RNAs together to a larger polycistronic RNA molecule. A linker is provided with functional groups at each end that can be suitably protected or activated. The functional groups are covalently attached to each RNA molecule, e.g., via an ether, ester, carbamate, phosphate ester or amine linkage to either the 5'-hydroxyl or the 3'-hydroxyl. Preferred linkages are phosphate ester linkages similar to typical oligonucleotide linkages. For example, hexaethyleneglycol can be protected on one terminus with a photolabile protecting group (i.e., NVOC or MeNPOC) and activated on the other terminus with 2-cyanoethyl-N, N-diisopropylaminochlorophosphite to form a phosphoramidite. Other methods of forming ether, carbamate or amine linkages are known to those of skill in the art and particular reagents and references can be found in such texts as March, Advanced Organic Chemistry, 4th Ed., Wiley-Interscience, New York, N.Y., 1992. In general, the flexible linkers are non-nucleotide molecules including spacers, attachments, bioconjugates, chromophores, reporter groups, dye labeled RNAs, and non-naturally occurring nucleotide analogues. Preferred linkers, spacers, bioconjugates, attachments, and chromophores are more specifically described in US Patent Application No. 20040058886, herein incorporated by reference.

In one embodiment, the first ribonucleic acid sequence (i.e., the sense strand) of the polycistronic RNA molecule of the invention (or the double-stranded RNA molecule resulting therefrom after removal of the removable RNA element) comprises a 5' group that allows for RNAi activity but which renders said first ribonucleic acid sequence inactive in terms of gene targeting. Preferably, such a 5' modifying group is a phosphate group or a group larger than a phosphate group In another embodiment, the antisense strand of an oligonucleotide comprises a 5' phosphate group.

In one embodiment, a polycistronic RNA molecule of the invention (or the dsRNA molecule resulting therefrom after removal of the removable RNA element) can include an agent which increases the affinity for its target sequence. The term "affinity enhancing agent" includes agents that increase the affinity of an polycistronic RNA molecule of the invention (or the dsRNA molecule resulting therefrom after removal of the removable RNA element) for its target. Such agents include, e.g., intercalating agents and high affinity nucleomonomers. Intercalating agents interact strongly and nonspecifically with nucleic acids. Intercalating agents serve to stabilize RNA-DNA duplexes and thus increase the affinity of the polycistronic RNA molecule of the invention (or the dsRNA molecule resulting therefrom after removal of the removable RNA element) for their targets. Intercalating agents are most commonly linked to the 3' or 5' end of oligonucleotides. Examples of intercalating agents include acridine, chlorambucil, benzopyridoquinoxaline, benzopyridoindole, benzophenanthridine, and phenazinium. The agents may also impart other characteristics to the oligonucleotide, for example, increasing resistance to endonucleases and exonucleases.

In one embodiment, a high affinity nucleomonomer is incorporated into an polycistronic RNA molecule of the invention (or the double-stranded RNA molecule resulting therefrom after removal of the removable RNA element). The language "high affinity nucleomonomer" as used herein includes modified bases or base analogs that bind to a complementary base in a target nucleic acid molecule with higher affinity than an unmodified base, for example, by having more energetically favorable interactions with the complementary base, e.g., by forming more hydrogen bonds with the complementary base. For example, high affinity nucleomonomer analogs such as aminoethyoxy phenoxazine (also referred to as a G clamp), which forms four hydrogen bonds with guanine are included in the term "high affinity nucleomonomer." A high affinity nucleomonomer is illustrated below (see, e.g., Flanagan, *et al*., 1999. Proc. Natl. Acad. Sci. 96:3513). Other exemplary high affinity nucleomonomers are known in the art and include 7-alkenyl, 7-alkynyl, 7-heteroaromatic-, or 7-alkynyl-heteroaromatic-substituted bases or the like which can be substituted for adenosine or guanosine in oligonucleotides (see, e.g., US 5,594,121). Also, 7-substituted deazapurines have been found to impart enhanced binding properties to oligonucleotides, i.e., by allowing them to bind with higher affinity to complementary target nucleic acid molecules as compared to unmodified oligonucleotides. High affinity nucleomonomers can be incorporated into the oligonucleotides of the instant invention using standard techniques.

In another embodiment, an agent that increases the affinity of a polycistronic RNA molecule of the invention (or the dsRNA molecule resulting therefrom after removal of the removable RNA element) for its target comprises an intercalating agent. As used herein, the language "intercalating agent" includes agents which can bind to a DNA double helix. When covalently attached to a polycistronic RNA molecule of the invention (or the dsRNA molecule resulting therefrom after removal of the removable RNA element), an intercalating agent enhances the binding of the oligonucleotide to its complementary genomic DNA target sequence. The intercalating agent may also increase resistance to endonudeases and exonucleases. Exemplary intercalating agents are taught by Helene and Thuong (1989. Genome 31:413), and include e.g., acridine derivatives (Lacoste *et al.* 1997. Nucleic Acids Research. 25:1991; Kukreti *et al.* 1997. Nucleic Acids Research. 25:4264); quinoline derivatives (Wilson *et al.* 1993. Biochemistry 32:10614); benzo[f]quino[3,4-b]quioxaline derivatives (Marchand *et al.* 1996. Biochemistry. 35:5022; Escude *et al.* 1998. Proc. Natl. Acad. Sci. 95: 3591). Intercalating agents can be incorporated into a polycistronic RNA molecule of the invention (or the double-stranded RNA molecule resulting therefrom after removal of the removable RNA element) using any convenient linkage. For example, acridine or psoralen can be linked to the oligonucleotide through any available -OH or -SH group, e.g., at the terminal 5' position of the oligonucleotide, the 2' positions of sugar moieties, or an OH, NH2, COOH, or SH incorporated into the 5-position of pyrimidines using standard methods.

In one embodiment, the double-stranded duplex constructs of the invention can be further stabilized against nucleases by forming loop structures at the 5' or 3' end of the sense or antisense strand of the construct. Suitable loop-structure and other structures to stabilize an RNA molecule of the invention are for example described in US patent Application No. 20040014956.

The polycistronic RNA molecule of the invention (or an expression construct or vector for its production, or an dsRNA molecule derived by RNA processing from said polycistronic RNA molecule) can be derivatized, chemically modified, combined with and/or linked to various agents to enhance its activity or specificity. Such agents include but are not limited to conjugation agents (e.g., for improvement of cellular uptake), protein carriers (e.g., for improvement of cellular uptake and greater cellular accumulation), encapsulating agents (such as liposomes; e.g., to facilitate the cellular uptake or targeting), complexing agents (such as cationic lipid, e.g., to increase cellular uptake), basic oligopeptides, transporting peptides (e.g., HIV TAT transcription factor, lactoferrin, Herpes VP22 protein), and targeting agents (for targeting to a cellular receptor). Suitable conjugation agents, protein carriers, encapsulating agents, complexing agents, basic oligopeptides, transporting peptides, and targeting agents are described for example in US Patent Application No. 20040014956. Additional ways to contact a polycistronic RNA molecule of the invention (or the dsRNA molecule resulting therefrom after removal of the removable RNA element) with its target cell are described below in the context of pharmaceutical application. Alternatively, the target gene for suppression or reducing can be in a non-transgenic organism which acquires the dsRNA or DNA producing dsRNA by ingestion or infection by a transgenic organism. See e.g., US 6,506,559. Methods for increase stability of the RNA molecules of the invention against nuclease degradation (e.g., by serum nucleases and cellular nucleases and nucleases found in other bodily fluids) are described in United States Patent Application No. 20040014956.

If synthesized chemically or by in vitro enzymatic synthesis, the polycistronic RNA molecule of the invention (or the dsRNA molecule resulting therefrom after removal of the removable RNA element) may be purified prior to introduction into the cell. For example, RNA can be purified from a mixture by extraction with a solvent or resin, precipitation, electrophoresis (e.g., polyacrylamide gel electrophoresis), chromatography (e.g., gel chromatography and high pressure liquid chromatography) or a combination thereof. Alternatively, the dsRNA construct may be used with no or a minimum of purification to avoid losses due to sample processing. The dsRNA construct may be dried for storage or dissolved in an aqueous solution. The solution may contain buffers or salts to promote annealing, and/or stabilization of the duplex strands.The quality of the synthesized polycistronic RNA molecules of the invention synthesized can be verified by capillary electrophoresis and denaturing strong anion HPLC (SAX-HPLC) using, e.g., the method of Bergot and Egan. 1992. J. Chrom. 599:35.

### 4. Introduction of the polycistronic RNA into cells and organism

The polycistronic RNA of the invention or the dsRNA derived therefrom or its precursor or delivery agents (e.g., expression cassettes or vectors) (hereinafter together the "dsRNA agent") can be introduced into an organism or a cell in various ways with which the skilled worker is familiar. "To introduce" is to be understood in the broad sense and comprises, for the purposes of the present invention, all those methods which are suitable for directly or indirectly introducing, into an organism or a cell, compartment, tissue, organ or seed of same, a dsRNA agent of the invention, or generating it/them therein. The introduction can bring about the transient presence of a dsRNA agent, or else a stable presence.

The dsRNA agent (e.g., the polycistronic RNA molecule of the invention) is typically is introduced or administered in an amount that allows delivery of at least one copy per cell. Higher amounts (for example at least 5, 10, 100, 500 or 1000 copies per cell) can, if appropriate, effect a more efficient reduction of the expression of the target genes.

The amount of dsRNA agent administered to a cell, tissue, or organism depends on the nature of the cell, tissue, or organism, the nature of the target gene, and the nature of the dsRNA agent, and can readily be optimized to obtain the desired level of gene inhibition. To attenuate gene expression in a single cell embryo, for example, at least about $0.8X10^6$ molecules of dsRNA agent are injected; more preferably, at least about $20X10^6$ molecules of dsRNA agent are injected; most preferably, at least about $50X10^6$ molecules of dsRNA are injected. The amount of dsRNA agent injected into a single cell embryo is, however, preferably at most about $1000X10^6$ molecules; more preferably, it is at most about $500X10^6$ molecules, most preferably, at most about $100X10^6$ molecules. In the case of administration of dsRNA agent to a cell culture or to cells in tissue, by methods other than injection, for example by soaking, electroporation, or lipid-mediated transfection, the cells are preferably exposed to similar levels of dsRNA agent in the medium. Since dsRNA molecules are described to be mobile within an organism (i.e. to migrate from cell to cell), it is not necessarily required to apply the dsRNA agent into each cell of the organism. It suffices to introduce, or to express, the dsRNA in few cells.

For examples the dsRNA agent may be introduced into cells via transformation, transfection, injection, projection, conjugation, endocytosis, and phagocytosis. Preferred method for introduction comprise but are not limited to:

a) methods of the direct or physical introduction of the polycistronic RNA molecule of the invention (or the dsRNA derived therefrom) into the target cell or organism, and

b) methods of the indirect introduction of polycistronic RNA of the invention (or the dsRNA derived therefrom) into the target cell or organism (e.g., by a first introduction of an expression cassette and a subsequent intracellular expression).

### 4.1 Direct and physical introduction of RNA into target cells or organism

In case the polycistronic RNA of the invention (or a dsRNA agent) is produced outside the target cell or organism, it can be contacted with (i.e., brought into contact with, also referred to herein as administered or delivered to) and taken up by one or more cell or the target organism (preferably human, pathogen or plant cells or organisms). The contact may be in vitro, e.g., in a test tube or culture dish, (and may or may not be introduced into a subject) or in vivo, e.g., in a subject such as a mammalian, pathogen or plant subject. The pathogen is preferably a nematode.

The polycistronic RNA of the invention (or a dsRNA agent) may be directly introduced into the cell (i.e., intracellularly); or introduced extracellularly into a cavity, interstitial space, into the circulation of an organism, introduced orally, or may be introduced by bathing an organism in a solution containing the polycistronic RNA of the invention (or a dsRNA agent). Methods for oral introduction include direct mixing of RNA with food of the organism, as well as engineered approaches in which a species that is used as food is engineered to express a polycistronic RNA of the invention (or a dsRNA agent), then fed to the organism to be affected.

Physical methods of introducing nucleic acids include injection of a solution of the polycistronic RNA of the invention (or a dsRNA agent) directly into the cell or extracellular injection into the organism. For example, in the case of an embryo or a cell, the polycistronic RNA of the invention (or a dsRNA agent) is conveniently administered by microinjection; other methods of introducing nucleic acids into a cell include bombardment by particles covered by the polycistronic RNA of the invention (or a dsRNA agent), soaking the cell or organism in a solution of the polycistronic RNA of the invention (or a dsRNA agent), electroporation of cell membranes in the presence of the polycistronic RNA of the invention (or a dsRNA agent), liposome-mediated delivery of polycistronic RNA of the invention (or a dsRNA agent) and transfection mediated by chemicals such as calcium phosphate.

The polycistronic RNA of the invention (or a dsRNA agent) agent may be introduced along with components that enhance RNA uptake by the cell, stabilize the annealed strands, or otherwise increase inhibition of the target gene. Delivery into cells can be enhanced by suitable art recognized methods including calcium phosphate, DMSO, glycerol or dextran, electroporation, or by transfection, e.g., using cationic, anionic, or neutral lipid compositions or liposomes using methods known in the art (see e.g., WO 90/14074; WO 91/16024; WO 91/17424; US 4,897,355; Bergan *et al*. 1993. Nucleic

Acids Research. 21:3567). Also polyamine or polycation conjugates using compounds such as polylysine, protamine, or N1, N12-bis (ethyl) spermine (see, e.g., Bartzatt, R. *et al*. 1989. Biotechnol. Appl. Biochem. 11:133; Wagner E. *et al*. 1992. Proc. Natl. Acad. Sci. 88:4255) can be employed. In the case of a cell culture or tissue explant, the cells are conveniently incubated in a solution containing the polycistronic RNA of the invention (or a dsRNA agent) or lipid-mediated transfection; in the case of a whole animal or plant, the polycistronic RNA of the invention (or a dsRNA agent) is conveniently introduced by injection or perfusion into a cavity or interstitial space of an organism, or systemically via oral, topical, parenteral (including subcutaneous, intramuscular and intravenous administration), vaginal, rectal, intranasal, ophthalmic, or intraperitoneal administration.

In addition, the polycistronic RNA of the invention (or a dsRNA agent) can be administered via an implantable extended release device. Methods for oral introduction include direct mixing of RNA with food of the organism, as well as engineered approaches in which a species that is used as food is engineered to express an RNA, then fed to the organism to be affected. The polycistronic RNA of the invention (or a dsRNA agent) may be sprayed onto a plant or a plant may be genetically engineered to express the RNA in an amount sufficient to kill some or all of a pathogen known to infect the plant.

### 4.2 Indirect introduction of RNA

Alternatively, the dsRNA agent can be supplied to a cell indirectly by introducing (e.g., by transformation or transfection) one or more expression constructs or expression vectors that encode the polycistronic RNA molecule of the invention. The expression of the polycistronic RNA of the invention (or a dsRNA molecule derived therefrom after removal of the removable RNA element) can be transient or - for example after integration into the genome (for example using selection markers) of the organism - stable. Preferably, the polycistronic RNA expression system is integrated stably into the genome - for example the chromosomal DNA or the DNA of the organelles (for example the plastids (e.g., chloroplasts), mitochondria and the like) - of a cell. Integration into the chromosomal DNA is preferred.

Expression constructs and vectors are generally described above (see DEFINITION section and section 3.1). Preferred expression constructs are described in more detailed below for the specific applications the composition and methods of the present invention. Methods for supplying a cell with dsRNA by introducing an expression construct or vector from which it can be transcribed are set forth in WO 99/32619. Principally also all the methods for direct introduction of RNA molecules into cells as described above can be employed for introduction of the nucleic acid molecules resembling the expression cassette or vector.

**5. Use of the polycistronic RNA in gene silencing**

The polycistronic RNA molecules of the invention, the expression cassettes and the expression vectors for their expression, and the transgenic organism comprising said molecules can be utilized in gene silencing (i.e. to attenuate, reduce or suppress expression of target genes in target cells or organism).

Another embodiment of the invention relates to composition for altering, preferably reducing or attenuating, expression of a target gene, comprising at least one polycistronic RNA of the invention.

Yet another embodiment of the invention relates to a method for attenuating (or reducing or suppressing) expression of at least one target gene in an eukaryotic cell, comprising introducing a polycistronic RNA molecule of the invention (or an expression construct or vector encoding the same) into the cells in an amount sufficient to attenuate expression of the target gene, wherein the polycistronic RNA molecule comprises at least one ribonucleotide sequence that is substantially identical to at least a part of the nucleotide sequence of the target gene.

Any gene being expressed in a cell (preferably an eucaryotic cell) can be targeted. A gene that is expressed in the cell is one that is transcribed to yield an RNA (e.g., a mRNA) and, optionally, a protein. Preferably the target gene is an endogenous gene of the cell or a heterologous gene relative to the genome of the cell, such as a pathogen gene. More preferably, the first ribonucleotide sequence is substantially identical to a target gene is selected from the group consisting of eukaryotic endogenous genes, genes of pathogens and transgenes. Even more preferably, said gene of a pathogen is from a pathogen capable to infect an eukaryotic organism. Most preferably, said pathogen is selected from the group of virus, bacteria, fungi and nematodes. Preferably the target gene is a eukaryotic gene, more preferably a mammalian, nematode, fungal or plant gene.

The polycistronic RNA may be produced outside the cell (i.e. the host cell in which gene silencing should be achieved), or may be recombinantly produced by an expression construct or expression vector within the cell. The host cell is preferably eukaryotic cell, more preferably a nematode, mammalian cell or a plant cell.

Preferably, the target gene expression is attenuated (or reduced or suppressed) by at least about 10%, preferably at least about 30%, more preferably at least about 50%, even more preferably at least about 70%, most preferably at least about 90%.

To achieve gene silencing the polycistronic RNA of the invention comprises at least one first ribonucleotide sequence that is substantially identical (as defined above), preferably identical, to at least a part of at least one target gene. Preferably, said part of a target gene having substantial identity to said first ribonucleotide sequence has a length of least 15 nucleotides, preferably at least 19 nucleotides, more preferably at least 50 nucleotides, even more preferably at least 100 nucleotides, most preferably at least 250 nucleotides.

More preferably, said first nucleotide sequence has an identity of at least 65%, preferably at least 80%, more preferably at least 90%, most preferably 95%, even more preferably 100% to a sequence of at least 15 nucleotides, preferably at least 19 nucleotides, more preferably at least 50 nucleotides, even more preferably 100 nucleotides, most preferably at least 250 nucleotides of at least one target gene. Preferably, said first ribonucleotide sequence hybridizes (preferably under stringent conditions, more preferably under low stringency conditions, most preferably under high stringency conditions) to a sequence of the target gene.

In another embodiment the first ribonucleic acid includes a fragment of the nucleic acid molecule of a target gene. Said fragments may comprise smaller oligonucleotides having from about 15 to about 400 contiguous nucleotide residues and more preferably, about 15 to about 45 contiguous nucleotide residues, about 20 to about 45 contiguous nucleotide residues, about 15 to about 30 contiguous nucleotide residues, about 21 to about 30 contiguous nucleotide residues, about 21 to about 25 contiguous nucleotide residues, about 21 to about 24 contiguous nucleotide residues, about 19 to about 25 contiguous nucleotide residues, or about 21 contiguous nucleotides. In a preferred embodiment, a fragment shows 100% identity to the target gene.

In an preferred embodiment the first nucleotide sequence is substantially identical, preferably identical, to a part of the coding sequence or the non-coding sequence of the target gene (preferably an eukaryotic gene, such as a mammalian or plant gene). The non-coding sequence can be the 5'- or 3'-untranslated sequence or the introns but can also be a non-transcribed sequence. Non-coding sequences as target sequence are preferred in cases where the target gene encodes a member of a gene family (i.e. different genes encoding very similar proteins). A particularly preferred use of the present invention is where two or more genes within the gene family exhibit similar nucleic acid sequences within a coding region for the target protein but exhibit dissimilar nucleic acid sequences within a transcribed 5'-untranslated, 3'-untranslated or intron region.

In one preferred embodiment of the invention, the polycistronic RNA molecule of the invention generates after removal of said third ribonucleotide sequence a short interfering double-stranded RNA (siRNA). In one preferred embodiment of the method of the invention, the polycistronic RNA is processed to an short-interfering double-stranded RNA (siRNA) molecule by said host cells. Preferably, said siRNA does not produce a significant PKR-dependent response in the host cells at concentrations effective for attenuating expression of the target gene. Methods, how to suppress the PKR-dependent response are described above. Preferably, said generated short-interfering double-stranded RNA is about 15 to about 45 nucleotides in length, preferably 19 to about 50 nucleotides.

The target gene can be an endogenous gene or an exogenous or foreign gene (i.e., a transgene or a pathogen gene). For example, a transgene that is present in the genome of a cell as a result of genomic integration of the viral delivery construct can be regulated using polycistronic RNA according to the invention. The foreign gene can be integrated into the host genome (preferably the chromosomal DNA), or it may be present on an extrachromosomal genetic construct such as a plasmid or a cosmid. For example, the target gene may be present in the genome of the cell into which the polycistronic RNA is introduced, or in the genome of a pathogen, such as a virus, a bacterium, a fungus or a protozoan, which is capable of infecting such organism or cell.

The eukaryotic cell or organism to which the polycistronic RNA of the invention (or the dsRNA derived therefrom) can be delivered can be derived from any eukaryotic organism, such as for example without limitation, plants or animals, such as mammals, insects, nematodes, fungi, algae, fish, and birds. Likewise, the polycistronic RNA molecule of the invention (or the dsRNA molecule resulting therefrom after removal of the removable RNA element) or the expression constructs or vectors for their expression can be used to suppress or reduce any target gene in any eukaryotic organism. In some embodiments of the invention also prokaryotic organism comprising the polycistronic RNA of the invention are useful. For example prokaryotic cells and organism can be used to produce or amplify the polycistronic RNA of the invention or an expression construct or vector encoding the same. Furthermore, prokaryotic organism can be utilized as vehicles to introduce the polycistronic RNA of the invention into animals e.g. by feeding. Also, prokaryotic organisms, for example *Agrobacteria*, can advantageously be employed as vehicles for the transformation of, for example, plant organisms.

Thus a further aspect of the invention relates to cells and organism (e.g., plant, animal, protozoan, virus, bacterium, or fungus), which comprise at least one polycistronic RNA of the invention, or an expression construct or expression vectors encoding said polycistronic RNA molecule. In certain embodiments, the cell is suspended in culture; while in other embodiments the cell is in (or part of) a whole organism (e.g., a microorganism, plant or an animal, such as a non-human mammal). The cell can be prokaryotic or of eukaryotic nature. Preferably, the expression construct is comprised with the genomic DNA, more preferably within the chromosomal or plastidic DNA, most preferably in the chromosomal DNA of the cell.

The cell having the target gene may be from the germ line or somatic, totipotent or pluripotent, dividing or non-dividing, parenchyma or epithelium, immortalized or transformed, or the like. The cell can be a gamete or an embryo; if an embryo, it can be a single cell embryo or a constituent cell or cells from a multicellular embryo. The term "embryo" thus also includes fetal tissue. The cell having the target gene may be an undifferentiated cell, such as a stem cell, or a differentiated cell, such as from a cell of an organ or tissue, including fetal tissue, or any other cell present in an organism. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands.

Preferred prokaryotes are mainly bacteria such as bacteria of the genus *Escherichia, Corynebacterium, Bacillus, Clostridium, Proionibacterium, Butyrivibrio, Eubacterium, Lactobacillus, Erwinia, Agrobacterium, Flavobacterium, Alcaligenes, Phaeodactylum, Colpidium, Mortierella, Entomophthora, Mucor, Crypthecodinium* or *Cyanobacteria,* for example of the genus *Synechocystis.* Microorganisms which are preferred are mainly those which are capable of infecting plants and thus of transferring the constructs according to the invention. Preferred microorganisms are those of the genus *Agrobacterium* and in particular the species *Agrobacterium tumefaciens* and *rhizogenes*.

Eukaryotic cells and organisms comprises plant and animal (preferably nonhuman) organisms and/or cells and eukaryotic microorganisms such as, for example, yeasts, algae or fungi. A corresponding transgenic organism can be generated for example by introducing the expression systems in question into a zygote, stem cell, protoplast or another suitable cell which is derived from the organism. A transgenic animal that expresses a polycistronic RNA of the invention from a recombinant expression construct may be produced by introducing the construct into a zygote, an embryonic stem cell, or another multipotent cell derived from the appropriate organism. A viral construct packaged into a viral particle would accomplish both efficient introduction of an expression construct into the cell and transcription of RNA encoded by the expression construct. Suitable vector are will known in the art (see e.g., Shi, Y. 2003. Trends Genet 2003 Jan 19:9; Reichhart J M *et al.* Genesis. 2002. 34(1-2):160-4, Yu *et al.* 2002. Proc Natl Acad Sci U S A 99:6047; Sui *et al.* 2002. Proc Natl Acad Sci U S A 99:5515).

The plant may be a monocot, dicot or gymnosperm; the animal may be a vertebrate or invertebrate. Preferred animal and plant organisms are specified above in the DEFINITION section. Preferred fungi are Aspergillus, Trichoderma, Ashbya, Neurospora,

Fusarium, Beauveria or further fungi described in Indian Chem Engr. Section B. Vol 37, No 1,2 (1995), page 15, Table 6. Especially preferred is the filamentous Hemiascomycete Ashbya gossypii. Preferred yeasts are Candida, Saccharomyces, Hansenula or Pichia, especially preferred are Saccharomyces cerevisiae or Pichia pastoris (ATCC Accession No. 201178). Especially preferred animal organism are nematodes.

Preferred as organisms are plant organisms. Preferred plants are selected in particular from among crop plants. Most preferred are

a) Plants which are suitable for oil production such as, for example, oilseed rape, sunflower, sesame, safflower (Carthamus tinctorius), olive tree, soybean, maize, peanut, castor-oil plant, oil palm, wheat, cacao shrub, or various nut species such as, for example, walnut, coconut or almond. Especially preferred among these, in turn, are dicotyledonous plants, in particular oilseed rape, soybean and sunflower.
b) Plants, which serve for the production of starch, such as, for example, maize, wheat or potato.
c) Plants, which are used as foodstuffs and/or feeding stuffs and/or useful plant and in which a resistance to pathogens would be advantageous such as, for example, barley, rye, rice, potato, cotton, flax, or linseed.
d) Plants, which can serve for the production of fine chemicals such as, for example, vitamins and/or carotenoids such as, for example, oilseed rape.

Plant varieties may be excluded, particularly registrable plant varieties according to Plant Breeders Rights. It is noted that a plant need not be considered a "plant variety" simply because it contains stably within its genome a transgene, introduced into a cell of the plant or an ancestor thereof. In addition to a plant, the present invention provides any clone of such a plant, seed, selfed or hybrid progeny and descendants, and any part or propagule of any of these, such as cuttings and seed, which may be used in reproduction or propagation, sexual or asexual. Also encompassed by the invention is a plant which is a sexually or asexually propagated off-spring, clone or descendant of such a plant, or any part or propagule of said plant, off- spring, clone or descendant. Genetically modified plants according to the invention, which can be consumed by humans or animals, can also be used as food or feedstuffs, for example directly or following processing known in the art. The present invention also provides for parts of the organism especially plants, particularly reproductive or storage parts. Plant parts, without limitation, include seed, endosperm, ovule, pollen, roots, tubers, stems, leaves, stalks, fruit, berries, nuts, bark, pods, seeds and flowers. In a particularly preferred embodiment of the present invention, the plant part is a seed.

Another embodiment of the present invention relates to a method for attenuating (or reducing) expression of at least one target gene in an eukaryotic cell, comprising introducing a polycistronic RNA molecule of the invention into the cell in an amount sufficient to attenuate expression of the target gene, wherein the polycistronic RNA molecule comprises at least one ribonucleotide sequence that is substantially identical, preferably identical, to at least part of the nucleotide sequence of the target gene. Depending on the particular target gene and the dose of polycistronic RNA delivered, the method may partially or completely inhibit expression of the gene in the cell. Preferably the polycistronic RNA of the invention (or the dsRNA derived therefrom after removal of the removable RNA element) is capable of effectively eliminating, substantially reducing, or at least partially reducing the level of a RNA (preferably mRNA) transcript or protein encoded by the target gene (or gene family). Preferably, the expression of the target gene (as measured by the expressed RNA or protein) is reduced, inhibited or attenuated by at least 10%, preferably at least 30% or 40%, preferably at least 50% or 60%, more preferably at least 80%, most preferably at least 90% or 95%. The levels of target products such as transcripts or proteins may be decreased throughout an organism such as a plant or mammal, or such decrease in target products may be localized in one or more specific organs or tissues of the organism. For example, the levels of products may be decreased in one or more of the tissues and organs of a plant including without limitation: roots, tubers, stems, leaves, stalks, fruit, berries, nuts, bark, pods, seeds and flowers. A preferred organ is a seed of a plant.

The expression of two or more genes can be attenuated concurrently by introducing two or more polycistronic RNAs or one polycistronic NA capable to provide more than one dsRNA molecules into the cell in amounts sufficient to attenuate expression of their respective target genes. In a preferred embodiment the reduction is specific i.e. the polycistronic RNA (or any dsRNA molecule derived therefrom by removal of the removable RNA element) leaves the level of a second gene essentially unaffected, substantially unaffected, or partially unaffected (as defined above).

DsRNAs in mammalian cells typically activate protein kinase PKR and lead to apoptosis. The mechanism of action of PKR includes phosphorylation and inactivation of eIF2$\alpha$ (Fire (1999) Trends Genet 15: 358). This sequence-independent response may reflect a form of primitive immune response, since the presence of dsRNA is a common feature of many viral lifecycles. The PKR response can be overcome in favor of the sequence-specific RNAi response. Preferably, the polycistronic RNA molecules of the invention (or the dsRNA or siRNA molecules derived therefrom) do not activate the interferon pathway, e.g., as evidenced by the lack of induction of the dsRNA, interferon-inducible protein kinase, PKR. In one embodiment, modifications are made to a polycistronic. RNA molecule, which would normally activate the interferon pathway such that the interferon pathway is not activated. For example, the interferon pathway is activated by double-stranded unmodified RNA comprising 5'-triphosphate residues (Kim et al 2004 Nature Biotechnology 22 (3) 321-325). The cellular recognition of dsRNA is highly specific and modifying one or both of the strands of a double-stranded duplex enable the dsRNA molecule to evade the dsRNA recognition machinery of the cell but would still allow for the activation of the RNAi pathway. The ability of a double-stranded oligonucleotide to activate interferon could be assessed by testing for expression of the dsRNA, Interferon-Inducible Protein Kinase, PKR using techniques known in the art and also testing for the ability of the double-stranded molecule to effect target gene inhibition. Accordingly, in one embodiment, the invention provides a method of testing for the ability of a polycistronic RNA of the invention to induce interferon by testing for the ability of the oligonucleotide to activate PKR. Compositions that do not activate PKR (i.e., do not activate the interferon pathway) are then selected for use to inhibit gene transcription in cells, e.g., in therapeutics or functional genomics.

In certain embodiments, the cells can be treated with an agent(s) that inhibits the general dsRNA response(s) by the host cells, such as may give rise to sequence-independent apoptosis. For instance, the cells can be treated with agents that inhibit the dsRNA-dependent protein kinase known as PKR (protein kinase RNA-activated). In certain instances, it may be desirable to treat the cells with agents which inhibit expression of PKR, cause its destruction, and/or inhibit the kinase activity of PKR, and such methods are specifically contemplated for use in the present invention. Likewise, over-expression of agents, which ectopically activate eIF2α, can be used. Other agents, which can be used to suppress the PKR response, include inhibitors of IκB phosphorylation of IκB, inhibitors of IκB ubiquitination, inhibitors of IκB degradation, inhibitors of NFκB nuclear translocation, and inhibitors of NF-κB interaction with κB response elements. Other inhibitors of sequence-independent dsRNA response in cells include the gene product of the vaccinia virus E3L. The E3L gene product contains two distinct domains. A conserved carboxy-terminal domain has been shown to bind dsRNA and inhibit the antiviral dsRNA response by cells. Expression of at least that portion of the E3L gene in the host cell, or the use of polypeptide or peptidomimetics thereof, can be used to suppress the general dsRNA response. Caspase inhibitors sensitize cells to killing by dsRNA. Accordingly, ectopic expression or activation of caspases in the host cell can be used to suppress the general dsRNA response.

The subsequent application of compositions and methods according to the invention may be mentioned by way of example, but not by limitation:

### 5.1 Applications in plant biotechnology

The method according to the invention is preferably employed for the purposes of plant biotechnology for generating plants with advantageous properties. Thus, the suitability of the plants or their seeds as foodstuff or feeding stuff can be improved, for example via a modification of the compositions and/or the content of metabolites, in particular proteins, oils, vitamins and/or starch. Also, growth rate, yield or resistance to biotic or abiotic stress factors can be increased. The subsequent applications in the field of plant biotechnology are particularly advantageous.

A further aspect of the invention relates to the a transgenic plant or plant cell comprising a polycistronic RNA of the invention, or an expression cassette or expression vector for expression of said polycistronic RNA. Another embodiment relates to the use of the transgenic organism according to the invention (e.g., the transgenic plant) and of the cells, cell cultures, parts - such as, for example, in the case of transgenic plant organisms roots, leaves and the like - derived from them and transgenic propagation material such as seeds or fruits for the production of foodstuffs or feeding stuffs, pharmaceuticals or fine chemicals, such as, for example, enzymes, vitamins, amino acids, sugars, fatty acids, natural or synthetic flavorings, aromas and colorants. Especially preferred is the production of triacylglycerides, lipids, oils, fatty acids, starches, tocopherols and tocotrienols and carotenoids. Genetically modified plants according to the invention which can be consumed by humans and animals can also be used as foodstuffs or feeding stuffs, for example directly or after undergoing a processing which is known per se.

#### 5.1.1 Target genes for plant biotechnology applications

The possible target genes stated are to be understood by way of example, but not by limitation:

**5.1.1.1** Improved protection against abiotic stress factors (heat, chill, drought, increased moisture, environmental toxins, UV radiation). It is preferred to reduce the expression of genes, which are involved in stress reactions.

**5.1.1.2 Modification of the composition and/or the content of fatty acids, lipids or oils**

A modification of the fatty acid contents or the fatty acid composition, preferably in an oil crop such as oilseed rape or sunflower, can be achieved, for example, by reducing the gene expression of fatty acid biosynthesis genes, preferably those selected from the group consisting of genes encoding acetyl transacylases, acyl transport proteins ("acyl carrier protein"), desaturases such as stearyl desaturases or microsomal D12-desaturases, in particular Fad2-1 genes, malonyl transacylase, β-ketoacyl-ACP synthetases, 3-keto-ACP reductases, enoyl-ACP hydrases, thioesterases such as acyl-ACP thioesterases, enoyl-ACP reductases. Various further advantageous approaches for modifying the lipid composition are described (Shure M *et al*. (1983) Cell 35:225-233; Preiss *et al*.(1987) Tailoring Genes for Crop Improvement (Bruening *et al*., eds.), Plenum Press, S.133-152; Gupta *et al*. (1988) Plant Mol Biol. 10:215-224; Olive *et al*. (1989) Plant Mol Biol 12:525-538; Bhattacharyya *et al*. (1990) Cell 60:155-122; Dunwell

JM (2000) J Exp Botany 51 Spec No:487-96; Brar DS *et al*. (1996) Biotech Genet Eng Rev 13:167-79; Kishore GM and Somerville CR (1993) Curr Opin Biotech 4(2):152-8). Preferred are, in particular, Fad2 genes (for example those described by Genbank Acc. No.: AF124360 *(Brassica carinata)*, AF042841 *(Brassica rapa),* L26296 *(Arabidopsis thaliana)*, A65102 (Corylus avellana)). Further advantageous genes and methods for modifying the lipid content are described, for example, in US 5,530,192 and WO 94/18337. An elevated lipid content can also be achieved by reducing the starch content, for example as the result of the reduced expression of enzymes of the carbohydrate metabolism (for example ADP-glucose pyrophosphorylases).

### 5.1.1.3 Modification of the carbohydrate composition

A modification of the carbohydrate composition can be achieved for example by reducing the gene expression of carbohydrate metabolism genes or of carbohydrate biosynthesis genes, for example genes of the biosynthesis of amylose, pectins, cellulose or cell-wall carbohydrates. A multiplicity of cellular processes (maturation, storability, starch composition or starch content and the like) can thereby be influenced in an advantageous manner. Target genes which may be mentioned by way of example, but not by limitation, are phosphorylases, starch synthetases, Q-enzymes, sucrose-6-phosphate synthetases, sucrose-6-phosphate phosphatases, ADP-glucose pyrophosphorylases, branching enzymes, debranching enzymes and various amylases. The corresponding genes are described (Dunwell JM (2000) J Exp Botany 51 Spec No:487-96; Brar DS *et al*. (1996) Biotech Genet Eng Rev 13:167-79; Kishore GM and Somerville CR (1993) Curr Opin Biotech 4(2):152-8). Advantageous genes for influencing the carbohydrate metabolism - in particular starch biosynthesis - are described in WO 92/11375, WO 92/11376, US 5,365,016 and WO 95/07355.

### 5.1.1.4 Modification of the color or pigmentation

A modification of the color or pigmentation, preferably of ornamentals, can be achieved for example by reducing the gene expression of flavonoid biosynthesis genes such as, for example, the genes of chalcone synthases, chalcone isomerases, phenylalanine ammonia lyases, dehydrokaempferol(flavone) hydroxylases such as flavanone 3-hydroxylases or flavanone 2-hydroxylases, dihydroflavonol reductases, dihydroflavanol 2-hydroxylases, flavonoid 3'-hydroxylases, flavonoid 5'-hydroxylases, flavonoid glycosyltransferases (for example glucosyltransferases such as UDPG:flavonoid 3-O-glucosyltransferases, UDPG:flavonol 7-O-glucosyltransferases or rhamnosyltransferases), flavonoid methyltransferases (such as, for example, SAM:anthocyanidin-3-(p-coumaroyl)rutinoside-5-glucoside-3',5'-O-methyltransferases) and flavonoid acyltransferases (Hahlbrock (1981) Biochemistry of Plants, Vol.7, Conn (Ed.); Weiring and de Vlaming (1984) "Petunia", KC Sink (Ed.), Springer-Verlag, New York). Particularly suitable are the sequences described in EP-A1 522 880.

### 5.1.1.5. Reduction of the storage protein content

The reduction of the gene expression of genes encoding storage proteins (SP hereinbelow) has a large number of advantages such as, for example, the reduction of the allergenic potential or modification in the composition or quantity of other metabolites. Storage proteins are described, inter alia, in EP-A 0 591 530, WO 87/47731, WO 98/26064, EP-A 0 620 281; Kohno-Murase J *et al*. (1994) Plant Mol Biol 26(4): 1115-1124. SP serve for the storage of carbon, nitrogen and sulfur, which are required for the rapid heterotrophic growth in the germination of seeds or pollen. In most cases, they have no enzymatic activity. SP are synthesized in the embryo only during seed development and, in this process, accumulate firstly in protein storage vacuoles (PSV) of differently differentiated cells in the embryo or endosperm. Storage proteins can be classified into subgroups, as the function of further characteristic properties, such as, for example, their sedimentation coefficient or the solubility in different solutions (water, saline, alcohol). The sedimentation coefficient can be determined by means of ultracentrifugation in the manner with which the skilled worker is familiar (for example as described in Correia JJ (2000) Methods in Enzymology 321:81-100). In total, four large gene families for storage proteins can be assigned, owing to their sequences: 2S albumins (napin-like), 7S globulins (phaseolin-like), 11S/12S globulins (legumin/cruciferin-like) and the zein prolamins.

2S albumins are found widely in seeds of dicots, including important commercial plant families such as Fabaceae (for example soybean), Brassicaceae (for example oilseed rape), Euphorbiaceae (for example castor-oil plant) or Asteraceae (for example sunflower). 2S albumins are compact globular proteins with conserved cysteine residues which frequently form heterodimers. 7S globulins occur in trimeric form and comprise no cysteine residues. After their synthesis, they are cleaved into smaller fragments and glycosylated, as is the case with the 2S albumins. Despite differences in polypeptide size, the different 7S globulins are highly conserved and can probably be traced to a shared precursor protein, as is the case with the 2S albumins. Only small amounts of the 7S globulins are found in monocots. In dicots, they always amount to less than the 11 S/12S globulins. 11 S/12S globulins constitute the main fraction of the storage proteins in dicots, in addition to the 2S albumins. The high degree of similarity of the different 11S globulins from different plant genera, in turn, allow the conclusion of a shared precursor protein in the course of evolution. The storage protein is preferably selected from the classes of the 2S albumins (napin-like), 7S globulins (phaseolin-like), 11S/12S globulins (legumin/cruciferin-like) or zein prolamins. Especially preferred 11 S/12S globulins comprise preferably 11 S globulins from oilseed rape, soybean and Arabidopsis, sunflower, linseed, sesame, safflower, olive tree, soybean or various nut species. Especially preferred zein prolamins preferably comprise those from monocotyledonous plants, in particular maize, rice, oats, barley or wheat.

**5.1.1.6. Obtaining a resistance to plant pathogens**

The methods and means of the invention will be especially suited for obtaining pathogen (e.g., virus or nematode) resistance, in eukaryotic cells or organisms, particularly in plant cells and plants. It is expected that the polycistronic RNA molecules (or the dsRNA molecules derived therefrom) produced by transcription in a host organism (e.g., a plant), can spread systemically throughout the organism. Thus it is possible to reduce the phenotypic expression of a nucleic acid in cells of a non-transgenic scion of a plant grafted onto a transgenic stock comprising the chimeric genes of the invention (or vice versa) a method which may be important in horticulture, viticulture or in fruit production.

A resistance to plant pathogens such as arachnids, fungi, insects, nematodes, protozoans, viruses, bacteria and diseases can be achieved by reducing the gene expression of genes which are essential for the growth, survival, certain developmental stages (for example pupation) or the multiplication of a certain pathogen. A suitable reduction can bring about a complete inhibition of the above steps, but also a delay of same. This may be plant genes which, for example, allow the pathogen to enter, but may also be pathogen-homologous genes. Preferably, the polycistronic RNA (or the dsRNA derived therefrom) is directed against genes of the pathogen. For example, plants can be treated with suitable formulations of abovementioned agents, for example sprayed or dusted, the plants themselves, however, may also comprise the agents in the form of a transgenic organism and pass them on to the pathogens, for example in the form of a stomach poison. Various essential genes of a variety of pathogens are known to the skilled worker (for example for nematode resistance: WO 93/10251, WO 94/17194).

Thus, an aspect of this invention provides a method where the target gene for suppression encodes a protein in a plant pathogen (e.g., an insect or nematode). In an aspect, a method comprises introducing into the genome of a pathogen-targeted plant a nucleic acid construct comprising DNA which is transcribed into a polycistronic RNA that forms at least one dsRNA molecule which is effective for reducing expression of a target gene within the pathogen when the pathogen (e.g., insect or nematode) ingests or infects cells from said plant. In a preferred embodiment, the gene suppression is fatal to the pathogen.

Most preferred as pathogen are fungal pathogens such as *Phytophthora infestans, Fusarium nivale, Fusarium graminearum, Fusarium culmorum, Fusarium oxysporum, Blumeria graminis, Magnaporthe grisea, Sclerotinia sclerotium, Septoria nodorum, Septoria tritici, Alternaria brassicae, Phoma lingam, bacterial pathogens such as Corynebacterium sepedonicum, Erwinia carotovora, Erwinia amylovora, Streptomyces scabies, Pseudomonas syringae pv. tabaci, Pseudomonas syringae pv. phaseolicola, Pseudomonas syringae pv. tomato, Xanthomonas campestris pv. malvacearum and Xanthomonas campestris pv. oryzae,* and nematodes such as *Globodera rostochiensis, G. pallida, Heterodera schachtii, Heterodera avenae, Ditylenchus dipsaci, Anguina tritici* and *Meloidogyne hapla.*

Resistance to viruses can be obtained for example by reducing the expression of a viral coat protein, a viral replicase, a viral protease and the like. A large number of plant viruses and suitable target genes are known to the skilled worker. The methods and compositions of the present invention are especially useful to obtain nematode resistant plants (for target genes see e.g., WO 92/21757, WO 93/10251, WO 94/17194).

Also provided by the invention is a method for obtaining pathogen resistant organisms, particularly plants, comprising the steps of providing cells of the organism with an polycistronic RNA molecule of the invention, said polycistronic RNA molecule capable to provide in an eukaryotic cell an at least partially double-stranded RNA molecule, said polycistronic RNA molecule comprising

a) at least one first ribonucleotide sequence that is substantially identical to at least a part of a target nucleotide sequence of at least one gene of a pathogen, and

b) at least one second ribonucleotide sequence which is substantially complementary to said first nucleotide sequence and is capable to hybridizes to said first nucleotide sequence to form a double-stranded RNA structure, and

c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

Preferably, said first ribonucleotide sequence has between 65 and 100% sequence identity, preferably, between 75 and 100%, more preferably between 85 and 100%, most preferably between 95 and 100%, with at least part of the nucleotide sequence of the genome of a pathogen. More preferably the pathogen is selected from the group of virus, bacteria, fungi, and nematodes.

**5.1.1.7. Prevention of stem break**

A reduced susceptibility to stem break can be obtained for example by reducing the gene expression of genes of the carbohydrate metabolism (see above). Advantageous genes are described (WO 97/13865, inter alia) and comprise tissue-specific polygalacturonases or cellulases.

**5.1.1.8. Delay of fruit maturation**

Delayed fruit maturation can be achieved for example by reducing the gene expression of genes selected from the group consisting of polygalacturonases, pectin esterases, β-(1-4)glucanases (cellulases), β-galactanases (β-galactosidases), or genes of ethylene biosynthesis, such as 1-aminocyclopropane-1-carboxylate synthase, genes of carotenoid biosynthesis such as, for example, genes of prephytoene or phytoene biosynthesis, for example phytoene desaturases. Further advantageous genes are, for example, in WO 91/16440, WO 91/05865, WO 91/16426, WO 92/17596, WO 93/07275 or WO 92/04456, US 5,545,366).

5.1.1.9. Achieving male sterility. Suitable target genes are described in WO 94/29465, WO89/10396, WO 92/18625, inter alia. A particular application for reduction of the phenotypic expression of a transgene in a plant cell, inter alia, has been described for the restoration of male fertility, the latter being obtained by introduction of a transgene comprising a male sterility DNA (WO 94/09143, WO 91/02069). The nucleic acid of interest is specifically the male sterility DNA.

**5.1.1.10.** Reduction of undesired or toxic plant constituents such as, for example, glucosinolates. Suitable target genes are described (in WO 97/16559, inter alia).

**5.1.1.11.** Delay of senescence symptoms. Suitable target genes are, inter alia, cinnamoyl-CoA:NADPH reductases or cinnamoyl alcohol dehydrogenases. Further target genes are described (in WO 95/07993, inter alia).

**5.1.1.12**. Modification of the lignification and/or the lignin content, mainly in tree species. Suitable target genes are described in WO 93/05159, WO 93/05160, inter alia.

**5.1.1.13.** Modification of the fiber content in foodstuffs, preferably in seeds, by reducing the expression of coffeic acid O-methyltransferase or of cinnamoyl alcohol dehydrogenase.

**5.1.1.14.** Modification of the fiber quality in cotton. Suitable target genes are described in US 5,597,718, inter alia.

**5.1.1.15.** Reduction of the susceptibility to bruising of, for example, potatoes by reducing for example polyphenol oxidase (WO 94/03607) and the like.

**5.1.1.16.** Enhancement of vitamin E biosynthesis, for example by reducing the expression of genes from the homogentisate catabolic pathway such as, for example, homogentisate 1,2-dioxygenase (HGD; EC No.: 1.13.11.5), maleylacetoacetate isomerase (MAAI; EC No.: 5.2.1.2.) or fumaryl-acetoacetate hydrolase (FAAH; EC No.: 3.7.1.2).

**5.1.1.17.** Reduction of the nicotine content for example in tobacco by reduced expression of, for example, N-methylputrescin oxidase and putrescin N-methyltransferase.

**5.1.1.18.** Reduction of the caffeine content in coffee bean (e.g., *Coffea arabica*) by reducing the gene expression of genes of caffeine biosynthesis such as 7-methylxanthine 3-methyltransferase.

**5.1.1.19.** Reduction of the theophylline content in tea (Camellia sinensis) by reducing the gene expression of genes of theophylline biosynthesis such as, for example, 1-methylxanthine 3-methyltransferase.

**5.1.1.20.** Increase of the methionine content by reducing threonine biosynthesis, for example by reducing the expression of threonine synthase (Zeh M *et al* .(2001) Plant Physiol 127(3):792-802).

Furthermore the method and compounds of the invention can be used for obtaining shatter resistance (WO 97/13865), for obtaining modified flower color patterns (EP 522 880, US 5,231,020), for reducing the presence of unwanted (secondary) metabolites in organisms, such as glucosinolates (WO97/16559) or chlorophyll content (EP 779 364) in plants, for modifying the profile of metabolites synthesized in a eukaryotic cell or organisms by metabolic engineering e.g. by reducing the expression of particular genes involved in carbohydrate metabolism (WO 92/11375, WO 92/11376, US 5,365,016, WO 95/07355) or lipid biosynthesis (WO 94/18337, US 5,530,192) etc. Further examples of advantageous genes are mentioned for example in Dunwell JM, Transgenic approaches to crop improvement, J Exp Bot. 2000;51 Spec No; pages 487-96.

Each of the abovementioned applications can be used as such on its own. Naturally, it is also possible to use more than one of the abovementioned approaches simultaneously. If, in this context, all approaches are used, the expression of at least two differing target genes as defined above is reduced. In this context, these target genes can originate from a single group of genes which is preferred for a use, or else be assigned to different use groups.

## 5.2. Plant Transformation Technology

A polycistronic RNA of the invention can be expressed within a plant cell using conventional recombinant DNA technology. Generally, this involves inserting a nucleotide sequence encoding the polycistronic RNA of the invention into an expression cassette or expression vector using standard cloning procedures known in the art.

### 5.2.1. Requirements for Construction of Plant Expression Cassettes

The expression cassette or expression construct of the invention comprises one or more genetic control sequences (or regulatory sequences) operably linked to a nucleic acid sequence encoding the polycistronic RNA of the invention. These genetic control sequences regulate expression of the polycistronic RNA in host cells. Genetic control sequences are described, for example, in "Goeddel; Gene Expression Technology: Methods in Enzymology 185, Academic Press, San Diego, CA (1990)° or "Gruber and Crosby, in: Methods in Plant Molecular Biology and Biotechnolgy, CRC Press, Boca Raton, Florida, eds.: Glick and Thompson, Chapter 7, 89-108" and the references cited therein. Sequences intended for expression in plants are first operatively linked to a suitable promoter functional in plants. Such expression cassettes optionally comprise further sequences required or selected for the expression of the transgene. Such sequences include, but are not restricted to, transcription terminators, extraneous sequences to enhance expression. These expression cassettes are easily transferred to the plant transformation vectors described infra.

#### 5.2.1.1. Promoters

The nucleic acid sequence encoding the polycistronic RNA of the invention is preferably operably linked to a plant-specific promoter. The term "plant-specific promoter" means principally any promoter which is capable of governing the expression of genes, in particular foreign nucleic acid sequences or genes, in plants or plant parts, plant cells, plant tissues, plant cultures. In this context, the expression specificity of said plant-specific promoter can be for example constitutive, tissue-specific, inducible or development-specific. The following are preferred:

##### 5.2.1.1.1 Constitutive promoters

Where expression of a gene in all tissues of a transgenic plant or other organism is desired, one can use a "constitutive" promoter, which is generally active under most environmental conditions and states of development or cell differentiation. Useful promoters for plants also include those obtained from Ti-or Ri-plasmids, from plant cells, plant viruses or other organisms whose promoters are found to be functional in plants. Bacterial promoters that function in plants, and thus are suitable for use in the methods of the invention include the octopine synthetase promoter, the nopaline synthase promoter, and the mannopine synthetase promoter. The promoter controlling moter, and the mannopine synthetase promoter. The promoter controlling expression of the polycistronic RNA of the invention (and/or selection marker) can be constitutive. Suitable constitutive promoters for use in plants include, for example, the cauliflower mosaic virus (CaMV) 35S transcription initiation region (Franck *et al.* (1980) Cell 21:285-294; Odell *et al.* (1985) Nature 313:810-812; Shewmaker *et al.* (1985) Virology 140:281-288; Gardner *et al.* (1986) Plant Mol Biol 6:221-228), the 19S transcription initiation region (US 5,352,605 and WO 84/02913), and region VI promoters, the 1'-or 2'-promoter derived from T-DNA of *Agrobacterium tumefaciens*, and other promoters active in plant cells that are known to those of skill in the art. Other suitable promoters include the full-length transcript promoter from Figwort mosaic virus, actin promoters (*e.g.*, the rice actin promoter; McElroy *et al.* (1990) Plant Cell 2: 163-171), histone promoters, tubulin promoters, or the mannopine synthase promoter (MAS). Other constitutive plant promoters include various ubiquitin or poly-ubiquitin promoters (Sun and Callis (1997) Plant J 11 (5): 1017-1027, Cristensen *et al.* (1992) Plant Mol Biol 18:675-689; Christensen *et al.* (1989) Plant Mol. Biol. 12: 619-632; Bruce *et al.* (1989) Proc Natl Acad Sci USA 86:9692-9696; Holtorf *et al.* (1995) Plant Mol Biol 29:637-649), the mas, Mac or DoubleMac promoters (US 5,106,739; Comai *et al.* (1990) Plant Mol Biol 15:373-381), the ubiquitin promoter (Holtorf *et al.* (1995) Plant Mol Biol 29:637-649), Rubisco small subunit (SSU) promoter (US 4,962,028), the legumin B promoter (GenBank Acc. No. X03677), the promoter of the nopaline synthase (NOS) from *Agrobacterium*, the TR dual promoter, the octopine synthase (OCS) promoter from *Agrobacterium*, the Smas promoter, the cinnamyl alcohol dehydrogenase promoter (US 5,683,439), the promoters of the vacuolar ATPase subunits, the pEMU promoter (Last *et al.* (1991) Theor. Appl. Genet. 81, 581-588); the MAS promoter (Velten *et al.* (1984) EMBO J. 3(12): 2723-2730), the maize H3 histone promoter (Lepetit *et al.* (1992) Mol. Gen. Genet. 231: 276-285; Atanassova *et al.* (1992) Plant J 2(3): 291-300), α-conglycinin promoter, the phaseolin promoter, the ADH promoter, and heatshock promoters, the nitrilase promoter from *Arabidopsis thaliana* (WO 03/008596; GenBank Acc. No.: U38846, nucleotides 3,862 to 5,325 or else 5342), promoter of a proline-rich protein from wheat (WO 91/13991), the promoter of the *Pisum sativum* ptxA gene, and other transcription initiation regions from various plant genes known to those of skill in the art.

However, it has to be noted that because of the high efficiency of the polycistronic RNA of the invention, the method of the current invention does not rely on the presence of strong promoter regions to drive the transcriptional production of the polycistronic RNA. In other words, a whole range of promoters, particularly plant expressible promoters, is available to direct the transcription.

**5.2.1.1.2 Tissue-specific promoters**

Alternatively promoters can be employed which regulate expression in only one or some tissues or organs, such as leaves, roots, fruit, seeds, anthers, ovaries, pollen, meristem, stems or flowers, or parts thereof. For example, the tissue-specific ES promoter from tomato is particularly useful for directing gene expression so that a desired gene product is located in fruits (see, e.g., Lincoln *et al.* (1988) Proc Natl Acad Sci USA 84:2793-2797; Deikman *et al.* (1988) EMBO J 7:3315-3320; Deikman *et al.* (1992) Plant Physiol 100:2013-2017). Suitable seed specific promoters include those derived from the following genes: MAC1 from maize (Sheridan *et al.* (1996) Genetics 142:1009-1020), Cat3 from maize (GenBank No. L05934), the gene encoding oleosin 18kD from maize (GenBank No. J05212) viviparous-1 from Arabidopsis (Genbank Acc.-No. U93215), the gene encoding oleosin from *Arabidopsis* (Genbank Acc.-No. Z17657), Atmycl from Arabidopsis (Urao *et al.* (1996) Plant Mol Biol 32:571-576), the 2S seed storage protein gene family from *Arabidopsis* (Conceicao *et al.* (1994) Plant 5:493-505;) the gene encoding oleosin 20kD from *Brassica napus* (GenBank No. M63985), napin from *Brassica napus* (GenBank No. J02798, Joseffson *et al.* (1987) J Biol Chem 262:12196-12201), the napin gene family (e.g., from *Brassica napus;* Sjodahl *et al.* (1995) Planta 197:264-271), US 5,608,152; Stalberg *et al.* (1996) Planta 199:515-519), the gene encoding the 2S storage protein from *Brassica napus* (Dasgupta *et al.* (1993) Gene 133: 301-302), the genes encoding oleosin A (Genbank Acc.-No. U09118) and oleosin B (Genbank No. U09119) from soybean, the gene encoding low molecular weight sulphur rich protein from soybean (Choi *et al.* (1995) Mol Gen Genet 246:266-268), the phaseolin gene (US 5,504,200, Bustos *et al.* (1989) Plant Cell 1(9):839-53; Murai *et al.* (1983) Science 23: 476-482; Sengupta-Gopalan *et al.* (1985) Proc. Nat'l Acad. Sci. USA 82: 3320-3324 (1985)), the 2S albumin gene, the legumin gene (Shirsat *et al.* (1989) Mol Gen Genet 215(2):326-331), the USP (unknown seed protein) gene, the sucrose binding protein gene (WO 00/26388), the legumin B4 gene (LeB4; Fiedler *et al.* (1995) Biotechnology (NY) 13(10):1090-1093), Bäumlein *et al.* (1992) Plant J 2(2):233-239; Bäumlein *et al.* (1991 a) Mol Gen Genet 225(3):459-467; Bäumlein *et al.* (1991 b) Mol Gen Genet 225:121-128), the *Arabidopsis* oleosin gene (WO 98/45461), the *Brassica* Bce4 gene (WO 91/13980), genes encoding the "high-molecular-weight glutenin" (HMWG), gliadin, branching enzyme, ADP-glucose pyrophosphatase (AGPase) or starch synthase. Furthermore preferred promoters are those which enable seed-specific expression in monocots such as maize, barley, wheat, rye, rice and the like. Promoters which may advantageously be employed are the promoter of the *lpt2* or *lpt1* gene (WO 95/15389, WO 95/23230) or the promoters described in WO 99/16890 (promoters of the hordein gene, the glutelin gene, the oryzin gene, the prolamine gene, the gliadin gene, the zein gene, the kasirin gene or the secalin gene). Further preferred are a leaf-specific and light-induced promoter such as that from cab or Rubisco (Timko *et al.* (1985) Nature 318: 579-582; Simpson *et al.* (1985) EMBO J 4:2723-2729); an anther-specific promoter such as that from LAT52 (Twell *et al.* (1989) Mol Gen Genet 217:240-245); a pollen-specific promoter such as that from Zml3 (Guerrero *et al.* (1993) Mol Gen Genet 224:161-168); and a microspore-preferred promoter such as that from apg (Twell *et al.* (1983) Sex. Plant Reprod. 6: 217-224). Further suitable promoters are, for example, specific promoters for tubers, storage roots or roots such as, for example, the class I patatin promoter (B33), the potato cathepsin D inhibitor promoter, the starch synthase (GBSS1) promoter or the sporamin promoter, and fruit-specific promoters such as, for example, the tomato fruit-specific promoter(EP-A 409 625). Promoters which are furthermore suitable are those which ensure leaf-specific expression. Promoters which may be mentioned are the potato cytosolic FBPase promoter (WO 98/18940), the Rubisco (ribulose-1,5-bisphosphate carboxylase) SSU (small subunit) promoter or the potato ST-LSI promoter (Stockhaus *et al.* (1989) EMBO J 8(9):2445-2451). Other preferred promoters are those which govern expression in seeds and plant embryos. Further suitable promoters are, for example, fruit-maturation-specific promoters such as, for example, the tomato fruit-maturation-specific promoter (WO 94/21794), flower-specific promoters such as, for example, the phytoene synthase promoter (WO 92/16635) or the promoter of the P1-rr gene (WO 98/22593) or another node-specific promoter as described in EP-A 249676 may be used advantageously. The promoter may also be a pith-specific promoter, such as the promoter isolated from a plant TrpA gene as described in W0 93/07278.

**5.2.1.1.3 Chemically inducible promoters**

An expression cassettes may also contain a chemically inducible promoter (review article: Gatz *et al.* (1997) Annu Rev Plant Physiol Plant Mol Biol 48:89-108), by means of which the expression of the nucleic acid sequence encoding the polycistronic RNA of the invention in the plant can be controlled at a particular point in time. Such promoters such as, for example, a salicylic acid-inducible promoter (WO 95/19443), a benzenesulfonamide-inducible promoter (EP 0 388 186), a tetracycline-inducible promoter (Gatz *et al.* (1991) Mol Gen Genetics 227:229-237), an abscisic acid-inducible promoter EP 0 335 528) or an ethanol-cyclohexanone-inducible promoter (WO 93/21334) can likewise be used. Also suitable is the promoter of the glutathione-S transferase isoform II gene (GST-II-27), which can be activated by exogenously applied safeners such as, for example, N,N-diallyl-2,2-dichloroacetamide (WO 93/01294) and which is operable in a large number of tissues of both monocotyledonous and dicotyledonous. Further exemplary inducible promoters that can be utilized in the instant invention include that from the ACE1 system which responds to copper (Mett *et al.* PNAS 90: 4567-4571 (1993)); or the In2 promoter from maize which responds to benzenesulfonamide herbicide safeners (Hershey *et al.* (1991) Mol Gen Genetics 227:229-237; Gatz *et al.* (1994) Mol Gen Genetics 243:32-38). A promoter that responds to an inducing agent to which plants do not normally respond can be utilized. An exemplary inducible promoter is the inducible promoter from a steroid hormone gene, the transcriptional activity of which is induced by a glucocorticosteroid hormone (Schena *et al.* (1991) Proc Nat'l Acad Sci USA 88:10421). Other preferred promoters are promoters induced by biotic or abiotic stress, such as, for example, the pathogen-inducible promoter of the PRP1 gene (Ward *et al.* (1993) Plant Mol Biol 22:361-366), the tomato heat-inducible hsp80 promoter (US 5,187,267), the potato chill-inducible alpha-amylase promoter (WO 96/12814) or the wound-induced pinll promoter (EP-A1 0 375091).

**5.2.1.1.4 Stress- or pathogen-inducible promoters**

One can use a promoter that directs expression environmental control. Examples of environmental conditions that may affect transcription by inducible promoters include biotic or abiotic stress factors or other environmental conditions, for example, pathogen attack, anaerobic conditions, ethylene or the presence of light.

Promoters inducible by biotic or abiotic stress include but are not limited to the pathogen-inducible promoter of the PRP1 gene (Ward *et al.* (1993) Plant Mol Biol 22:361-366), the heat-inducible hsp70 or hsp80 promoter from tomato (US 5,187,267), the chill-inducible alpha-amylase promoter from potato (WO 96/12814), the light-inducible

PPDK promoter or the wounding-inducible pinll promoter (EP375091). Pathogen-inducible promoters comprise those of genes which are induced as the result of attack by pathogens such as, for example, genes of PR proteins, SAR proteins, b-1,3-glucanase, chitinase and the like (for example Redolfi *et al.* (1983) Neth J Plant Pathol 89:245-254; Uknes, *et al.* (1992) The Plant Cell 4:645-656; Van Loon (1985) Plant Mol Viral 4:111-116; Marineau *et al.* (1987) Plant Mol Biol 9:335-342; Matton *et al.* (1987) Molecular Plant-Microbe Interactions 2:325-342; Somssich *et al.* (1986) Proc Natl Acad Sci USA 83:2427-2430; Somssich *et al.* (1988) Mol Gen Genetics 2:93-98; Chen *et al.* (1996) Plant J 10: 955-966; Zhang and Sing (1994) Proc Natl Acad Sci USA 91:2507-2511; Warner, *et al.* (1993) Plant J 3:191-201; Siebertz *et al.* (1989) Plant Cell 1:961-968(1989)). Also comprised are wounding-inducible promoters such as that of the pinll gene (Ryan (1990) Ann Rev Phytopath 28:425-449; Duan *et al.* (1996) Nat Biotech 14:494-498), of the wun1 and wun2 gene (US 5,428,148), of the win1 and win2 gene (Stanford *et al.* (1989) Mol Gen Genet 215:200-208), of systemin (McGurl *et al.* (1992) Science 225:1570-1573), of the WIP1 gene (Rohmeier *et al.* (1993) Plant Mol Biol 22:783-792; Eckelkamp *et al.* (1993) FEBS Letters 323:73-76), of the MPI gene (Corderok *et al.* (1994) The Plant J 6(2):141-150) and the like.

**5.2.1.1.5 Development-dependent promoters**

Further suitable promoters are, for example, fruit-maturation-specific promoters, such as, for example, the fruit-maturation-specific promoter from tomato (WO 94/21794, EP 409 625). Development-dependent promoters include partly the tissue-specific promoters described above since individual tissues are, naturally, formed as a function of the development. A development-regulated promoter is, inter alia, described (Baerson and Lamppa (1993) Plant Mol Biol 22(2):255-67).

**5.2.1.1.6 Other suitable promoter and promoter elements**

Promoters may also encompass further promoters, promoter elements or minimal promoters capable of modifying the expression-governing characteristics. Thus, for example, the tissue-specific expression may take place in addition as a function of certain stress factors, owing to genetic control sequences. Such elements are, for example, described for water stress, abscisic acid (Lam and Chua (1991) J Biol Chem 266(26):17131 -17135) and heat stress (Schoffl *et al.* (1989) Molecular & General Genetics 217(2-3):246-53).

**5.2.1.2 Other genetic control elements**

Genetic control sequences are furthermore to be understood as those permitting removal of the inserted sequences from the genome. Methods based on the cre/lox (Dale and Ow (1991) Proc Nat'l Acad Sci USA 88:10558-10562; Sauer (1998) Methods 14(4):381-92; Odell *et al.* (1990) Mol Gen Genet 223:369-378), FLP/FRT (Lysnik *et al.* (1993) NAR 21:969-975), or Ac/Ds system (Lawson *et al.* (1994) Mol Gen Genet 245:608-615;; Wader *et al.* (1987) in TOMATO TECHNOLOGY 189-198 (Alan R. Liss, Inc.); US 5,225,341; Baker *et al.* (1987) EMBO J 6: 1547-1554) permit a - if appropriate tissue-specific and/or inducible - removal of a specific DNA sequence from the genome of the host organism. Control sequences may in this context mean the specific flanking sequences (e.g., lox sequences), which later allow removal (e.g., by means of cre recombinase).

**5.2.1.2.1 Transcriptional Terminators**

A variety of transcriptional terminators are available for use in expression cassettes. These are responsible for the termination of transcription beyond the transgene and its correct polyadenylation. Appropriate transcriptional terminators are those that are known to function in plants and include the CaMV 35S terminator, the tml terminator, the OCS (octopin synthase) terminator and the NOS (nopalin synthase) terminator and the pea rbcS E9 terminator. These can be used in both monocotyledons and dicotyledons.

**5.2.1.2.2 Sequences for the Enhancement or Regulation of Expression**

Genetic control sequences furthermore also comprise the 5'-untranslated regions, introns or noncoding 3' region of genes, such as, for example, the actin-1 intron, or the Adh1-S introns 1, 2 and 6 (general reference: The Maize Handbook, Chapter 116, Freeling and Walbot, Eds., Springer, New York (1994)). It has been demonstrated that they can play a significant role in the regulation of gene expression and have been shown to enhance expression, particularly in monocotyledonous cells. Thus, it has been demonstrated that 5'-untranslated sequences can enhance the transient expression of heterologous genes. An example which may be mentioned of such translation enhancers is the tobacco mosaic virus 5' leader sequence (Gallie *et al.* (1987) Nucl Acids Res 15:8693-8711) and the like. They can furthermore promote tissue specificity (Rouster J *et al.* (1998) Plant J 15:435-440).

**5.2.2. Construction of Plant Transformation Vectors**

The expression cassette for expression of the polycistronic RNA molecule of the invention is preferably comprised in an expression vector. Numerous transformation vectors for plant transformation are known to the person skilled in the plant transformation arts. The selection of vector will depend upon the preferred transformation technique and the target species for transformation.

**5.2.2.1 Vector elements**

Expression cassettes and the vectors derived therefrom may comprise further functional elements. The term functional element is to be understood in the broad sense and means all those elements, which have an effect on the generation, multiplication or function of the expression cassettes, vectors or transgenic organisms according to the invention. The following may be mentioned by way of example, but not by limitation:

#### 5.2.2.1.1. Selectable Marker Genes

Selectable marker genes are useful to select and separate successfully transformed cells. Preferably, within the method of the invention one marker may be employed for selection in a prokaryotic host, while another marker may be employed for selection in a eukaryotic host, particularly the plant species host. The marker may confer resistance against a biocide, .such as antibiotics, toxins, heavy metals, or the like, or may function by complementation, imparting prototrophy to an auxotrophic host. Preferred selectable marker genes for plants may include but are not be limited to the following:

##### 5.2.2.1.1.1. Negative selection markers

Negative selection markers confer a resistance to a biocidal compound such as a metabolic inhibitor *(e.g.,* 2-deoxyglucose-6-phosphate, WO 98/45456), antibiotics *(e.g.,* kanamycin, G 418, bleomycin or hygromycin) or herbicides (e.g., phosphinothricin or glyphosate). Especially preferred negative selection markers are those which confer resistance to herbicides. These markers can be used - beside their function as a marker - to confer a herbicide resistance trait to the resulting plant. Examples, which may be mentioned, are:

- Phosphinothricin acetyltransferases (PAT; also named Bialophos resistance; bar; de Block *et al.* (1987) EMBO J 6:2513-2518; EP 0 333 033; US 4,975,374)
- 5-enolpyruvylshikimate-3-phosphate synthase (EPSPS; US 5,633,435) or glyphosate oxidoreductase gene (US 5,463,175) conferring resistance to Glyphosate (N-phosphonomethyl glycine) (Shah *et al.* (1986) Science 233: 478)
- Glyphosate degrading enzymes (Glyphosate oxidoreductase; gox),
- Dalapon inactivating dehalogenases (deh)
- Sulfonylurea- and imidazolinone-inactivating acetolactate synthases (for example mutated ALS variants with, for example, the S4 and/or Hra mutation
- Bromoxynil degrading nitrilases (bxn)
- Kanamycin- or. G418- resistance genes (*NPTII*; *NPTI*) coding e.g., for neomycin phos-photransferases (Fraley *et al.* (1983) Proc Natl Acad Sci USA 80:4803), which expresses an enzyme conferring resistance to the antibiotic kanamycin and the related antibiotics neomycin, paromomycin, gentamicin, and G418,
- 2-Deoxyglucose-6-phosphate phosphatase (DOGR1-Gene product; WO 98/45456; EP 0 807 836) conferring resistance against 2-desoxyglucose (Randez-Gil *et al.* (1995) Yeast 11:1233-1240)
- Hygromycin phosphotransferase (HPT), which mediates resistance to hygromycin (Vanden Elzen *et al.* (1985) Plant Mol Biol. 5:299).
- Dihydrofolate reductase (Eichholtz *et al.* (1987) Somatic Cell and Molecular Genetics 13, 67-76)

Additional negative selectable marker genes of bacterial origin that confer resistance to antibiotics include the *aadA* gene, which confers resistance to the antibiotic spectinomycin, gentamycin acetyl transferase, streptomycin phosphotransferase (SPT), aminoglycoside-3-adenyl transferase and the bleomycin resistance determinant (Svab *et al.* (1990) Plant Mol. Biol. 14:197; Jones *et al.*(1987) Mol. Gen. Genet. 210:86; Hille *et al.* (1986) Plant Mol. Biol. 7:171 (1986); Hayford *et al.* (1988) Plant Physiol. 86:1216).

Especially preferred are negative selection markers which confer resistance against the toxic effects imposed by D-amino acids like e.g., D-alanine and D-serine (WO 03/060133; Erikson et al. Nat Biotechnol. 22(4):455-8 (2004)). Especially preferred as negative selection marker in this contest are the daoI gene (EC: 1.4. 3.3 : GenBank Acc.-No.: U60066) from the yeast Rhodotorula gracilis (Rhodosporidium toruloides) and the *E. coli* gene *dsdA* (D-serine dehydratase (D-serine deaminase) [EC: 4.3. 1.18; GenBank Acc.-No.: J01603). Depending on the employed D-amino acid the D-amino acid oxidase markers can be employed as dual function marker offering negative selection (e.g., when combined with for example D-alanine or D-serine) or counter selection (e.g., when combined with D-leucine or D-isoleucine).

##### 5.2.2.1.1.2. Positive selection marker

Positive selection markers are conferring a growth advantage to a transformed plant in comparison with a non-transformed one. Genes like isopentenyltransferase from *Agrobacterium tumefaciens* (strain:PO22; Genbank Acc.-No.: AB025109) may - as a key enzyme of the cytokinin biosynthesis - facilitate regeneration of transformed plants *(e.g.,* by selection on cytokinin-free medium). Corresponding selection methods are described (Ebinuma *et al.* (2000a) Proc Natl Acad Sci USA 94:2117-2121; Ebinuma *et al.* (2000b) Selection of Marker-free transgenic plants using the oncogenes (ipt, rol A, B, C) of *Agrobacterium* as selectable markers, In Molecular Biology of Woody Plants. Kluwer Academic Publishers). Additional positive selection markers, which confer a growth advantage to a transformed plant in comparison with a non-transformed one, are described *e.g.*, in EP-A 0 601 092. Growth stimulation selection markers may include (but shall not be limited to) β-Glucuronidase (in combination with *e.g.*, cytokinin glucuronide), mannose-6-phosphate isomerase (in combination with mannose), UDP-galactose-4-epimerase (in combination with *e.g.*, galactose), wherein mannose-6-phosphate isomerase in combination with mannose is especially preferred.

**5.2.2.1.1.3. Counter selection marker**

Counter selection markers are especially suitable to select organisms with defined deleted sequences comprising said marker (Koprek *et al.* (1999) Plant J 19(6): 719-726). Examples for counter selection marker comprise thymidine kinases (TK), cytosine deaminases (Gleave *et al.* (1999) Plant Mol Biol. 40(2):223-35; Perera *et al.* (1993) Plant Mol. Biol 23(4): 793-799; Stougaard (1993) Plant J 3:755-761), cytochrom P450 proteins (Koprek *et al.* (1999) Plant J 19(6): 7.19-726), haloalkan dehalogenases (Naested (1999) Plant J 18:571-576), *iaaH* gene products (Sundaresan *et al.* (1995) Gene Develop 9: 1797-1810), cytosine deaminase *codA* (Schlaman and Hooykaas (1997) Plant J 11:1377-1385), or *tms2* gene products (Fedoroff and Smith (1993) Plant J 3:273- 289).

**5.2.2.1.2. Reporter genes**

Reporter genes encode readily quantifiable proteins and, via their color or enzyme activity, make possible an assessment of the transformation efficacy, the site of expression or the time of expression. Very especially preferred in this context are genes encoding reporter proteins (Schenbom and Groskreutz (1999) Mol Biotechnol 13(1):29-44) such as the green fluorescent protein (GFP) (Haseloff *et al.*(1997) Proc Natl Acad Sci USA 94(6):2122-2127; Sheen *et al.* (1995) Plant J 8(5):777-784; Reichel *et al.*(1996) Proc Natl Acad Sci USA 93(12):5888-5893; Chui *et al.* (1996) Curr Biol 6:325-330; Leffel *et al.* (1997) Biotechniques. 23(5):912-8; Tian *et al.* (1997) Plant Cell Rep 16:267-271; WO 97/41228), chloramphenicol transferase, a luciferase (Millar *et al.* (1992) Plant Mol Biol Rep 10:324-414; Ow *et al.* (1986) Science 234:856-859), the aequorin gene (Prasher *et al.* (1985) Biochem Biophys Res Commun 126(3):1259-1268), β—galactosidase, R locus gene (encoding a protein which regulates the production of anthocyanin pigments (red coloring) in plant tissue and thus makes possible the direct analysis of the promoter activity without addition of further auxiliary substances or chromogenic substrates (Dellaporta *et al.* (1988) In: Chromosome Structure and Function: Impact of New Concepts, 18th Stadler Genetics Symposium, 11:263-282; Ludwig *et al.* (1990) Science 247:449), with β-glucuronidase (GUS) being very especially preferred (Jefferson (1987b) Plant Mol. Bio. Rep., 5:387-405; Jefferson *et al.* (1987) EMBO J 6:3901-3907). β-glucuronidase (GUS) expression is detected by a blue color on incubation of the tissue with 5-bromo-4-chloro-3-indolyl-β-D-glucuronic acid, bacterial luciferase (LUX) expression is detected by light emission; firefly luciferase (LUC) expression is detected by light emission after incubation with luciferin; and galactosidase expression is detected by a bright blue color after the tissue was stained with 5-bromo-4-chloro-3-indolyl-β-D-galactopyranoside. Reporter genes may also be used as scorable markers as alternatives to antibiotic resistance markers. Such markers are used to detect the presence or to measure the level of expression of the transferred gene. The use of scorable markers in plants to identify or tag genetically modified cells works well only when efficiency of modification of the cell is high.

**5.2.2.1.3. Origins of replication.**

Origins of replication which ensure amplification of the expression cassettes or vectors ac-cording to the invention in, for example, E. coli. Examples which may be mentioned are ORI (origin of DNA replication), the pBR322 ori or the P15A ori (Maniatis T, Fritsch EF and Sambrook J (1989) Molecular Cloning: A Laboratory Manual, 2nd Ed., Cold Spring Harbor Laboratory, Cold Spring Harbor (NY)). Additional examples for replication systems functional in *E. coli*, are ColE1, pSC101, pACYC184, or the like. In addition to or in place of the *E. coli* replication system, a broad host range replication system may be employed, such as the replication systems of the P-1 Incompatibility plasmids; e.g., pRK290. These plasmids are particularly effective with armed and disarmed Ti-plasmids for transfer of T-DNA to the plant host.

**5.2.2.1.4.** Elements, which are necessary for Agrobacterium-mediated transformation, such as, for example, the right and/or - optionally - left border of the T-DNA or the *vir* region.

**5.2.2.1.5.** Multiple cloning sites (MCS) to enable and facilitate the insertion of one or more nucleic acid sequences.

#### 5.2.2.2 Vectors for plant transformation

##### 5.2.2.2.1 Vectors Suitable for Agrobacterium Transformation

If *Agrobacteria* are used, the expression cassette is to be integrated into specific plasmids vectors, either into a shuttle, or intermediate, vector or into a binary vector. If a Ti or Ri plasmid is to be used for the transformation, at least the right border, but in most cases the right and the left border, of the Ti or Ri plasmid T-DNA is flanking the region with the expression cassette to be introduced into the plant genome. It is preferred to use binary vectors for the *Agrobacterium* transformation. Binary vectors are capable of replicating both in *E.coli* and in *Agrobacterium.* They preferably comprise a selection marker gene and a linker or polylinker flanked by the right and - optionally - left T-DNA border sequence. They can be transformed directly into *Agrobacterium* (Holsters *et al.* (1978) Mol Gen Genet 163:181-187). A selection marker gene may be included in the vector which permits a selection of transformed *Agrobacteria* (e.g., the nptIII gene).

The Agrobacterium, which acts as host organism in this case, should already comprise a disarmed (i.e., non-oncogenic) plasmid with the vir region. This region is required for transferring the T-DNA to the plant cell. The use of T-DNA for the transformation of plant cells has been studied and described extensively (EP 120 516; Hoekema, In: The Binary Plant Vector System, Offsetdrukkerij Kanters B.V., Alblasserdam, Chapter V; An *et al.* (1985) EMBO J 4:277-287). A variety of binary vectors are known and available for transformation using Agrobacterium, such as, for example, pBI101.2 or pBIN19 (Clontech Laboratories, Inc. USA; Bevan *et al.*(1984) Nucl Acids Res 12:8711), pBi-nAR, pPZP200 or pPTV.

##### 5.2.2.2.2 Vectors Suitable for Non-Agrobacterium Transformation

Transformation without the use of *Agrobacterium tumefaciens* circumvents the requirement for T-DNA sequences in the chosen transformation vector and consequently vectors lacking these sequences can be utilized in addition to vectors such as the ones described above which contain T-DNA sequences. Transformation techniques that do not rely on Agrobacterium include transformation via particle bombardment, protoplast uptake (e.g. PEG and electroporation) and microinjection. The choice of vector depends largely on the preferred selection for the species .being transformed. Typical vectors suitable for non-Agrobacterium transformation include pCIB3064, pSOG19, and pSOG35. (See, for example, US 5,639,949).

### 5.2.3. Transformation Techniques

#### 5.2.3.1 General techniques

Once an expression cassette or expression vector of the invention has be established, it can be transformed into a plant cell. A variety of methods for introducing nucleic acid sequences (e.g., vectors) into the genome of plants and for the regeneration of plants from plant tissues or plant cells are known (Plant Molecular Biology and Biotechnology (CRC Press, Boca Raton, Florida), chapter 6/7, pp. 71-119 (1993); White FF (1993) Vectors for Gene Transfer in Higher Plants; in: Transgenic Plants, vol. 1, Engineering and Utilization, Ed.: Kung and Wu R, Academic Press, 15-38; Jenes B *et al.* (1993) Techniques for Gene Transfer, in: Transgenic Plants, vol. 1, Engineering and Utilization, Ed.: Kung and R. Wu, Academic Press, pp. 128-143; Potrykus (1991) Annu Rev Plant Physiol Plant Molec Biol 42:205-225; Halford NG, Shewry PR (2000) Br Med Bull 56(1):62-73).

Transformation methods may include direct and indirect methods of transformation. Suitable direct methods include polyethylene glycol induced DNA uptake, liposome-mediated transformation (US 4,536,475), biolistic methods using the gene gun ("particle bombardment"; Fromm ME *et al.* (1990) Bio/Technology. 8(9):833-9; Gordon-Kamm *et al.* (1990) Plant Cell 2:603), electroporation, incubation of dry embryos in DNA-comprising solution, and microinjection. In the case of these direct transformation methods, the plasmid used need not meet any particular requirements. Simple plasmids, such as those of the pUC series, pBR322, M13mp series, pACYC184 and the like can be used. If intact plants are to be regenerated from the transformed cells, an additional selectable marker gene is preferably located on the plasmid. The direct transformation techniques are equally suitable for dicotyledonous and monocotyledonous plants.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). *Agrobacterium* based transformation techniques (especially for dicotyledonous plants) are well known in the art. The Agrobacterium strain (e.g., *Agrobacterium tumefaciens* or *Agrobacterium rhizogenes*) comprises a plasmid (Ti or Ri plasmid) and a T-DNA element which is transferred to the plant following infection with *Agrobacterium*. The T-DNA (transferred DNA) is integrated into the genome of the plant cell. The T-DNA may be localized on the Ri- or Ti-plasmid or is separately comprised in a so-called binary vector. Methods for the *Agrobacterium*-mediated transformation are described, for example, in Horsch RB *et al.* (1985) Science 225:

1229f. The *Agrobacterium*-mediated transformation is best suited to dicotyledonous plants but has also be adopted to monocotyledonous plants. The transformation of plants by Agrobacteria is described (White FF, Vectors for Gene Transfer in Higher Plants; in Transgenic Plants, Vol. 1, Engineering and Utilization, edited by S.D. Kung and R. Wu, Academic Press, 1993, pp. 15 - 38; Jenes B *et al.*(1993) Techniques for Gene Transfer, in: Transgenic Plants, Vol. 1, Engineering and Utilization, edited by S.D. Kung and R. Wu, Academic Press, pp. 128-143; Potrykus (1991) Annu Rev Plant Physiol Plant Molec Biol 42:205- 225).

Transformation may result in transient or stable transformation and expression. Although a nucleotide sequence of the present invention can be inserted into any plant and plant cell falling within these broad classes (as specified above in the DEFINITION section), it is particularly useful in crop plant cells.

Various tissues are suitable as starting material (explant) for the *Agrobacterium*-mediated transformation process including but not limited to callus (US 5,591,616; EP-A1 604 662), immature embryos (EP-A1 672 752), pollen (US 54,929,300), shoot apex (US 5,164,310), or *in planta* transformation (US 5,994,624). The method and material described herein can be combined with virtually all *Agrobacterium* mediated transformation methods known in the art. Preferred combinations include - but are not limited - to the following starting materials and methods:

| Variety | Material / Citation |
|---|---|
| Monocotyledonous plants: | Immature embryos (EP-A1 672 752) Callus (EP-A1 604 662) Embryogenic callus (US 6,074,877) Inflorescence (US 6,037,522) Flower (in planta) (WO 01/12828) |
| Banana | US 5,792,935; EP-A1 731 632; US 6,133,035 |
| Barley | WO 99/04618 |
| Maize | US 5,177,010; US 5,987,840 |
| Pineapple | US 5,952,543; WO 01/33943 |
| Rice | EP-A1 897 013; US 6,215,051; WO 01/12828 |
| Wheat | AU-B 738 153; EP-A1 856 060 |
| Beans | US 5,169,770; EP-A1 397 687 |
| Brassica | US 5,188,958; EP-A1 270 615; EP-A1 1,009,845 |
| Cacao | US 6,150,587 |
| Citrus | US 6,103,955 |
| Coffee | AU 729 635 |
| Cotton | US 5,004,863; EP-A1 270 355; US 5,846,797; EP-A1 1,183,377; EP-A1 1,050,334; EP-A1 1,197,579; EP-A1 1,159,436 Pollen transformation (US 5,929,300) *In planta* transformation (US 5,994,624) |
| Pea | US 5,286,635 |
| Pepper | US 5,262,316 |
| Poplar | US 4,795,855 |
| Soybean | cotyledonary node of germinated soybean seedlings shoot apex (US 5,164,310) axillary meristematic tissue of primary, or higher leaf node of about 7 days germinated soybean seedlings organogenic callus cultures dehydrated embryo axes US 5,376,543; EP-A1 397 687; US 5,416,011; US 5,968,830; US 5,563,055; US 5,959,179; EP-A1 652 965; EP-A1 1,141,346 |
| Sugarbeet | EP-A1 517 833; WO 01/42480 |
| Tomato | US 5,565,347 |

#### 5.2.3.2. Plastid Transformation

In another preferred embodiment, a nucleotide sequence of the present invention (preferably an expression cassette for the polycistronic RNA molecule of the invention) is directly transformed into the plastid genome. Plastid expression, in which genes are inserted by homologous recombination into the several thousand copies of the circular plastid genome present in each plant cell, takes advantage of the enormous copy number advantage over nuclear-expressed genes to permit high expression levels. In a preferred embodiment, the nucleotide sequence is inserted into a plastid targeting vector and transformed into the plastid genome of a desired plant host. Plants homoplasmic for plastid genomes containing the nucleotide sequence are obtained, and are preferentially capable of high expression of the nucleotide sequence.

Plastid transformation technology is for example extensively described in U.S. Pat. Nos. 5,451,513, 5,545,817, 5,545,818, and 5,877,462 in PCT application no. WO 95/16783 and WO 97/32977, and in McBride *et al.* (1994) Proc. Natl. Acad. Sci. USA 91, 7301-7305, all incorporated herein by reference in their entirety. The basic technique for plastid transformation involves introducing regions of cloned plastid DNA flanking a selectable marker together with the nucleotide sequence into a suitable target tissue, e.g., using biolistic or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab et al. (1990) Proc. Natl. Acad. Sci. USA 87, 8526-8530; Staub et al. (1992) Plant Cell 4, 39-45). The presence of cloning sites between these markers allowed creation 39-45). The presence of cloning sites between these markers allowed creation of a plastid targeting vector for introduction of foreign genes (Staub et al. (1993) EMBO J. 12, 601-606). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3'-adenyltransferase (Svab et al. (1993) Proc. Natl. Acad. Sc. USA 90, 913-917). Other selectable markers useful for plastid transformation are known in the art and encompassed within the scope of the invention.

For using the methods according to the invention, the skilled worker has available well-known tools, such as expression vectors with promoters which are suitable for plants, and methods for the transformation and regeneration of plants.

### 5.2.4. Selection and regeneration techniques

To select cells which have successfully undergone transformation, it is preferred to introduce a selectable marker which confers, to the cells which have successfully undergone transformation, a resistance to a biocide (for example a herbicide), a metabolism inhibitor such as 2-deoxyglucose-6-phosphate (WO 98/45456) or an antibiotic. The selection marker permits the transformed cells to be selected from untransformed cells (McCormick *et al.* (1986) Plant Cell Reports 5:81-84). Suitable selection marker are described above.

Transgenic plants can be regenerated in the known manner from the transformed cells. The resulting plantlets can be planted and grown in the customary manner. Preferably, two or more generations should be cultured to ensure that the genomic integration is stable and hereditary. Suitable methods are described (Fennell *et al.* (1992) Plant Cell Rep. 11: 567-570; Stoeger *et al* (1995) Plant Cell Rep. 14:273-278; Jahne *et al.* (1994) Theor Appl Genet 89:525-533).

## 5.3. Pharmaceutical (therapeutic or prophylactic) compositions and methods of the invention

The specificity and sensitivity of compounds, compositions and methods of the invention can also be harnessed by those of skill in the art for therapeutic uses and are suitable for the preparation of pharmaceuticals for the treatment of human and animal diseases and for the production of pharmaceuticals.

Thus, the invention further provides a method for treating or preventing a disease or infection in an animal or human being, preferably a mammal. Yet another embodiment of the invention relates to a pharmaceutically preparation comprising at least one polycistronic RNA of the invention. Preferably, said preparation gives rise to at least one short interfering double stranded RNA (siRNA) in a mammalian cell and attenuates expression of at least one target gene, which polycistronic RNA does not produce a significant PKR-dependent response in the mammalian cell at concentrations effective for attenuating expression of the target gene. Yet another embodiment relates a polycistronic RNA of the invention, an expression cassette or expression vector for its expression, or an non-human organism comprising said polycistronic RNA molecule for use as a pharmaceutical, preferably for the treatment of one or more human or animal diseases. Yet another embodiment relates to the use of a polycistronic RNA of the invention, an expression cassette or expression vector for its expression, or an non-human organism comprising said polycistronic RNA molecule for the preparation of a pharmaceutical, preferably for the treatment of one or more human or animal diseases.

The polycistronic RNA of the invention (or a dsRNA derived therefrom after removal of the removable RNA element) is administered to animal or human being (e.g., the mammal) in a therapeutically or prophylactically effective amount sufficient to attenuate expression of a target gene, the expression of which is associated with the disease or infection. In one embodiment, the expression of the target gene (or alternatively the activity of the target protein expressed therefrom) is inhibited by at least about 10%, preferably by at least about 30%, more preferably by at least 50% or more.

For example, the reduction of the expression of a protein may be measured in serum, adipose tissue, liver or any other body fluid, tissue or organ of the animal. Preferably, the cells contained within the fluids, tissues or organs being analyzed contain a nucleic acid molecule encoding a protein and/or the protein itself.

A variety of disorders can be treated, including infections by heterologous pathogenic organisms, either extracellular or intracellular pathogens. Additionally, the compositions of this invention are useful in preventing infection with a pathogen, or preventing the occurrence of disorders caused by reactivation of a latent pathogen. These compositions are also useful for the treatment of pathogenically-induced cancers. The composition and methods of the invention are especially suitable to treat viral diseases (i.e., HIV, Hepatitis C).

Furthermore other (not pathogen related) disorders and diseases can be treated. Examples of diseases that can be treated by oligonucleotide compositions include: cancer, retinopathies, autoimmune diseases, inflammatory diseases (i.e., ICAM-1 related disorders, Psoriasis, Ulcerative Colitus, Crohn's disease), cardiovascular diseases (such as hypertension), diseases of the central or peripheral nervous system such as Alzheimer's disease, Parkinson's disease or multiple sclerosis, and autosomal dominant genetic disease such as Huntington's chorea (For example, see US 6,506,559; US 2002/0,173,478 A1; US 2002/0,086,356 A1; Shuey, *et al*., "RNAi: gene-silencing in therapeutic intervention." Drug Discov. Today 2002 Oct 15;7(20):1040-6; Aoki, *et al*., "Clin. Exp. Pharmacol. Physiol. 2003 Jan;30(1-2): 96-102; Cioca, *et al*., "RNA interference is a functional pathway with therapeutic potential in human myeloid leukemia cell lines. Cancer Gene Ther. 2003 Feb;10(2):125-33). There are numerous medical conditions for which gene silencing therapy is reported to be suitable (see, e.g., US 5,830,653) as well as respiratory syncytial virus infection (WO 95/22,553) influenza virus (WO 94/23,028), and malignancies (WO 94/08,003). Other examples of clinical uses of antisense sequences are reviewed, e.g., in Glaser. 1996. Genetic Engineering News 16:1. Exemplary targets for cleavage by oligonucleotides include, e.g., protein kinase Ca, ICAM-1, c-raf kinase, p53, c-myb, and the bcr/abl fusion gene found in chronic myelogenous leukemia. The method of the invention can further be used to reduce or prevent the rejection response to transplant tissue (e.g., by silencing MHC proteins). A polycistronic RNA (or the double stranded RNA derived therefrom) that attenuates the expression of a gene in the transplant tissue that can elicit an immune response in the recipient is administered to the transplant tissue.

Also, the method according to the invention makes possible the parallel treatment of more than one disease, such as, for example, a cardiovascular disease and a disease of the central nervous system, which is not generally possible when traditional approaches are used. Such approaches are advantageous especially in the case of multiple diseases as occur frequently with advanced age. An example which may be mentioned is the parallel treatment of hypertension and, for example, Alzheimer's disease or senile dementia.

The compounds and compositions of the invention can be utilized in pharmaceutical compositions by adding an effective amount of the compound or composition to a suitable pharmaceutically acceptable diluent or carrier. Use of the oligomeric compounds and methods of the invention may also be useful prophylactically.

### 5.3.1 Diseases and disorders preferred to be treated bycompositions and methods of the invention

The method according to the invention is particularly suitable for the treatment of the below mentioned diseases and disorders.

#### 5.3.1.1 Pathogen infections

Infection with pathogens, such as, for example, viral or bacterial diseases, in which case the polycistronic RNA (or the dsRNA derived therefrom) attenuates the expression of a bacterial or viral gene. Specifically some of the more desirable viruses to treat with this method include, without limitation, viruses of the species Retrovirus, Herpesvirus, Hepadenovirus, Poxvirus, Parvovirus, Papillomavirus, and Papovavirus, espcially HIV, HBV, HSV, CMV, HPV, HTLV and EBV. The polycistronic RNA used in this method provides to the cell (e.g., of an mammal) an at least partially double-stranded RNA molecule as described above, which is substantially identical to a target polynucleotide which is a virus polynucleotide sequence necessary for replication and/or pathogenesis of the virus in an infected mammalian cell. Among such target polynucleotide sequences are protein-encoding sequences for proteins necessary for the propagation of the virus, e.g., the HIV gag, env, gp41, and pol genes, the HPV6 L1 and E2 genes, the HPV11 L1 and E2 genes, the HPV16 E6 and E7 genes, the HPV18 E6 and E7 genes, the HBV surface antigens, the HBV core antigen, HBV reverse transcriptase, the HSV gD gene, the HSVvp16 gene, the HSV gC, gH, gL and gB genes, the HSV ICP0, ICP4 and ICP6 genes, Varicella zoster gB, gC and GH genes, and the BCR-abl chromosomal sequences, and non-coding viral polynucleotide sequences which provide regulatory functions necessary for transfer of the infection from cell to cell, e.g., the HIV LTR, and other viral promoter sequences, such as HSV vp16 promoter, HSV-ICP0 promoter, HSV-ICP4, ICP6 and gD promoters, the HBV surface antigen promoter, the HBV pre-genomic promoter, among others. The composition (e.g., an dsRNA agent such as the polycistronic RNA molecule of the invention) is administered with an polynucleotide uptake enhancer or facilitator and an optional pharmaceutically acceptable carrier. The amount or dosage which is administered to the mammal is effective to reduce or inhibit the function of the viral sequence in the cells of the mammal.

The method can be used to treat animals (e.g., mammals) already infected with a virus in order to shut down or inhibit a viral gene function essential to virus replication and/or pathogenesis. In still another embodiment of this invention, the compositions described above can be employed in a method to prevent viral infection (e.g., in a mammal). When the polycistronic RNA of the invention is administered prior to exposure of the mammal to the virus, it is expected that the exogenous RNA molecule remains in the mammal and work to inhibit any homologous viral sequence which presents itself to the mammal thereafter. Thus, the compositions of the present invention may be used to inhibit or reduce the function of a viral polynucleotide sequence for vaccine use. Still an analogous embodiment of the above "anti-viral" methods of the invention includes a method for treatment or prophylaxis of a virally induced cancer in a mammal (such cancers include HPV E6/E7 virus-induced cervical carcinoma, and EBV induced cancers).

The compositions of this invention can also be employed for the treatment or prophylaxis of infection by a non-viral pathogen, either intracellular or extracellular. As used herein, the term "intracellular pathogen" is meant to refer to a virus, bacteria, protozoan or other pathogenic organism that, for at least part of its reproductive or life cycle, exists within a host cell and therein produces or causes to be produced, pathogenic proteins. Intracellular pathogens which infect cells which include a stage in the life cycle where they are intracellular pathogens include, without limitation, *Listeria, Chlamydia*, *Leishmania, Brucella, Mycobacteria, Shigella,* and as well as Plasmodia, e.g., the causative agent of malaria, *P. falciparum.* Extracellular pathogens are those which replicate and/or propagate outside of the mammalian cell, e.g., Gonorrhoeae, and Borrellia, among others. According to this embodiment, such infection by an pathogen may be treated or possibly prevented by administering to a mammalian subject, either already infected or anticipating exposure to the pathogen, with a composition as described above with an optional second agent that facilitates polynucleotide uptake in a cell, in a pharmaceutically acceptable carrier. In this case, the RNA molecule of the composition has a polynucleotide sequence which is substantially identical to a target polynucleotide sequence of the pathogen that is necessary for replication and/or pathogenesis of the pathogen in an infected mammal or mammalian cell. As above, the amount of the composition administered is an amount effective to reduce or inhibit the function of the pathogenic sequence in the mammal. The dosages, timing, routes of administration and the like are as described below.

Thus one embodiment of the invention related to a method for reducing the susceptibility of host cells or host organisms to infection by pathogen, comprising introducing a polycistronic RNA of the invention into said host cells or host organisms in an amount sufficient to attenuate expression of one or more genes necessary for expression by said pathogen. Preferably, the pathogen is a virus, a fungus or a nematode. Preferably, the host cell is a plant or an animal, preferably a mammalian, more preferably a human cell.

One of skill in the art, given this disclosure can readily select viral families and genera, or pathogens including prokaryotic and eukaryotic protozoan pathogens as well as multicellular parasites, for which therapeutic or prophylactic compositions according to the present invention can be made. See, e.g., the tables of such pathogens in general immunology texts and in US 5,593,972, incorporated by reference herein.

**5.3.1.2 Cancer**

Treatment of cancer (for example solid tumors and/or leukemias) and inherited disorders. Among conditions particularly susceptible to treatment or prophylaxis according to this invention are those conditions which are characterized by the presence of an aberrant polynucleotide sequence, the function of which is necessary to the initiation or progression of the disorder, but can be inhibited without causing harm or otherwise unduly adversely impacting the health of the organism (e.g. the mammal). Mammalian cancers which are characterized by the presence of abnormal and normal polynucleotide sequences (for details see, e.g., WO94/13793) include chronic myelogenous leukemia (CML) and acute lymphoblastic leukemia (ALL), where the abnormal sequence is a fusion of two normal genes, i.e., bcr-abl. In such cancers or diseases, such as CML, the afflicted mammal also possesses a normal copy of the polynucleotide sequence or gene, and the differences between the abnormal and normal sequences or genes are differences in nucleotide sequence. For example, for CML, the abnormal sequence is the bcr-abl fusion, while the normal sequence is bcr and abl. Thus, the method above can be employed with the target polynucleotide sequence being the sequence which spans the fusion. A method of treatment or prophylaxis of such a cancer in a mammal comprises administering to the mammal a composition of this invention wherein the target polynucleotide is a polynucleotide sequence of an abnormal cancer-causing gene in a mammal which also possesses a normal copy of the gene, and wherein the differences between the abnormal gene and the normal gene are differences in polynucleotide sequence. The skilled worker is familiar with a large number of potential target genes for cancer therapy (for example oncogenes such as ABL1, BCL1, BCL2, BCL6, CBFA2, CBL, CSF1R, ERBA, ERBB, EBRB2, FGR, FOS, FYN, HRAS, JUN, LCK, LYN, MYB, MYC, NRAS, RET or SRC; tumor suppressor genes such as BRCA1 or BRCA2; adhesion molecules; cyclin kinases and their inhibitors). An exemplary list of potential target genes, including developmental genes, oncogenes, and enzymes, and a list of cancers that can be treated according to the present invention can be found in WO 99/32619. A candidate target gene derived from a pathogen might, for example, cause immunosuppression of the host or be involved in replication of the pathogen, transmission of the pathogen, or maintenance of the infection.

### 5.3.2 Formulations and administration

For the purpose of pharmaceutical applications it is preferred that the polycistronic RNA molecule of the invention (or a dsRNA or siRNA derived therefrom) is applied or administered to the target cell or organism directly. Various means for application of RNA as pharmaceutical active ingredient are described in the art.

As used herein "administration" refers to contacting cells (e.g., either in isolated form or comprised in an organism) with the pharmaceutical agent and can be performed in vitro or in vivo. With respect to in vivo applications, the formulations of the present invention can be administered to a patient in a variety of forms adapted to the chosen route of administration, e.g., parenterally, orally, or intraperitoneally. Parenteral administration, which is preferred, includes administration by the following routes: intravenous; intramuscular; interstitially; intraarterially; subcutaneous; intra ocular; intrasynovial; trans epithelial, including transdermal; pulmonary via inhalation; ophthalmic; sublingual and buccal; topically, including ophthalmic; dermal; ocular; rectal; and nasal inhalation via insufflation. Preferably pharmaceutical preparations for the various ways of administration (such as parenteral, transmucosal, transdermal, oral, or topical application) are well known in the art and for example described in US Patent Application No. 20040014956. The pharmaceutical agent of the invention may be administered systemically to a subject. Systemic absorption refers to the entry of drugs into the blood stream followed by distribution throughout the entire body. Administration routes which lead to systemic absorption include: intravenous, subcutaneous, intraperitoneal, and intranasal. The chosen method of delivery will result in entry into cells. Preferred delivery methods include liposomes (10-400 nm), hydrogels, controlled-release polymers, and other pharmaceutically applicable vehicles, and microinjection or electroporation (for ex vivo treatments). Drug delivery vehicles can be chosen e.g., for in vitro, for systemic, or for topical administration. These vehicles can be designed to serve as a slow release reservoir or to deliver their contents directly to the target cell. An advantage of using some direct delivery drug vehicles is that multiple molecules are delivered per uptake. Some examples of such specialized drug delivery vehicles which fall into this category are liposomes, hydrogels, cyclodextrins, biodegradable nanocapsules, and bioadhesive microspheres. In one embodiment, in vitro treatment of cells with oligonucleotides can be used for ex vivo therapy of cells removed from a subject (e.g., for treatment of leukemia or viral infection) or for treatment of cells which did not originate in the subject, but are to be administered to the subject (e.g., to eliminate transplantation antigen expression on cells to be transplanted into a subject). In addition, in vitro treatment of cells can be used in non-therapeutic settings, e.g., to evaluate gene function, to study gene regulation and protein synthesis or to evaluate improvements made to oligonucleotides designed to modulate gene expression or protein synthesis. In vivo treatment of cells can be useful in certain clinical settings where it is desirable to inhibit the expression of a protein.

Compositions for pharmaceutical use of this invention desirably contain a polycistronic RNA molecule, an expression cassette for its production, or a dsRNA molecule derived from said polycistronic RNA as described above (hereinafter the "pharmaceutical dsRNA agent"). Any of the pharmaceutical dsRNA agents can be used alone or in conjunction with a pharmaceutically acceptable carrier and with additional optional components for pharmaceutical delivery. As used herein, "pharmaceutically acceptable carrier" includes appropriate solvents, dispersion media, coatings, antibacterial and antifungal agents, isotonic and absorption delaying agents, and the like. The use of such media and agents for pharmaceutical active substances is well known in the art. Suitable pharmaceutically acceptable carriers facilitate administration of the polynucleotide compositions of this invention, but are physiologically inert and/or nonharmful. Carriers may be selected by one of skill in the art. Such carriers include but are not limited to, sterile saline, phosphate, buffered saline, dextrose, sterilized water, glycerol, ethanol, lactose, sucrose, calcium phosphate, gelatin, dextran, agar, pectin, peanut oil, olive oil, sesame oil, and water and combinations thereof Additionally, the carrier or diluent may include a time delay material, such as glycerol monostearate or glycerol distearate alone or with a wax. In addition, slow release polymer formulations can be used. The formulation should suit not only the form of the delivery agent, but also the mode of administration. Selection of an appropriate carrier in accordance with the mode of administration is routinely performed by those skilled in the art. Additional components for the carrier may include but are not limited to adjuvants, preservatives, chemical stabilizers, or other antigenic proteins. Suitable exemplary preservatives include chlorobutanol, potassium sorbate, sorbic acid, sulfur dioxide, propyl gallate, the parabens, ethyl vanillin, glycerin, phenol, and parachlorophenol. Suitable stabilizing ingredients which may be used include, for example, casamino acids, sucrose, gelatin, phenol red, N-Z amine, monopotassium diphosphate, lactose, lactalbumin hydrolysate, and dried milk. A conventional adjuvant is used to attract leukocytes or enhance an immune response. Such adjuvants include, among others, Ribi, mineral oil and water, aluminum hydroxide, Amphigen, Avridine, L121/squalene, D-lactidepolylactide/glycoside, pluronic plyois, muramyl dipeptide, killed Bordetella, and saponins, such as Quil A.
The pharmaceutical agent may be incorporated into liposomes or liposomes modified with polyethylene glycol or admixed with cationic lipids for parenteral administration. Incorporation of additional substances into the liposome, for example, antibodies reactive against membrane proteins found on specific target cells, can help target the oligonucleotides to specific cell types.

The composition of the invention may also involve lyophilized polynucleotides, which can be used with other pharmaceutically acceptable excipients for developing powder, liquid or suspension dosage forms, including those for intranasal or pulmonary applications. See, e.g., Remington: The Science and Practice of Pharmacy, Vol. 2, 19.sup.th edition (1995), e.g., Chapter 95 Aerosols; and Intemational Patent Application No. PCT/US99/05547, the teachings of which are hereby incorporated by reference.

In some preferred embodiments, the pharmaceutical compositions of the invention are prepared for administration to mammalian subjects in the form of, for example, liquids, emulsions, powders, aerosols, tablets, capsules, enteric coated tablets or capsules, or suppositories. The optimal course of administration or delivery of the pharmaceutical agent may vary depending upon the desired result and/or on the subject to be treated.

The pharmaceutical preparations of the present invention may be prepared and formulated as emulsions or microemulsions. Emulsions are usually heterogenous systems of one liquid dispersed in another in the form of droplets usually exceeding 0.1 $\mu$m in diameter. The emulsions of the present invention may contain excipients such as emulsifiers, stabilizers, dyes, fats, oils, waxes, fatty acids, fatty alcohols, fatty esters, humectants, hydrophilic colloids, preservatives, and anti-oxidants may also be present in emulsions as needed. These excipients may be present as a solution in either the aqueous phase, oily phase or itself as a separate phase. Suitable examples for emulsifiers and preservatives are given in US Patent Application No. 20040014956. A microemulsion is a system of water, oil and amphiphile which is a single optically isotropic and thermodynamically stable liquid solution. Typically microemulsions are prepared by first dispersing an oil in an aqueous surfactant solution and then adding a sufficient amount of a 4th component, generally an intermediate chain-length alcohol to form a transparent system. Suitable examples for surfanctants and cosurfactants are described in US Patent Application No. 20040014956. Microemulsions are particularly of interest from the standpoint of drug solubilization and the enhanced absorption of drugs. Lipid based microemulsions (both oil/water and water/oil) have been proposed to enhance the oral bioavailability of drugs. It is expected that the microemulsion compositions and formulations of the present invention will facilitate the increased systemic absorption of pharmaceutical agents of the invention from the gastrointestinal tract, as well as improve the local cellular uptake of oligonucleotides within the gastrointestinal tract, vagina, buccal cavity and other areas of administration.

In an embodiment, the present invention employs various penetration enhancers to effect the efficient delivery of the pharamaceutical agents of the invention (especially nucleic acids, particularly oligonucleotides) to the skin of humans and animals. Suitable penetration enhancer are described in US Patent Applicatipon NO. 20040146902, herein incorporated by reference.

The useful dosage to be administered and the particular mode of administration will vary depending upon such factors as the cell type, or for in vivo use, the age, weight and the particular animal and region thereof to be treated, the particular oligonucleotide and delivery method used, the therapeutic or diagnostic use contemplated, and the form of the formulation, for example, suspension, emulsion, micelle or liposome, as will be readily apparent to those skilled in the art. Typically, dosage is administered at lower levels and increased until the desired effect is achieved. The dosage of the pharmaceutical agent may be adjusted to optimally reduce expression from the target gene, e.g., as measured by a readout of RNA stability or by a therapeutic response, without undue experimentation. The exact dosage of an oligonucleotide and number of doses administered will depend upon the data generated experimentally and in clinical trials. Several factors such as the desired effect, the delivery vehicle, disease indication, and the route of administration, will affect the dosage. Dosages can be readily determined by one of ordinary skill in the art and formulated into the subject pharmaceutical compositions. Preferably, the duration of treatment will extend at least through the course of the disease symptoms. For example, the compositions of the present invention, when used as pharmaceutical compositions, can comprise about 1 ng to about 20 mgs of the pharmaceutical agent of the invention (, e.g., the synthetic RNA molecules or the delivery agents which can be DNA molecules, plasmids, viral vectors, recombinant viruses, and mixtures thereof). The compositions of the present invention in which the delivery agents are donor cells or bacterium can be delivered in dosages of between about 1 cell to about $10^7$ cells/dose. Similarly, where the delivery agent is a live recombinant virus, a suitable vector-based composition contains between $1X10^2$ pfu to $1X10^{12}$ pfu per dose.

The pharmaceutical agent of the invention may be combined with any other drug, preferably for the same medicinal indication. For example for pharmaceutical agents which have anti-cancer properties the agent may be combined with one or more chemotherapeutic agents (e.g., such as daunorubicin, idarubicin, mitomycin C, 5-fluorouracil (5-FU), methotrexate (MTX), taxol, vincristine, and cisplatin) that function by a non-antisense mechanism.

Additional suitable teachings for pharmaceutical compositions and their preparation, administration and dosing in relation to oligonucleotide compounds which may be utilized within the scope of the present invention are given in US Patent Application No. 20040146902

In one embodiment, the pharmaceutical agents of the invention (e.g., oligonucleotides) can be administered to subjects. Examples of subjects include mammals, e.g., humans and other primates; cows, pigs, horses, and farming (agricultural) animals; dogs, cats, and other domesticated pets; mice, rats, and transgenic non-human animals.

## 5.4. Biotechnological applications

The methods and compositions according to the invention can be applied advantageously in biotechnological applications and methods, including but not limited to optimization of metabolic pathways e.g., in yeasts, fungi or other eukaryotic microorganisms or cells which are used in fermentation for the production of fine chemicals such as amino acids (for example lysin or methionin), vitamins (such as vitamin B2, vitamin C, vitamin E), carotenoids, oils and fats, polyunsaturated fatty acids, biotin and the like.

Preferred vectors for expression in eukaryotes comprise pWLNE0, pSV2CAT, pOG44, pXT1 and pSG (Stratagene Inc.); pSVK3, pBPV, pMSG and pSVL (Pharmacia Biotech, Inc.). Inducible vectors which may be mentioned are pTet-tTak, pTet-Splice, pcDNA4/TO, pcDNA4/TO /LacZ, pcDNA6/TR, pcDNA4/TO/Myc-His /LacZ, pcDNA4/TO/Myc-His A, pcDNA4/TO/Myc-His B, pcDNA4/TO/Myc-His C, pVgRXR (Invitrogen, Inc.) or the pMAM series (Clontech, Inc.; GenBank Accession No.: U02443). These vectors already provide the inducible regulatory control element, for example for a chemically inducible expression of a DSBI enzyme. The nucleic acid sequence encoding a DSBI enzyme can be inserted directly into these vectors. Vectors for expression in yeast comprise for example pYES2, pYD1, pTEFI/Zeo, pYES2/GS, pPICZ,pGAPZ, pGAPZalph, pPIC9, pPIC3.5, PHIL-D2, PHIL-SI, pPIC3SK, pPIC9K and PA0815 (Invitrogen, Inc.). In principle, for the transformation of animal cell or of yeast cells, similar methods as the "direct" transformation of plant cells are to be applied. In particular, methods such as the calcium-phosphate- or liposome-mediated transformation or else electroporation are preferred. Selection markers which can be used are, in principle, many of the selection systems which are also preferred for plants. Especially preferred are for mammalian cell the neomycin (G418) resistance, the hygromycin resistance, the zeocin resistance or the puromycin resistance. The ampicillin resistance, the kanamycin resistance or the tetracycline resistant are especially preferred for prokaryotes.

Depending on the host organism, the organisms used in the method are grown or cultured in a manner with which the skilled worker is familiar. As a rule, microorganisms are grown in a liquid medium comprising a carbon source, usually in the form of sugars, a nitrogen source, usually in the form of organic nitrogen sources such as yeast extracts or salts such as ammonium sulfate, trace elements such as salts of iron, manganese and magnesium, and, if appropriate, vitamins, at temperatures of between 0°C and 100°C, preferably between 10°C to 60°C, while passing in oxygen. The pH of the liquid medium can be kept at a constant value, that is to say regulated during the culturing period, or else not. The culture can be batchwise, semibatchwise or continuous. Nutrients can be provided at the beginning of the fermentation or fed in semicontinuously or continuously.

## 5.5. Applications in screening, target validation and drug discovery

The compositions and methods of the present invention to inhibit or reduce the function of a target gene, can also be applied to a variety of other applications including research, and in vitro applications. For example, the method of this invention can be applied to research to determine the function of a selected gene or polynucleotide sequence in a cell line, or an (preferably non-human) animal, by administering to that cell in tissue culture or that animal in vivo a composition of the invention. The inhibition of the function of that target sequence permits study of its influence on the animal's biology and physiology. Similarly, application of this method can be used to make cell lines of mammalian, bacterial, yeast, fungal, insect and other origins defective in selected pathways by "silencing" a selected functional sequence, such as an enzymatic sequence, a protein expressing sequence, or regulatory sequences necessary to the expression thereof. Such manipulated cells may be employed in conventional assays or drug screening assays, etc. In an analogous method, a "knock-out" laboratory animal can be prepared by altering the dosage of administration sufficient to permanently shut off the function of a selected gene. Thus, the method of the present invention in delivering an RNA molecule with a polynucleotide sequence sufficiently homologous to the sequence selected to be "knocked out" in the laboratory animal as described above provides a simpler technique for developing "knock-out" mice and other laboratory animals useful for pharmaceutical and genetic research.

For use in screening and target validation, the compounds and compositions of the invention are used to modulate (preferably attenuate or reduce) the expression of a selected target gene or protein. The screening method comprises the steps of contacting a preferred target gene with one or more candidate polycistronic RNA molecules, and selecting for one or more candidate polycistronic RNA molecules which decrease the expression of the target gene or protein. Once it is shown that the candidate polycistronic RNA molecules are capable of decreasing the expression of a target gene, said polycistronic RNA molecules may then be employed in further investigative studies of the function of the peptide, or for use as a research, diagnostic, or therapeutic agent in accordance with the present invention.

For use in drug discovery and target validation, the polycistronic RNA molecules of the present invention are used to elucidate relationships that exist between proteins and a disease state, phenotype, or condition. These methods include detecting or modulating a target peptide comprising contacting a sample, tissue, cell, or organism with the polycistronic RNA molecules of the present invention, measuring the nucleic acid or protein level of the target and/or a related phenotypic or chemical endpoint at some time after treatment, and optionally comparing the measured value to a non-treated sample or sample treated with a further oligomeric compound of the invention. These methods can also be performed in parallel or in combination with other experiments to determine the function of unknown genes for the process of target validation or to determine the validity of a particular gene product as a target for treatment or prevention of a disease or disorder. Thus, this invention also relates to a method for validating whether a gene product is a target for drug discovery or development.

The invention also provides a method for identifying a phenotype associated with the expression of a nucleic acid of interest in a eukaryotic cell. The method of the present invention is also useful to identify and characterize gene function in an organism. In this "functional genomics" approach, the polycistronic RNA of the invention (or the dsRNA derived therefrom) is targeted to a gene of previously unknown function, and the resultant change in phenotype is observed and, optionally, quantified. This approach is useful to identify potential targets for pharmaceutics, to promote understanding normal and pathological events associated with development, to determine signaling pathways responsible for postnatal development and aging, and the like. For example, the polycistronic RNA of the invention (or the dsRNA derived therefrom) can be designed to target a partial sequence of an expressed sequence tag (EST). Functional alterations in growth, development, metabolism, disease resistance, or other biological processes would be indicative of the normal role of the EST's gene product. As another example, the polycistronic RNA of the invention (or the dsRNA derived therefrom) targeted to new genes found by genomic sequencing programs or other "data mining" of genomic data can be used to understand the physiological roles of these new genes.

The ease with which the polycistronic RNA of the invention can be introduced into an intact cell or organism containing the target gene allows the present invention to be used in high throughput screening (HTS) applications. For example, the polycistronic RNA of the invention can be produced by an amplification reaction using primers flanking the inserts of any cDNA or genomic DNA gene library derived from the target cell or organism. It will be appreciated that the methods and means described in the specification can also be applied in High Throughput Screening (HTS) methods, for the identification or confirmation of phenotypes associated with the expression of a nucleic acid sequence with hitherto unidentified function in an eukaryotic cell, particularly in a plant or animal cell. For example a large number of target genes (e.g., as many as about 1000 or more, depending on the particular format used) are assayed rapidly and concurrently. Further, many assay formats (e.g., plates or surfaces) can be processed at one time. For example, because the oligonucleotides of the invention do not need to be tested individually before incorporating them into a composition, they can be readily synthesized and large numbers of target genes can be tested at one time. For example, a large number of samples, each comprising a biological sample containing a target nucleic acid molecule (e.g., a cell) and a composition of the invention can be added to separate regions of an assay format and assays can be performed on each of the samples.

Thus, still another aspect of the present invention provides an assay for identifying nucleic acid sequences, either coding or non-coding sequences, responsible for conferring a particular phenotype in a cell, comprising

(i) constructing a variegated library of expression constructs for polycistronic RNA molecules
(ii) introducing said variegated library polycistronic RNA molecules into a culture of target cells;
(iii) identifying members of the library which confer a particular phenotype on the cell, and identifying the sequence from a cell which correspond, such as being identical.

Yet another aspect of the present invention provides a method of conducting a drug discovery business comprising:

(i) identifying, by the subject assay, a target gene which provides a phenotypically desirable response when inhibited by RNAi;
(ii) identifying agents by their ability to inhibit expression of the target gene or the activity of an expression product of the target gene;
(iii) conducting therapeutic profiling of agents identified in step (b), or further analogs thereof, for efficacy and toxicity in animals; and
(iv) formulating a pharmaceutical preparation including one or more agents identified in step (iii) as having an acceptable therapeutic profile or homologous, to the library member.

Other potential applications for the compositions and methods of the present invention are specified in United States Patent Application No. 0040147475 and United States Patent Application No. 20040086884.

## 6. Exemplification

Those skilled in the art will recognize, or be able to ascertain using no more than routine experimentation, many equivalents to the specific embodiments of the invention described herein. Such equivalents are intended to be encompassed by the following claims. The entire contents of all patents, published patent applications and other references cited herein are hereby expressly incorporated herein in their entireties by reference.

The invention, now being generally described, will be more readily understood by reference to the following examples, which are included merely for purposes of illustration of certain aspects and embodiments of the present invention and are not intended to limit the invention.

## EXAMPLES

### General methods:

Unless otherwise specified, all chemicals are obtained from Fluka (Buchs), Merck (Darmstadt), Roth (Karlsruhe), Serva (Heidelberg) and Sigma (Deisenhofen). Restriction enzymes, DNA-modifying enzymes and molecular biology kits were from Amersham-Pharmacia (Freiburg), Biometra (Gottingen), Roche (Mannheim), New England Biolabs (Schwalbach), Novagen (Madison, Wisconsin, USA), Perkin-Elmer (Weiter-stadt), Qiagen (Hilden), Stratagen (Amsterdam, Netherlands), Invitrogen (Karlsruhe) and Ambion (Cambridgeshire, United Kingdom). The reagents used were employed in accordance with the manufacturer's instructions.

The practice of the present invention will employ, unless otherwise indicated, conventional techniques of cell biology, cell culture, molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, J. *et al.* (Cold Spring Harbor Laboratory Press (1989)); Short Protocols in Molecular Biology, 3rd Ed., ed. by Ausubel, F. *et al.* (Wiley, N.Y. (1995)); DNA Cloning, Volumes I and II (D. N. Glover ed., 1985); Oligonucleotide Synthesis (M. J. Gait ed. (1984)); Mullis *et al.* U.S. Pat. No: 4,683,195; Nucleic Acid Hybridization (B. D. Hames & S. J. Higgins eds. (1984)); the treatise, Methods In Enzymology (Academic Press, Inc., N.Y.); Immunochemical Methods In Cell And Molecular Biology (Mayer and Walker, eds., Academic Press, London (1987)); Handbook Of Experimental Immunology, Volumes I-IV (D. M. Weir and C. C. Blackwell, eds. (1986)); and Miller, J. Experiments in Molecular Genetics (Cold Spring Harbor Press, Cold Spring Harbor, N.Y. (1972)).

The chemical synthesis of oligonucleotides can be carried out for example in the known manner using the phosphoamidite method (Voet, Voet, 2nd edition, Wiley Press New York, pages 896-897). The cloning steps carried out for the purpose of the present invention such as, for example, restriction cleavages, agarose gel electrophoresis, purification of DNA fragments, transfer of nucleic acids to nitrocellulose and nylon membranes, linking DNA fragments, transformation of E. coli cells, bacterial cultures, propagation of phages and sequence analysis of recombinant DNA, are carried out as described in Sambrook *et al.* (1989) Cold Spring Harbor Laboratory Press; ISBN 0-87969-309-6. Recombinant DNA molecules are sequenced using an ABI laser fluorescence DNA sequencer by the method of Sanger (Sanger *et al.* (1977) Proc Natl Acad

Sci USA 74:5463-5467).

### Example 1: General methods

The plant Arabidopsis thaliana represents a member of the higher plants (spermatophytes). This plant is closely related to other plant species from the Cruciferea family such as, for example, Brassica napus, but also with other plant families of the dicots. Owing to the high degree of homology of its DNA sequences, or polypeptide sequences, Arabidopsis thaliana can be employed as model plant for other plant species.

**a) Culture of Arabidopsis plants**

The plants are grown either on Murashige-Skoog medium supplemented with 0.5% sucrose (Ogas *et al.* (1997) Science 277:91-94) or on compost (Focks & Benning (1998) Plant Physiol 118:91-101). To obtain uniform germination and flowering conditions, the seeds are first planted out or sprinkled on compost and then stratified for two days at 4°C. After flowering, the pods are labeled. Then, the pods are harvested according to the labels, at an age of 6 to 20 days after flowering.

**b) Isolation of total RNA and poly-A$^+$ RNA from plants**

RNA, or polyA$^+$ RNA is isolated for the generation of suppression constructs. RNA was isolated from pods of Arabidopsis plants using the following protocol: pod material aged 6 to 20 days after flowering was harvested and shock-frozen in liquid nitrogen. Prior to further use, the material was stored at -80°C. 75 mg of the material was ground to a fine powder in a cooled mortar and admixed with 200 μl of the lysis buffer from the Ambion RNAqueos kit. The total RNA was then isolated following the manufacturer's instructions. The RNA was eluted with 50 μl of elution buffer (Ambion) and the concentration was determined by absorption of a 1 : 100 dilution in a photometer (Eppendorf) at 260 nm. 40 μg/ml RNA corresponds to an absorption of 1. The RNA solutions were brought to a concentration of 1 μg/μl using RNase-free water. The concentrations were verified by agarose gel electrophoresis. To isolate polyA+ RNA, oligo(dT) cellulose from Amersham Pharmacia was used in accordance with the manufacturer's instructions. RNA or polyA+ RNA was stored at -70°C.

**c) Construction of the cDNA library**

To construct the cDNA library from Arabidopsis pod RNA, the first-strand synthesis was obtained using reverse transcriptase from murine leukemia virus (Clontech) and oligo-(dT) primers, while the second-strand synthesis was achieved by incubation with DNA polymerase I, Klenow enzyme and cleavage with RNase H at 12°C (2 hours), 16°C (1 hour) and 22°C (1 hour). The reaction was stopped by incubation at 65°C (10 minutes) and subsequently transferred to ice. Double-stranded DNA molecules were made blunt-ended with T4 DNA polymerase (Roche, Mannheim) at 37°C (30 minutes). The nucleotides were removed by phenol/chloroform extraction and by means of Sephadex G50 centrifugation columns. EcoRI/XhoI adapters (Pharmacia, Freiburg, Germany) were ligated onto the cDNA ends by means of T4 DNA ligase (Roche, 12°C, overnight), recut with XhoI and phosphorylated by incubation with polynucleotide kinase (Roche, 37°C, 30 minutes). This mixture was subjected to separation on a low-melting agarose gel. DNA molecules over 300 base pairs were eluted from the gel, phenol-extracted, concentrated on Elutip D columns (Schleicher & Schüll, Dassel, Germany), ligated onto vector arms and packaged into lambda-ZAPII phages or lambda-ZAP express phages, using the Gigapack Gold kit (Stratagene, Amsterdam, Netherlands), using the manufacturer's material and instructions.

**d) Isolation of genomic DNA from plants**

To isolate genomic DNA from plants such as *Arabidopsis thaliana*, approximately 0.25 g of leaf material of young plants in the vegetative stage are comminuted in liquid nitrogen in a pestle and mortar to give a fine powder. The pulverized plant material, together with 1 ml of CTAB I buffer (CTAB: Hexadecyltrimethylammonium bromide, also referred to as Cetyltrimethylammonium bromide; Sigma Cat. No.: H6269) at a temperature of 65°C and 20 μl of β-mercaptoethanol, is placed into a prewarmed second mortar and, after complete homogenization, the extract is transferred into a 2 ml Eppendorf vessel and incubated for 1 hour at 65°C with regular careful mixing. After the mixture has cooled to room temperature, it is extracted in 1 ml of chloroform/octanol (24:1, extracted by shaking with 1M Tris/HCl, pH 8.0) by slowly inverting, and centrifuged for 5 minutes at 8,500 rpm (7,500 x g) and room temperature to achieve phase separation. Thereafter, the aqueous phase is reextracted with 1 ml of chloroform/octanol, centrifuged and mixed carefully by inverting with 1/10 volume of CTAB II buffer which has been prewarmed to 65°C. Thereafter, the mixture is admixed with 1 ml chloroform/octanol mixture (see above) by careful rocking and centrifuged for 5 minutes at 8,500 rpm (7,500 x g) and room temperature to reseparate the phases. The aqueous lower phase is transferred into a fresh Eppendorf vessel and the upper organic phase is recentrifuged in a fresh Eppendorf vessel for 15 minutes at 8,500 rpm (7,500 x g) and room temperature. The resulting aqueous phase is combined with the aqueous phase of the previous centrifugation step, and all of the mixture is admixed with precisely the same volume of pre-warmed CTAB III buffer. This is followed by incubation at 65°C until the DNA precipitates in the form of floccules. This may take up to 1 hour or can be carried out overnight by incubation at 37°C. The sediment resulting from the subsequent centrifugation step (5 min, 2000 rpm (500 x g), 4°C) is admixed with 250 μl CTAB IV buffer which has been prewarmed to 65°C and incubated at 65°C for at least 30 minutes or until all of the sediment has dissolved. To precipitate the DNA, the solution is subsequently mixed with 2.5 volumes of ice-cold ethanol and incubated for 1 hour at -20°C. Alternatively, the mixture can be mixed with 0.6 volume of isopropanol and centrifuged immediately for 15 minutes at 8,500 rpm (7,500 x g) and 4°C, without further incubation. The sedimented DNA is washed by inverting the Eppendorf vessel twice with in each case 1 ml of 80% strength ice-cold ethanol, recentrifuged after each washing step (5 min, 8,500 rpm (7,500 x g), 4°C) and subsequently dried in the air for approximately 15 minutes. The DNA is subsequently resuspended in 100 μl of TE with 100 μg/ml RNase and incubated for 30 minutes at room temperature. After a further incubation phase overnight at 4°C, the DNA solution is homogeneous and can be used for subsequent experiments.

Solutions for CTAB:

Solution I (for 200 ml):
100 mM Tris/HCl pH 8.0 (2.42 g)
1.4 M NaCl (16.36 g)
20 mM EDTA (8.0 ml of 0.5 M stock solution)
2% (w/v) CTAB (4.0 g)
In each case the following is added freshly prior to use: 2% β-mercaptoethanol (20 μl for 1 ml of solution I).

Solution II (for 200 ml):
0.7 M NaCl (8.18 g)
10% (w/v) CTAB (20 g)

Solution III (for 200 ml):
50 mM Tris/HCl pH 8.0 (1.21 g)
10 mM EDTA (4 ml 0.5 M of 0.5 M stock solution)
1% (w/v) CTAB (2.0 g)

Solution IV (high-salt TE) (for 200 ml):
10 mM Tris/HCl pH 8.0 (0.242 g)
0.1 mM EDTA (40 μl of 0.5 M stock solution)
1 M NaCl (11.69 g)

Chloroform/octanol (24:1) (for 200 ml):
192 ml chloroform
8 ml octanol
The mixture is extracted twice by shaking with 1 M Tris-HCl, pH 8.0 and stored away from light.

**Example 2: Generation of suppression constructs**

As base vector the vector 1 bxPcUbiLic (SEQ ID NO: 17) has been chosen.

As tRNA an *A. thaliana* tRNA for glutamine with the anticodon UUG was chosen, as snoRNA cluster 9, a cluster comprising the genes z43, snR74 (z4) and snR60 (z15) was chosen. For cloning the Gln-tRNA and snoRNA cluster 9 in the respective vectors PCR primers were designed and PCR performed on genomic DNA of A. thaliana C24. The primer sequences were as follows, restriction enzyme recognition sequences SmaI, Asp718 and SalI were added to the forward primers, whereas restriction enzyme recognition sequences SacI, BsrGI and XhoI were added to the reverse primers.

For amplification of the sequence encoding $tRNA^{Gln}$ (SEQ ID NO: 3)
Oligonucleotide primer tRNAgln1fwSmaAspSal (SEQ ID NO: 9):
atacccgggtaccgtcgacagacaaagaacactgcaatc
Oligonucleotide primer tRNAgln1revSacBsrGXho (SEQ ID NO: 10):
atagagctctgtacactcgaggcatgagaggtcctacc

For amplification of the sequence encoding snoRNA cluster 9 (SEQ ID NO: 1)
Oligonucleotide primer sno9 fwSmaAspSal (SEQ ID NO: 11):
atacccgggtaccgtcgacttcatcttcttcttctgg
Oligonucleotide primer sno9 revSacBsrGXho (SEQ ID NO:12):
atagagctctgtacactcgagacttaaggaacaatttga

The resulting PCR fragments of the pre-tRNA (SEQ ID NO: 3) or the snoRNA cluster (SEQ ID NO: 1), respectively, were cut with the restriction enzymes SmaI/SacI (MBI Fermentas, St. Leon-Rot) and cloned into the vector 1 bxPcUbiLic (SEQ ID NO: 17) that has also been digested with SmaI/SacI. This yielded the vectors 1bxPcUbitRNA (SEQ ID NO: 19) and 1 bxPcUbisnoRNA (SEQ ID NO: 18) respectively.

The genes, which expression should be silenced to demonstrate efficiency of the system of the invention, were two genes involved in brassinosteroid synthesis and sensing, respectively. The down regulation of these genes leads to a cabbage phenotype, which can easily be screened. To enable cloning in the respective vectors a Asp718 restriction enzyme recognition sequence was added to the forward primers, whereas a SalI restriction enzyme recognition sequence was added to the reverse primers.

| | | |
|---|---|---|
| 4g39400Aspfw | (SEQ ID NO: 13): | 5'-ataggtaccGAGGTAACATGGCATGTG-3' |
| 4g39400Salrev | (SEQ ID NO: 14): | 5'-atagtcgacAGCTGAGATCAATCGAAG-3' |
| 4g18710Aspfw | (SEQ ID NO: 15): | 5'-ataggtaccAAGGGTGTTTACGTAATTG-3' |
| 4g18710Salrev | (SEQ ID NO: 16): | 5'-atagtcgacACGCATCAACTGAAGTTC-3' |

PCR was performed on pods cDNA of pod material aged 6 to 20 days after flowering of MC24 A. thaliana plants, resulting in a fragment from gene At4g39400 (SEQ ID NO: 7) and a fragment from gene at4g18710 (SEQ ID NO: 8).

The respective PCR fragments were cut with Asp718/SalI and ligated into the respective vectors that were digested with Asp718/SalI. A second portion of the Asp718/SalI digested PCR fragments were ligated into the vectors that came from that ligation and that were cut before with BsrGI/XhoI. This results in an inverted orientation of two copies of the same fragment in one vector. The resulting vectors were called 1 bxPcUbitRNAds4g39400, 1bxPcUbitRNAds4g18710, 1bxPcUbisnoRNAds4g39400 and 1 bxPcUbisnoRNAds4g 18710 respectively.

**Example 3: Plant transformation**

The *Agrobacterium-mediated* transformation of plants can be carried out using standard transformation and regeneration techniques (Gelvin SB, Schilperoort RA, Plant Molecular Biology Manual, 2nd ed., Dordrecht: Kluwer Academic Publ., 1995, in Sect., Ringbuc Zentrale Signatur: BT11-P ISBN 0-7923-2731-4; Glick BR, Thompson JE, Methods in Plant Molecular Biology and Biotechnology, Boca Raton: CRC Press, 1993, 360 pp., ISBN 0-8493-5164-2; Deblaere *et al.* (1984) Nucl Acids Res 13:4777-4788).). Agrobacterium tumefaciens strains GV3101 (pMP90) (Koncz and Schell (1986) Mol Gen Genet 204: 383- 396) or LBA4404 (Clontech) can be used. The transformation can be carried out by standard transformation techniques.

The transformation of Arabidopsis thaliana by means of Agrobacterium is carried out by the method of Bechthold *et al.*, 1993 (C.R. Acad. Sci. Ser. III Sci. Vie., 316, 1194-1199). Oil-seed rape can be transformed for example by cotyledon or hypocotyl transformation (Moloney *et al.*, Plant Cell Report 8 (1989) 238-242; De Block *et al.*, Plant Physiol. 91 (1989) 694-701). The use of antibiotics for selection of Agrobacterium and plants depends on the binary vector used for the transformation and the Agrobacterium strain. The selection of oilseed rape is usually carried out using kanamycin as selectable plant marker. Agrobacterium-mediated gene transfer into linseed (Linum usitatissimum) can be carried out using, for example, a technique described by Mlynarova *et al.* (1994) Plant Cell Report 13:282-285. The transformation of soybean can be carried out using, for example, a technique described in EP-A-0 0424 047 (Pioneer Hi-Bred International) or in EP-A-0 0397 687, US 5,376,543, US 5,169,770 (University Toledo).

The transformation of plants using particle bombardment, polyethylene-glycol-mediated DNA uptake or via the silicon carbonate fiber technique is described for example by Freeling and Walbot "The maize handbook" (1993) ISBN 3-540-97826-7, Springer Verlag New York).

**Example 4: Expression analysis**

Analysis can be performed on RNA-level (e.g., by Northern blot analysis or real time qPCR) or - in case of a translated gene - on protein level (e.g., by Western blot analysis).

**4.1 Northern hybridization:**

A suitable method for determining the amount of transcription of a gene is to carry out a Northern blot analysis (by way of reference, see Ausubel *et al.* (1988) Current Protocols in Molecular Biology, Wiley: New York, or the abovementioned example section), where a primer which is designed in such a way that it binds to the gene of interest is labeled with a detectable label (usually a radioactive label or chemiluminescent label) so that, when the total RNA of a culture of the organism is extracted, separated on a gel, transferred to a stable matrix and incubated with this probe, the binding and the extent of the binding of the probe indicate the presence and also the amount of the mRNA for this gene. This information also indicates the degree of transcription of the transformed gene. Cellular total RNA can be prepared from cells, tissues or organs in a plurality of methods, all of which are known in the art, such as, for example, the method described by Bormann, E.R., *et al.* (1992) Mol. Microbiol. 6:317-326.

To carry out the RNA hybridization, 20 $\mu$g of total RNA or 1 $\mu$g of poly(A)$^+$ RNA are separated by means of gel electrophoresis in agarose gels with a strength of 1.25% using formaldehyde, as described in Amasino (1986, Anal. Biochem. 152, 304), capillary-blotted to positively charged nylon membranes (Hybond N+, Amersham, Braunschweig) using 10 x SSC, immobilized by means of UV light and prehybridized for 3 hours at 68°C using hybridization buffer (10% dextran sulfate w/v, 1 M NaCl, 1% SDS, 100 mg herring sperm DNA). The DNA probe was labeled with the Highprime DNA labeling kit (Roche, Mannheim, Germany) during the prehybridization, using alpha-$^{32}$P-dCTP (Amersham Pharmacia, Braunschweig, Germany). After the labeled DNA probe had been added, the hybridization was carried out in the same buffer at 68°C overnight. The washing steps were carried out twice for 15 minutes using 2 X SSC and twice for 30 minutes using 1 X SSC, 1% SDS, at 68°C. The sealed filters were exposed at -70°C over a period of 1 to 14 days.

**4.2 Western blot analysis**

Standard techniques, such as a Western blot, can be employed for assaying the presence or the relative amount of protein translated by this mRNA (see, for example, Ausubel *et al.* (1988) Current Protocols in Molecular Biology, Wiley: New York). In this method, the cellular total proteins are extracted, separated by means of gel electrophoresis, transferred to a matrix such as nitrocellulose, and incubated with a probe, such as an antibody, which binds specifically to the desired protein. This probe is usually provided with a chemiluminescent or colorimetric label, which can be detected readily. The presence and the amount of the label observed indicates the presence and the amount of the desired mutated protein, which is present in the cell.

**Example 5: Analysis of the effect of the expression of the polycistronic RNA on the production of the target product**

As an example, the expression of two genes involved in brassinosteroid synthesis or sensing were chosen for analyzing the efficiency of the invention. The genes chosen were Arabidopsis thaliana gene at4g39400 (coding for Arabidopsis thaliana brassinosteroid insensitive 1 (BRI1) mRNA; GenBank Acc.-No.: NM_120100)) and at4g18710 (coding for Arabidopsis thaliana shaggy-related protein kinase eta / ASK-eta (ASK7) mRNA; GenBank Acc.-No.: NM_ 117987) respectively. Repression of these genes leads to the visible cabbage phenotype (Li,J. and Chory,J. A putative leucine-rich repeat receptor kinase involved in brassinosteroid signal transduction Cell 90 (5), 929-938 (1997); Choe, S., Schmitz,R.J., Fujioka,S., Takatsuto,S., Lee,M.O., Yoshida,S., Feldmann,K.A. and Tax,F.E.Arabidopsis Brassinosteroid-Insensitive dwarf12 Mutants Are Semidominant and Defective in a Glycogen Synthase Kinase 3beta-Like Kinase Plant Physiol. 130 (3), 1506-1515 (2002)), which can easily be visually screened in transgenic T1 plants. As a control, a construct was designed that comprised a random DNA spacer between the inverted repeat of the target gene fragments instead of the tRNA or snoRNA cluster 9 spacer, respectively.

In order to show increased efficiency of gene repression by using tRNA or snoRNA clusters as spacers between inverted repeats, the percentage of transgenic T1 plants exhibiting cabbage phenotype in populations transformed either with constructs comprising random DNA, tRNA or snoRNA cluster 9 spacer are determined. In addition, quantitative RT PCR (qRT PCR) was performed on total RNA of WT *A.thaliana* MC24 and transgenic T1 plants derived from the transformation with the vectors 1 bxPcUbitRNAds4g39400, 1 bxPcUbitRNAds4g18710, 1 bxPcUbisnoRNAds4g39400 and 1bxPcUbisnoRNAds4g18710 respectively. For this purpose sequence specific primers for the suppressed genes are employed (see above Example 2; SEQ ID NO 13 and 14, or SEQ ID NO: 15 and 16, respectively) and qRT PCR was performed as described in Gibson et al (1996) Genome Research 6 995 - 1001; and Kruse et al. (1997) Journal of Immunological Methods 210 195 - 203.

**Claims**

1. A polycistronic RNA molecule capable to provide in an eukaryotic cell an at least partially double-stranded RNA molecule, said polycistronic RNA molecule comprising

   a) at least one first ribonucleotide sequence that is substantially identical to at least a part of a target nucleotide sequence of at least one target gene, and
   b) at least one second ribonucleotide sequence which is substantially complementary to said first nucleotide sequence and is capable to hybridize to said first nucleotide sequence to form a double-stranded RNA structure, and
   c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

2. The polycistronic RNA molecule of claim 1, wherein said first and said second ribonucleotide sequence are arranged in an antiparallel structure capable to form in an eukaryotic cell a hairpin structure.

3. The polycistronic RNA molecule of claim 1 or 2, wherein said third ribonucleotide sequence comprises a sequence, which is at least 60% identical to at least one transcribed RNA selected from the group consisting of tRNAs, rRNAs and snoRNAs and their precursor molecules.

4. The polycistronic RNA molecule of any of claim 1 to 3, wherein said third ribonucleotide sequence comprises at least one sequence encoded by a sequence selected from the group of

   a) the sequences as described by SEQ ID NO: 1, 2, 3, 4, 5, and 6, and
   b) a sequence having an identity of at least 60% to a sequence as described by SEQ ID NO: 1, 2, 3, 4, 5, or 6 and comprising the functional elements necessary for RNA processing, and
   c) a sequences capable to hybridize with a sequence as described by SEQ ID NO: 1, 2, 3, 4, 5, or 6 or the complement thereof and comprising the functional elements necessary for RNA processing.

5. The polycistronic RNA molecule of any of claim 1 to 4, wherein said first ribonucleotide sequence hybridizes under stringent conditions to at least one sequence of the target gene.

6. The polycistronic RNA molecule of any of claim 1 to 5, wherein said target gene is selected from the group consisting of eukaryotic endogenous genes, genes of pathogens and transgenes.

7. The polycistronic RNA molecule of claim 6, wherein said gene of a pathogen is from a pathogen capable to infect an eukaryotic organism and is from a pathogen selected from the group of virus, bacteria, fungi and nematodes.

8. The polycistronic RNA molecule of any of claim 1 to 7, wherein said first ribonucleotide sequence of said polycistronic RNA has a length of at least 19 nucleotides.

9. The polycistronic RNA molecule of any of claim 1 to 8, wherein the first nucleotide sequence of said polycistronic RNA has a size of about 20 to 500 nucleotides.

10. The polycistronic RNA molecule of any of claim 1 to 9, wherein the first nucleotide sequence of polycistronic RNA is at least 65% identical to at least one mammalian, nematode, fungal or plant gene.

11. The polycistronic RNA molecule of any of claim 1 to 10, wherein the first nucleotide sequence is substantially identical to a nucleotide sequence corresponding to the coding sequence or the non-coding of at least one mammalian or plant gene.

12. The polycistronic RNA molecule of claim 11, wherein the non-coding sequence is a non-transcribed sequence.

13. The polycistronic RNA molecule of any of claims 1 to 12, which after removal of said third ribonucleotide sequence generates a short interfering double-stranded RNA which does not produce a significant PKR-dependent response in the host cells at concentrations effective for attenuating expression of the target gene.

**14.** The polycistronic RNA molecule of claim 13, wherein said generated short-interfering double-stranded RNA is 15 to 45 basepairs in length.

**15.** A composition for attenuating expression of a target gene, comprising at least one polycistronic RNA of any of claim 1 to 14.

**16.** A pharmaceutically preparation of at least one polycistronic RNA of any of claim 1 to 14 that gives rise to a short interfering double stranded RNA (siRNA) in a mammalian cell and attenuates expression of at least one target gene, which polycistronic RNA does not produce a significant PKR-dependent response in the mammalian cell at concentrations effective for attenuating expression of the target gene.

**17.** An expression construct comprising

a) a promoter functional in eukaryotic cells, and
b) a nucleic acid sequence encoding a polycistronic RNA molecule of any of claim 1 to 14,

wherein said promoter a) is operably linked and heterologous to said nucleic acid sequence b).

**18.** The expression construct of claim 17, which is DNA.

**19.** The expression construct of claim 17 or 18, which is in a plasmid.

**20.** An expression vector comprising an expression construct of any of claim 17 to 20.

**21.** The expression vector of claim 20, wherein the expression vector is an eukaryotic expression vector.

**22.** The expression vector of claim 20, wherein the eukaryotic expression vector is a viral vector.

**23.** A transformed cell or non-human organism comprising an expression construct of any of claim 17 to 19 or an expression vector of any of claim 20 to 22.

**24.** The transformed cell or non-human organism of claim 23 comprising said expression construct or expression vector inserted into its genome.

**25.** The transformed cell or non-human organism of claim 23 or 24, wherein said cell or organism is selected from the group of mammalian, bacterial, fungal, nematode or plant cells and organism.

**26.** A method for attenuating expression of at least one target gene in an eukaryotic cell, comprising introducing a polycistronic RNA molecule of any of claim 1 to 14 into the cells in an amount sufficient to attenuate expression of the target gene,
wherein the polycistronic RNA molecule comprises at least one ribonucleotide sequence that is substantially identical to at least a part of the nucleotide sequence of the target gene.

**27.** The method of claim 26, wherein the target gene is an endogenous gene of the cell or a heterologous gene relative to the genome of the cell, such as a pathogen gene.

**28.** The method of claim 26 or 27, wherein the heterologous gene is a transgene or viral, nematode or fungal gene.

**29.** The method of any of claims 26 to 28, wherein the polycistronic RNA is produced outside the cell.

**30.** The method of any of claims 26 to 28, wherein the polycistronic RNA is recombinantly produced by an expression construct or expression vector within the cell.

**31.** The method of any of claims 26 or 30, wherein the cell is a nematode, mammalian cell or a plant cell.

**32.** The method of any of claims 26 to 31, wherein the identity of said first ribonucleic acid sequence to the target gene is at least about 65%.

**33.** The method of any of claims 26 to 32, wherein the polycistronic RNA is processed to a short-interfering double-stranded RNA molecule by said host cells.

**34.** The method of any of claims 26 to 33, in which the target gene expression is attenuated by at least about 10%.

**35.** A method for generating short interfering double-stranded RNA, comprising:

(i) providing an in vitro transcription system including an expression construct of any of claim 17 to 19, and
(ii) isolating said short interfering double-stranded RNA from said in vitro transcription system.

**36.** The method of claim 35, wherein the RNA polymerase is a bacteriophage RNA polymerase.

**37.** Use of the compositions of any of claims 1 to 25 in the manufacture of a medicament for attenuating expression of one or more genes in vivo.

**38.** A method for reducing the susceptibility of host cells or host organisms to infection by pathogen, comprising introducing a polycistronic RNA of any of Claim 1 to 14 into said host cells or host organisms in an amount sufficient to attenuate expression of one or more genes necessary for expression by said pathogen.

**39.** The method of claim 38, wherein the pathogen is a virus, a fungus or a nematode.

**40.** A method for obtaining pathogen resistant organisms, comprising the steps of providing cells of the organism with an polycistronic RNA molecule of any of Claim 1 to 14, said polycistronic RNA molecule capable to provide in an eukaryotic cell an at least partially double-stranded RNA molecule, said polycistronic RNA molecule comprising

a) at least one first ribonucleotide sequence that is substantially identical to at least a part of a target nucleotide sequence of at least one target gene, and
b) at least one second ribonucleotide sequence which is substantially complementary to said first nucleotide sequence and is capable to hybridize to said first nucleotide sequence to form a double-stranded RNA structure, and
c) at least one third ribonucleotide sequence located between said first and said second ribonucleotide sequence comprising at least one removable RNA element, which can be removed by the RNA processing mechanism of an eukaryotic cell without subsequently covalently joining the resulting sequences comprising said first and said second ribonucleotide sequence, respectively.

**41.** The method of claim 40, wherein said first ribonucleotide sequence has between 75 and 100% sequence identity with at least part of the nucleotide sequence of the genome of a pathogen.

**42.** The method of claim 40 or 41, wherein the pathogen is selected from the group of virus, bacteria, fungi, and nematodes.

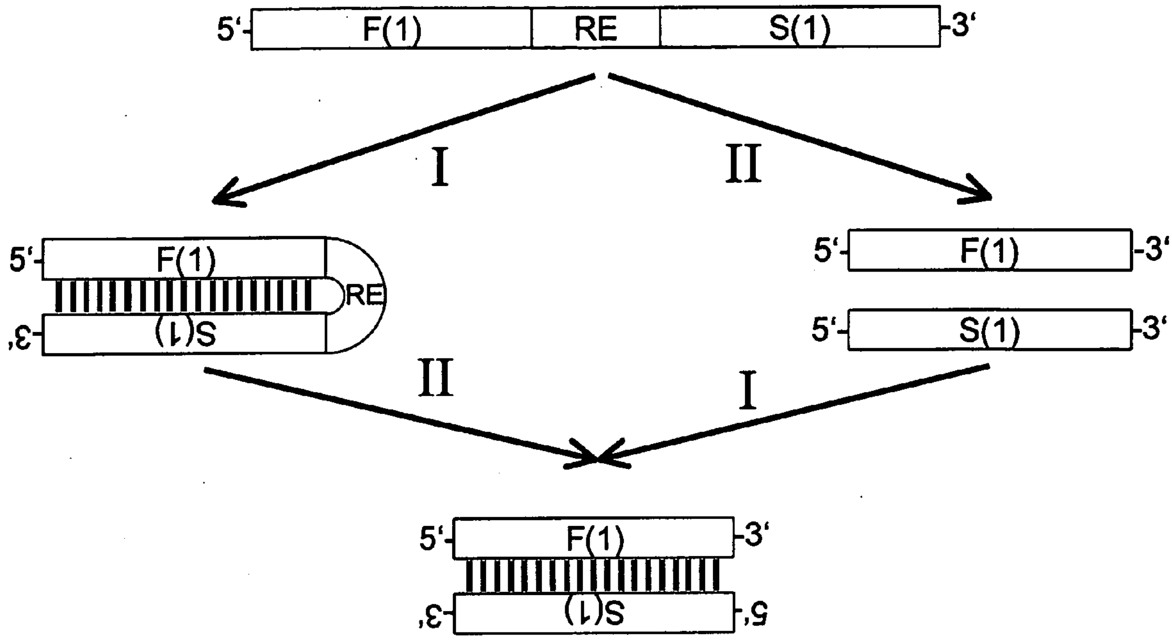


Fig. 1

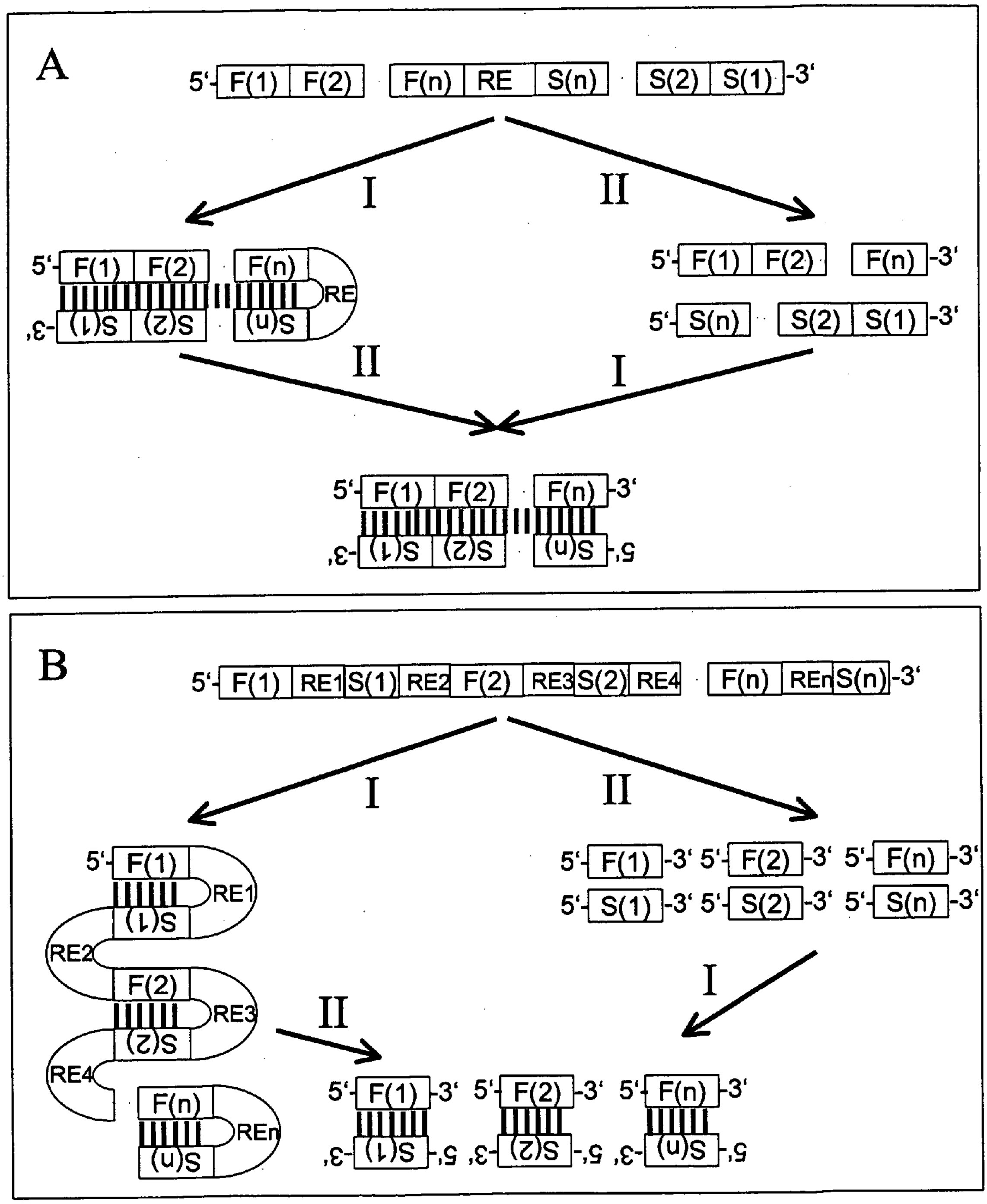
A
5'- F(1) F(2) F(n) RE S(n) S(2) S(1) -3'
I
II
5'- F(1) F(2) F(n)
RE
3'- S(1) S(2) S(n)
5'- F(1) F(2) F(n) -3'
5'- S(n) S(2) S(1) -3'
II
I
5'- F(1) F(2) F(n) -3'
3'- S(1) S(2) S(n) -5'
B
5'- F(1) RE1 S(1) RE2 F(2) RE3 S(2) RE4 F(n) REn S(n) -3'
I
II
5'- F(1) RE1 S(1) RE2 F(2) RE3 S(2) RE4 F(n) REn S(n)
5'- F(1) -3' 5'- F(2) -3' 5'- F(n) -3'
5'- S(1) -3' 5'- S(2) -3' 5'- S(n) -3'
I
II
5'- F(1) -3' 5'- F(2) -3' 5'- F(n) -3'
3'- S(1) -5' 3'- S(2) -5' 3'- S(n) -5'

Fig. 2

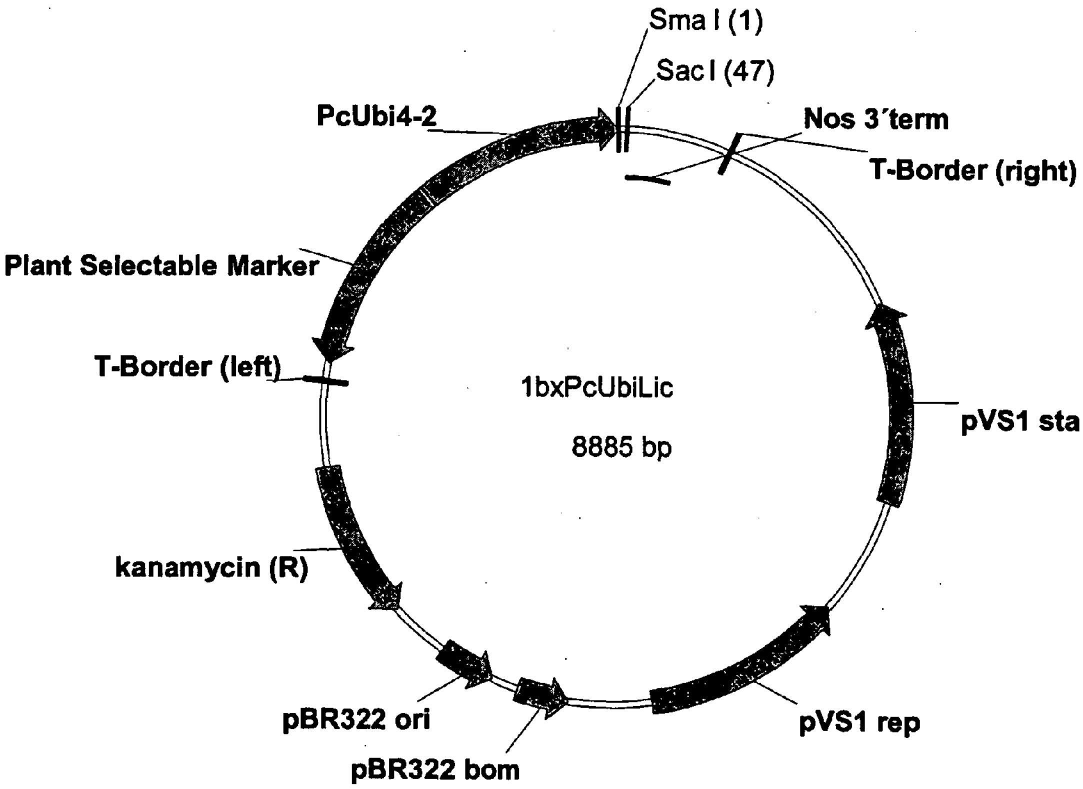
Sma I (1)
Sac I (47)
PcUbi4-2
Nos 3'term
T-Border (right)
Plant Selectable Marker
T-Border (left)
1bxPcUbiLic
8885 bp
pVS1 sta
kanamycin (R)
pBR322 ori
pBR322 bom
pVS1 rep

Fig. 3

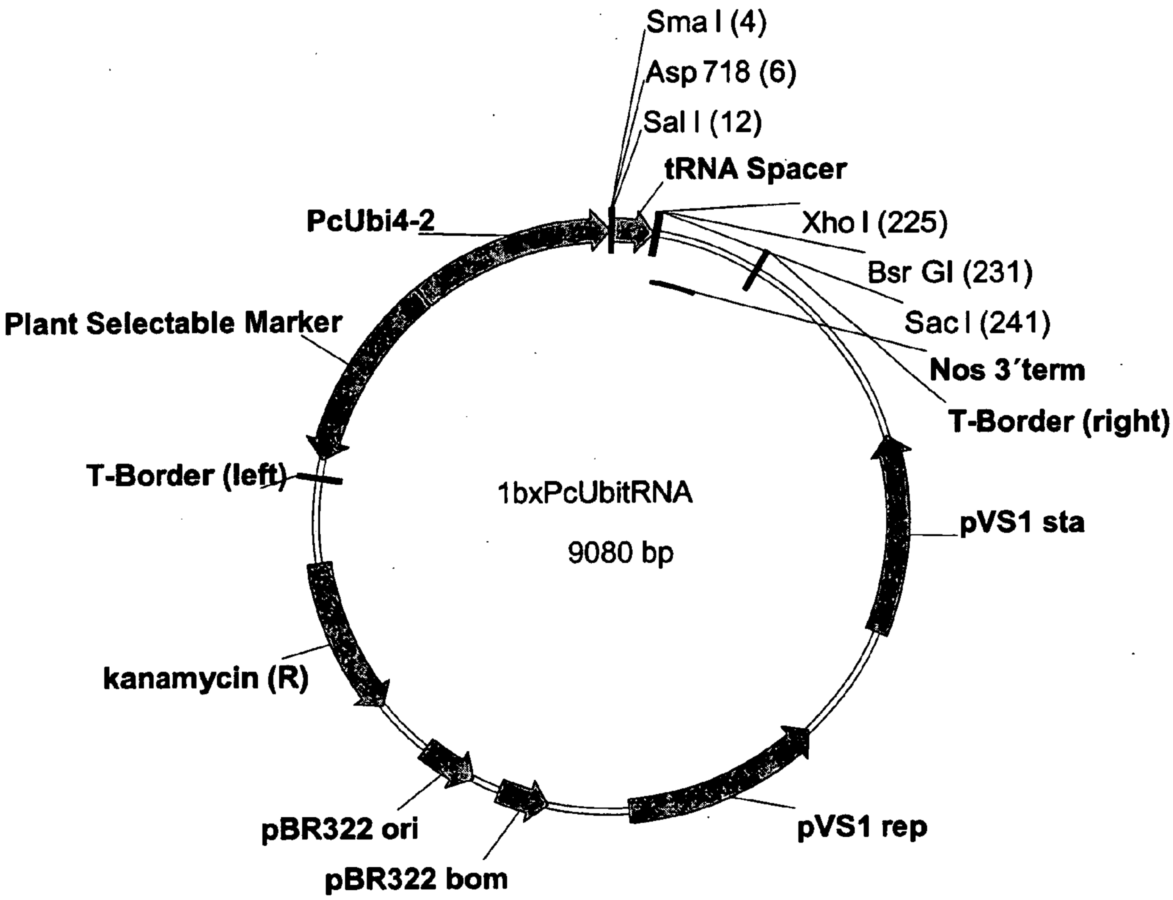
Sma I (4)
Asp 718 (6)
Sal I (12)
tRNA Spacer
Xho I (225)
Bsr GI (231)
Sac I (241)
Nos 3'term
T-Border (right)
PcUbi4-2
Plant Selectable Marker
T-Border (left)
1bxPcUbitRNA
9080 bp
pVS1 sta
kanamycin (R)
pBR322 ori
pBR322 bom
pVS1 rep

Fig. 4

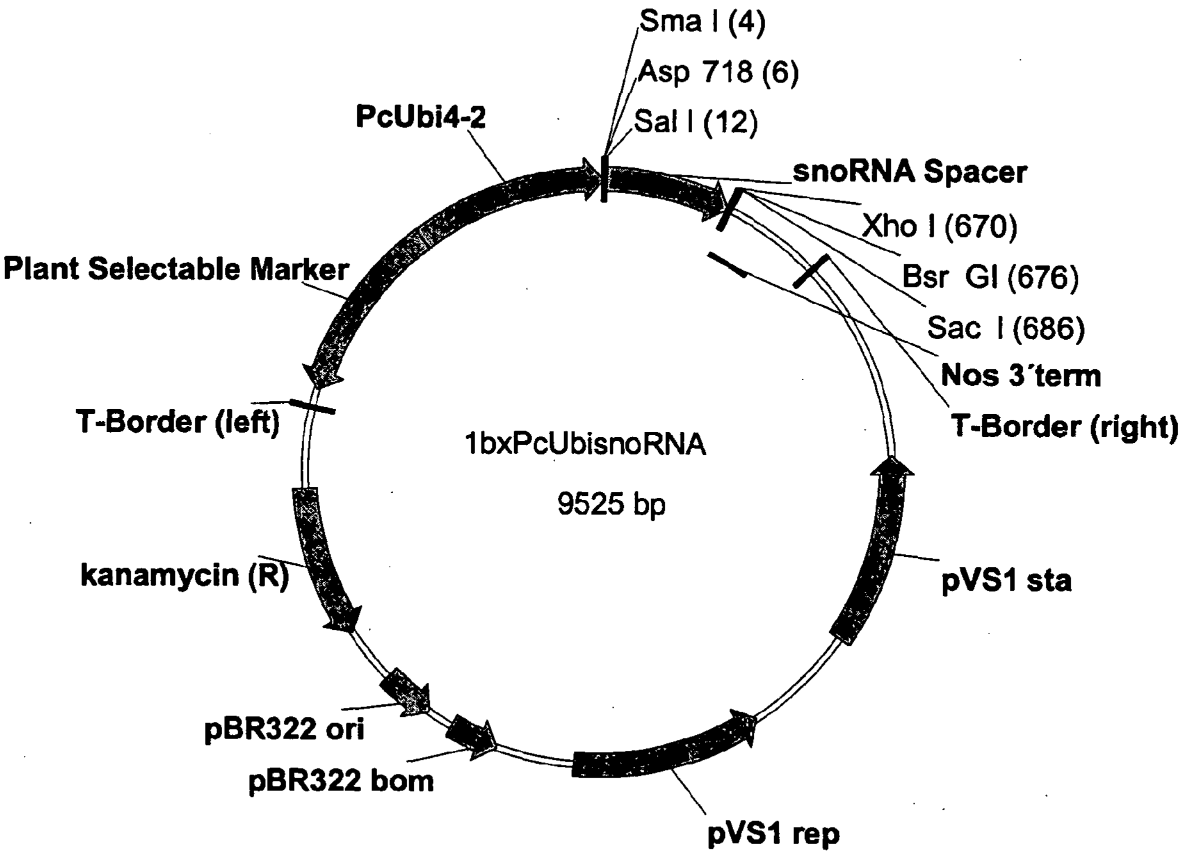
Sma I (4)
Asp 718 (6)
Sal I (12)
PcUbi4-2
snoRNA Spacer
Xho I (670)
Bsr GI (676)
Sac I (686)
Nos 3´term
T-Border (right)
Plant Selectable Marker
T-Border (left)
1bxPcUbisnoRNA
9525 bp
kanamycin (R)
pVS1 sta
pBR322 ori
pBR322 bom
pVS1 rep

Fig. 5

European Patent Office

# PARTIAL EUROPEAN SEARCH REPORT

which under Rule 45 of the European Patent Convention shall be considered, for the purposes of subsequent proceedings, as the European search report

Application Number
EP 05 00 0877

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2003/148519 A1 (ENGELKE DAVID R ET AL) 7 August 2003 (2003-08-07)<br>* page 1, paragraph 10 *<br>* page 13, paragraph 124 *<br>* page 14, paragraph 127 *<br>* page 16, paragraph 142 *<br>* pages 18-19, paragraph 164 *<br>* page 21, paragraph 194 *<br>----- | 1-42 | C12N15/11<br>A61K48/00 |
| X | EP 1 462 525 A (TOUDAI TLO, LTD) 29 September 2004 (2004-09-29)<br>* page 6, line 11 - line 12; figure 2D *<br>* page 10, line 15 - line 17 *<br>----- | 1-42 | |
| X | WO 03/056012 A (CANCER REC TECH LTD [GB]; AGAMI REUVEN [NL]; BRUMMELKAMP THIJN [NL]) 10 July 2003 (2003-07-10)<br>* page 16, line 1 - line 8; claims 7,8; figures 1,4 *<br>----- | 1,2,5-42 | |
| X | WO 03/093441 A (DUKE UNIVERSITY; CULLEN, BRYAN, R; ZENG, YANG) 13 November 2003 (2003-11-13)<br>* page 16, line 9 - line 12; figure 1A *<br>-----<br>-/-- | 1,2,5-42 | TECHNICAL FIELDS SEARCHED (Int.Cl.7)<br>C12N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 23 June 2005 | Guarinos Viñals, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 05 00 0877

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/160393 A1 (SYMONDS GEOFFREY P ET AL) 31 October 2002 (2002-10-31)<br>* page 5, paragraph 91; figure 8C *<br>----- | 1,2,5-42 | |
| X | WO 2004/035765 A (NUCLEONICS, INC; PACHUK, CHATERINE, J; SATISHCHANDRAN, C; MCCALLUS, DA) 29 April 2004 (2004-04-29)<br>* figures 2,3,8A *<br>----- | 1,2,5-42 | |
| X | US 2004/110296 A1 (VARGEESE CHANDRA ET AL) 10 June 2004 (2004-06-10)<br>* figures 36,38,40,42 *<br>----- | 1,2,5-42 | |
| X | WO 03/070918 A (RIBOZYME PHARMACEUTICALS, INCORPORATED; MCSWIGGEN, JAMES; BEIGELMAN, L) 28 August 2003 (2003-08-28)<br>* page 23, line 4 - line 9; figure 20 *<br>* page 65, line 9 - line 13 *<br>----- | 1,2,5-42 | TECHNICAL FIELDS SEARCHED (Int.Cl.7) |
| X | KAWASAKI H ET AL: "Short hairpin type of dsRNAs that are controlled by tRNA Val promoter significantly induce RNAi-mediated gene silencing in the cytoplasm of human cells"<br>NUCLEIC ACIDS RESEARCH, OXFORD UNIVERSITY PRESS, SURREY, GB,<br>vol. 31, no. 2, January 2003 (2003-01), pages 700-707, XP002965487<br>ISSN: 0305-1048<br>* the whole document *<br>----- | 1,2,5-42 | |

1

EPO FORM 1503 03.82 (P04C10)

European Patent Office

INCOMPLETE SEARCH SHEET C

Application Number EP 05 00 0877

Although claims 26-34, 38-42, as far as as an "in vivo" method is concerned, are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO.

EP 05 00 0877

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2005

| Patent document cited in search report | Publication date | Patent family member(s) | Publication date |
|---|---|---|---|
| US 2003148519 A1 | 07-08-2003 | NONE | |
| EP 1462525 A | 29-09-2004 | AU 2002354121 A1 | 10-06-2003 |
| | | CA 2468955 A1 | 05-06-2003 |
| | | EP 1462525 A1 | 29-09-2004 |
| | | JP 3642573 B2 | 27-04-2005 |
| | | US 2005048647 A1 | 03-03-2005 |
| | | AU 2002343792 A1 | 10-06-2003 |
| | | WO 03046173 A1 | 05-06-2003 |
| | | WO 03046186 A1 | 05-06-2003 |
| | | US 2004002077 A1 | 01-01-2004 |
| WO 03056012 A | 10-07-2003 | US 2003144232 A1 | 31-07-2003 |
| | | AU 2002352469 A1 | 15-07-2003 |
| | | CA 2470903 A1 | 10-07-2003 |
| | | EP 1458863 A1 | 22-09-2004 |
| | | WO 03056012 A1 | 10-07-2003 |
| | | GB 2383330 A ,B | 25-06-2003 |
| | | JP 2005512596 T | 12-05-2005 |
| | | US 2003144239 A1 | 31-07-2003 |
| WO 03093441 A | 13-11-2003 | AU 2003265978 A1 | 17-11-2003 |
| | | EP 1504126 A2 | 09-02-2005 |
| | | WO 03093441 A2 | 13-11-2003 |
| | | US 2004053411 A1 | 18-03-2004 |
| US 2002160393 A1 | 31-10-2002 | CA 2433680 A1 | 01-08-2002 |
| | | EP 1354038 A2 | 22-10-2003 |
| | | JP 2004532616 T | 28-10-2004 |
| | | WO 02059300 A2 | 01-08-2002 |
| WO 2004035765 A | 29-04-2004 | AU 2003284323 A1 | 04-05-2004 |
| | | WO 2004035765 A2 | 29-04-2004 |
| US 2004110296 A1 | 10-06-2004 | US 2004192626 A1 | 30-09-2004 |
| | | US 2005080031 A1 | 14-04-2005 |
| | | US 2005020525 A1 | 27-01-2005 |
| | | US 2004249178 A1 | 09-12-2004 |
| | | US 2005096284 A1 | 05-05-2005 |
| | | US 2005014172 A1 | 20-01-2005 |
| | | US 2005048529 A1 | 03-03-2005 |
| | | US 2005032733 A1 | 10-02-2005 |
| | | US 2005054598 A1 | 10-03-2005 |
| | | US 2005119211 A1 | 02-06-2005 |
| | | US 2005119212 A1 | 02-06-2005 |
| | | US 2005124566 A1 | 09-06-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 0877

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2005

| Patent document cited in search report | Publication date | Patent family member(s) | Publication date |
|---|---|---|---|
| US 2004110296 A1 | | US 2005124567 A1 | 09-06-2005 |
| | | US 2005124568 A1 | 09-06-2005 |
| | | US 2005124569 A1 | 09-06-2005 |
| | | US 2005070497 A1 | 31-03-2005 |
| | | US 2005079610 A1 | 14-04-2005 |
| | | WO 2004092383 A2 | 28-10-2004 |
| | | WO 2004111237 A1 | 23-12-2004 |
| | | WO 2004097020 A2 | 11-11-2004 |
| | | WO 2005041859 A2 | 12-05-2005 |
| | | CA 2447161 A1 | 28-11-2002 |
| | | JP 2005505504 T | 24-02-2005 |
| | | AU 2003221258 A1 | 09-09-2003 |
| | | CA 2459532 A1 | 28-08-2003 |
| | | EP 1458741 A2 | 22-09-2004 |
| | | GB 2397818 A ,B | 04-08-2004 |
| | | WO 03106476 A1 | 24-12-2003 |
| | | US 2005106726 A1 | 19-05-2005 |
| | | AU 2003216324 A1 | 16-09-2003 |
| | | CA 2455447 A1 | 12-09-2003 |
| | | EP 1423406 A2 | 02-06-2004 |
| | | GB 2396616 A | 30-06-2004 |
| | | US 2004219671 A1 | 04-11-2004 |
| | | WO 02094185 A2 | 28-11-2002 |
| | | US 2003104985 A1 | 05-06-2003 |
| | | US 2003130186 A1 | 10-07-2003 |
| | | AU 2003207708 A1 | 09-09-2003 |
| | | AU 2003210895 A1 | 09-09-2003 |
| | | AU 2003211058 A1 | 09-09-2003 |
| | | AU 2003211082 A1 | 09-09-2003 |
| | | AU 2003213005 A1 | 09-09-2003 |
| | | AU 2003213054 A1 | 09-09-2003 |
| | | AU 2003213057 A1 | 09-09-2003 |
| | | AU 2003213090 A1 | 09-09-2003 |
| | | AU 2003213119 A1 | 09-09-2003 |
| | | AU 2003213163 A1 | 09-09-2003 |
| | | AU 2003213203 A1 | 09-09-2003 |
| | | AU 2003215161 A1 | 09-09-2003 |
| WO 03070918 A | 28-08-2003 | AU 2003207708 A1 | 09-09-2003 |
| | | AU 2003210895 A1 | 09-09-2003 |
| | | AU 2003211058 A1 | 09-09-2003 |
| | | AU 2003211082 A1 | 09-09-2003 |
| | | AU 2003213005 A1 | 09-09-2003 |
| | | AU 2003213054 A1 | 09-09-2003 |
| | | AU 2003213057 A1 | 09-09-2003 |
| | | AU 2003213090 A1 | 09-09-2003 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 0877

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2005

| Patent document cited in search report | Publication date | Patent family member(s) | Publication date |
|---|---|---|---|
| WO 03070918 A | | AU 2003213119 A1 | 09-09-2003 |
| | | AU 2003213163 A1 | 09-09-2003 |
| | | AU 2003213203 A1 | 09-09-2003 |
| | | AU 2003215161 A1 | 09-09-2003 |
| | | AU 2003215203 A1 | 09-09-2003 |
| | | AU 2003215345 A1 | 09-09-2003 |
| | | AU 2003216245 A1 | 09-09-2003 |
| | | AU 2003216255 A1 | 09-09-2003 |
| | | AU 2003216265 A1 | 09-09-2003 |
| | | AU 2003216311 A1 | 09-09-2003 |
| | | AU 2003216315 A1 | 09-09-2003 |
| | | AU 2003216323 A1 | 09-09-2003 |
| | | AU 2003216324 A1 | 16-09-2003 |
| | | AU 2003217550 A1 | 09-09-2003 |
| | | AU 2003217594 A1 | 09-09-2003 |
| | | AU 2003219712 A1 | 09-09-2003 |
| | | AU 2003219751 A1 | 09-09-2003 |
| | | AU 2003219781 A1 | 09-09-2003 |
| | | AU 2003219817 A1 | 09-09-2003 |
| | | AU 2003219818 A1 | 09-09-2003 |
| | | AU 2003219833 A1 | 09-09-2003 |
| | | AU 2003220136 A1 | 09-09-2003 |
| | | AU 2003221258 A1 | 09-09-2003 |
| | | AU 2003247204 A1 | 09-09-2003 |
| | | CA 2455447 A1 | 12-09-2003 |
| | | CA 2455506 A1 | 28-08-2003 |
| | | CA 2456444 A1 | 28-08-2003 |
| | | CA 2457528 A1 | 28-08-2003 |
| | | CA 2459532 A1 | 28-08-2003 |
| | | CA 2463595 A1 | 28-08-2003 |
| | | CA 2471421 A1 | 28-08-2003 |
| | | CA 2476112 A1 | 28-08-2003 |
| | | CA 2476394 A1 | 28-08-2003 |
| | | CA 2477014 A1 | 28-08-2003 |
| | | EP 1432724 A1 | 30-06-2004 |
| | | EP 1463842 A1 | 06-10-2004 |
| | | EP 1472265 A2 | 03-11-2004 |
| | | EP 1465910 A2 | 13-10-2004 |
| | | EP 1423404 A2 | 02-06-2004 |
| | | EP 1442143 A2 | 04-08-2004 |
| | | EP 1448590 A2 | 25-08-2004 |
| | | EP 1432725 A1 | 30-06-2004 |

EPO FORM P0459

For more details about this annex : see Official Journal of the European Patent Office, No. 12/82